(19) Europäisches Patentamt
European Patent Office
Office européen des brevets

(11) EP 4 744 665 A1

(12) EUROPEAN PATENT APPLICATION
published in accordance with Art. 153(4) EPC

(43) Date of publication:
**20.05.2026 Bulletin 2026/21**

(21) Application number: **24838873.8**

(22) Date of filing: **11.07.2024**

(51) International Patent Classification (IPC):
***A61K 31/519** (2006.01)* ***A61K 31/428** (2006.01)*
***A61K 31/429** (2006.01)* ***A61P 35/00** (2006.01)*

(52) Cooperative Patent Classification (CPC):
**A61K 31/428; A61K 31/429; A61K 31/519; A61P 35/00**

(86) International application number:
**PCT/CN2024/105005**

(87) International publication number:
**WO 2025/011621 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **12.07.2023 CN 202310854573**

(71) Applicant: **Simcere Zaiming Pharmaceutical Co., Ltd.**
**Haikou, Hainan 570311 (CN)**

(72) Inventors:
- **ZHOU, Feng**
  **Shanghai 201318 (CN)**
- **JIANG, Lei**
  **Shanghai 201318 (CN)**
- **LIU, Lu**
  **Shanghai 201318 (CN)**
- **QIAN, Shuo**
  **Shanghai 201318 (CN)**
- **XUE, Liting**
  **Shanghai 201318 (CN)**
- **LI, Zhengtao**
  **Shanghai 201318 (CN)**
- **LI, Zhen**
  **Shanghai 201318 (CN)**
- **TANG, Renhong**
  **Shanghai 201318 (CN)**

(74) Representative: **V.O.**
**P.O. Box 87930**
**2508 DH Den Haag (NL)**

# (54) COMBINATION OF POLQ INHIBITOR COMPOUND AND ANTI-CANCER THERAPEUTIC AGENT OR RADIOTHERAPY

(57) Provided is a combination comprising a compound of formula (A) as a POLQ inhibitor or a pharmaceutically acceptable salt thereof or a phannaceutical composition thereof and at least one anti-cancer therapeutic agent or radiotherapy, which combination is used for preventing or treating cancers, and the use of the combination in the prevention or treatment of cancers. The structure of the compound of formula (A) is as shown below.

EP 4 744 665 A1

## Description

### CROSS-REFERENCE TO RELATED APPLICATIONS

The present disclosure claims priority to and the benefit of the Chinese Patent Application No. 202310854573.X, filed with the China National Intellectual Property Administration on July 12, 2023, which is incorporated herein by reference in its entirety.

### TECHNICAL FIELD

The present disclosure relates to a combination for preventing or treating cancer, which comprises a POLQ inhibitor compound and at least one anti-cancer therapeutic agent or radiotherapy, and use of the POLQ inhibitor compound and the at least one anti-cancer therapeutic agent or the radiotherapy in combination in preventing or treating cancer.

### BACKGROUND

DNA double-strand break repair is essential for maintaining genomic stability and cell survival. There are three main repair pathways for DNA double-strand breaks: homologous recombination (HR), non-homologous end joining (NHEJ), and alternative non-homologous end joining (alt-NHEJ). Microhomology-mediated end joining (MMEJ) is the most common non-traditional non-homologous end joining. Homologous recombination is a high-fidelity, error-free repair mechanism that maintains genome stability and avoids cancer induction. Non-homologous end joining and microhomology-mediated end joining are error-prone repair pathways that result in a mutation at the repair site.

Unlike normal cells, the survival of tumor cells often depends on the misregulation of DNA double-strand break repair. At the same time, aberrant DNA double-strand break repair can make tumor cells more sensitive to specific types of DNA damage. Thus, defects in DNA double-strand break repair can be exploited to develop targeted tumor therapies. Tumor cells with impaired homologous recombination or non-homologous end joining repair will be more dependent on microhomology-mediated end joining repair. Multiple lines of evidence in genetics, cell biology, and biochemistry indicate that DNA polymerase $\theta$ (POLQ or POL$\theta$) is a key protein in the process of microhomology-mediated end joining repair (Kent et al. Nature Structural & Molecular Biology (2015), 22(3), 230-237, Mateos-Gomez et al. Nature (2015), 518(7538), 254-257).

POLQ is a multifunctional enzyme composed of an N-terminal helicase domain (SF2 HEL308-type) and a C-terminal low-fidelity DNA polymerase domain (A-type) (Wood & Doublie DNA Repair (2016), 44, 22-32). The helicase domain mediates the removal of the RPA protein from single-stranded DNA and promotes annealing, the polymerase domain can extend single-stranded DNA ends and fill gaps, and the two domains work together to function in a microhomology-mediated end joining repair process.

Studies have shown that POLQ is essential for homologous recombination-deficient cells (e.g., synthetically lethal with FA/BRCA deficiency) and that the protein level of POLQ is upregulated in homologous recombination-deficient tumor cells (Ceccaldi et al. Nature (2015), 518(7538), 258-262). *In vivo* study results also show that POLQ is overexpressed in a series of homologous recombination-deficient ovarian, uterine, and breast cancers with poor prognosis (Higgins et al. Oncotarget (2010), 1, 175-184; Lemee et al. PNAS (2010), 107(30), 13390-13395; Ceccaldi et al. (2015), supra). More importantly, POLQ expression is inhibited in normal tissues compared to tumor tissues (Kawamura et al., International Journal of Cancer (2004), 109(1), 9-16).

In summary, POLQ is essential for homologous recombination-deficient cells, and there is an unmet market need for the treatment of homologous recombination-deficient tumors. Inhibition of the POLQ function can inhibit microhomology-mediated end joining repair in cells, and the development of POLQ function inhibitors can provide a novel strategy for a targeted therapy of homologous recombination-deficient tumors.

Although POLQ inhibitors have shown promising results as a monotherapy, there remains a need in the art to investigate combination therapies of POLQ inhibitors and other anti-cancer therapeutic agents, with the aim of obtaining better and more effective clinical therapeutic drugs and regimens.

### SUMMARY

In one aspect, the present disclosure provides a combination for preventing or treating cancer, which comprises a compound of formula (A) or a pharmaceutically acceptable salt thereof or a pharmaceutical composition thereof and at least one anti-cancer therapeutic agent or radiotherapy, wherein the compound of formula (A) is a POLQ inhibitor and has the following structure:

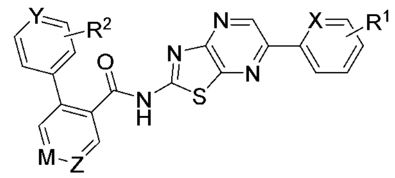

(A)

wherein X is selected from CH or N;
Y is selected from CH or N;
Z is selected from N or $CR^3$;
M is selected from N or $CR^4$;
at least one of Z and M is N;
$R^3$ is selected from H or $CH_3$;
$R^4$ is selected from H or $CH_3$;
$R^1$ is selected from halogen, cyano, =O, $C_1$-$C_6$ alkyl, $C_1$-$C_6$ alkoxy, or $C_3$-$C_6$ cycloalkyl;
$R^2$ is selected from halogen, cyano, $C_1$-$C_6$ alkoxy, $C_1$-$C_6$ alkyl, $C_2$-$C_6$ alkynyl, or $C_3$-$C_6$ cycloalkyl, wherein the $C_1$-$C_6$ alkoxy, $C_1$-$C_6$ alkyl, $C_2$-$C_6$ alkynyl, or $C_3$-$C_6$ cycloalkyl is optionally substituted with $R^{2a}$; and
$R^{2a}$ is selected from halogen or deuterium.

In another aspect, the present disclosure provides use of the combination of the compound of formula (A) or the pharmaceutically acceptable salt thereof or the pharmaceutical composition thereof and the at least one anti-cancer therapeutic agent or the radiotherapy in preparing a medicament for preventing or treating cancer in a patient.

In another aspect, the present disclosure provides a method for preventing or treating cancer in a mammal, which comprises administering to a mammal, preferably a human, in need of such treatment the combination of the compound of formula (A) or the pharmaceutically acceptable salt thereof or the pharmaceutical composition thereof and the at least one anti-cancer therapeutic agent or the radiotherapy.

In another aspect, the present disclosure provides use of the combination of the compound of formula (A) or the pharmaceutically acceptable salt thereof or the pharmaceutical composition thereof and the at least one anti-cancer therapeutic agent or the radiotherapy in preventing or treating cancer.

In some embodiments, Z is N, M is $CR^4$, and $R^4$ is $CH_3$.

In some embodiments, $R^1$ is selected from cyano or $C_3$-$C_6$ cycloalkyl.

In some embodiments, $R^1$ is selected from cyano or cyclopropyl.

In some embodiments, $R^2$ is selected from halogen, $C_1$-$C_3$ alkoxy, $C_1$-$C_3$ alkyl, or cyano, wherein the $C_1$-$C_3$ alkoxy or $C_1$-$C_3$ alkyl is optionally substituted with $R^{2a}$.

In some embodiments, $R^2$ is selected from halogen, $C_1$-$C_3$ alkoxy, $C_1$-$C_3$ alkyl, or cyano.

In some embodiments, $R^2$ is selected from chlorine, methoxy, methyl, or cyano.

In some embodiments, X is selected from CH or N; Y is selected from CH or N; Z is selected from N; M is $CR^4$, and $R^4$ is $CH_3$; $R^1$ is selected from cyano or $C_3$-$C_6$ cycloalkyl; and $R^2$ is selected from halogen, unsubstituted $C_1$-$C_3$ alkoxy, unsubstituted $C_1$-$C_3$ alkyl, or cyano.

In some embodiments, X is selected from CH or N; Y is selected from CH or N; Z is selected from N; M is $CR^4$, and $R^4$ is $CH_3$; $R^1$ is selected from cyano or cyclopropyl; and $R^2$ is selected from halogen, unsubstituted $C_1$-$C_3$ alkoxy, unsubstituted $C_1$-$C_3$ alkyl, or cyano.

In some embodiments, X is selected from CH or N; Y is selected from CH or N; Z is selected from N; M is $CR^4$, and $R^4$ is $CH_3$; $R^1$ is selected from cyano or cyclopropyl; and $R^2$ is selected from chlorine, methoxy, methyl, or cyano.

In some embodiments, the compound of formula (A) or the pharmaceutically acceptable salt thereof is selected from compound I, compound II, compound III, or a pharmaceutically acceptable salt thereof:

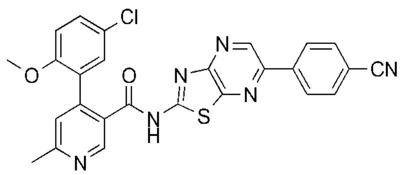

I

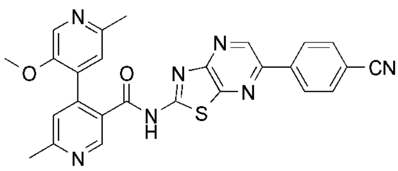

II

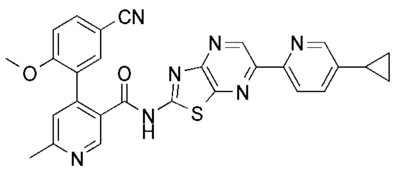

III

.

In some embodiments, the compound of formula (A) or the pharmaceutically acceptable salt thereof is selected

from the following compound I or a pharmaceutically acceptable salt thereof:

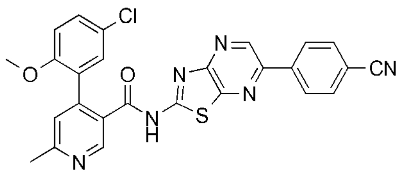

I .

In some embodiments, the pharmaceutically acceptable salt is a sodium salt. In some embodiments, the pharmaceutically acceptable salt is a sodium salt of compound I, a sodium salt of compound II, or a sodium salt of compound III. In some embodiments, the pharmaceutically acceptable salt is a sodium salt of compound I. For example, the sodium salt of compound I has the following structure:

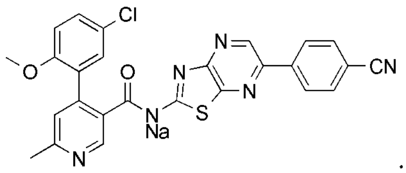

.

In some embodiments, the pharmaceutical composition comprises the compound of formula (A) or the pharmaceutically acceptable salt thereof and a pharmaceutically acceptable excipient.
In some embodiments, the compound of formula (A) or the pharmaceutically acceptable salt thereof and the at least one anti-cancer therapeutic agent are administered in the form of a pharmaceutical combination. In some embodiments, the pharmaceutical combination is a fixed combination or a non-fixed combination.
In some embodiments, the present disclosure provides a pharmaceutical composition for preventing or treating cancer, which comprises the compound of formula (A) or the pharmaceutically acceptable salt thereof and the at least one anti-cancer therapeutic agent.
In some embodiments, the present disclosure provides a pharmaceutical composition for preventing or treating cancer, which comprises the compound of formula (A) or the pharmaceutically acceptable salt thereof, and the pharmaceutical composition is administered in combination with the radiotherapy.
In some embodiments, the cancer is an HR-deficient cancer.
In some embodiments, the cancer is cancer with reduced or absent BRCA gene expression, BRCA gene deficiency, or a reduced BRCA protein function.
In some embodiments, the cancer is cancer with a BRCA mutation.
In some embodiments, the cancer is cancer with BRCA1 and/or BRCA2 mutations.
In some embodiments, the cancer is cancer with a BRCA1 mutation.
In some embodiments, the cancer is selected from colorectal cancer, lung cancer, ovarian cancer, breast cancer, gastric cancer, pancreatic cancer, or prostate cancer.
In some embodiments, the lung cancer is selected from non-small cell lung cancer or small cell lung cancer.
In some embodiments, the lung cancer is selected from non-small cell lung cancer.
In some embodiments, the colorectal cancer is selected from colorectal adenocarcinoma.
In some embodiments, the anti-cancer therapeutic agent is selected from one or more of a platinum derivative, a DNA damage repair pathway inhibitor, a topoisomerase inhibitor, or a tubulin inhibitor.
In some embodiments, the platinum derivative is selected from carboplatin, cisplatin, oxaliplatin, or satraplatin.
In some embodiments, the platinum derivative is selected from carboplatin or cisplatin.
In some embodiments, the DNA damage repair pathway inhibitor is selected from a PARP inhibitor, an ATR inhibitor, or a DNA protein kinase (i.e., DNA-PK) inhibitor.
In some embodiments, the PARP inhibitor is selected from olaparib, niraparib, talazoparib, rucaparib, fluzoparib, pamiparib, veliparib, or AZD5305.
In some embodiments, the PARP inhibitor is selected from olaparib, niraparib, talazoparib, rucaparib, fluzoparib, pamiparib, or veliparib.
In some embodiments, the PARP inhibitor is selected from olaparib, niraparib, talazoparib, or AZD5305.
In some embodiments, the PARP inhibitor is selected from olaparib, niraparib, or talazoparib.
In some embodiments, the ATR inhibitor is selected from RP3500, ceralasertib, berzosertib, ART-0380, M-4344 (VX-803), M-1774, or elimusertib.

In some embodiments, the ATR inhibitor is selected from RP3500.

In some embodiments, the DNA protein kinase (i.e., DNA-PK) inhibitor is selected from AZD7648, CC-115, BR-101801, BR-2002, SRX-2523, or SN-39884.

In some embodiments, the DNA protein kinase (i.e., DNA-PK) inhibitor is selected from AZD7648.

In some embodiments, the topoisomerase inhibitor is selected from irinotecan, etoposide, doxorubicin hydrochloride, or SN38.

In some embodiments, the topoisomerase inhibitor is selected from a topoisomerase II inhibitor or a topoisomerase I inhibitor.

In some embodiments, the topoisomerase inhibitor is selected from irinotecan or etoposide.

In some embodiments, the topoisomerase inhibitor is selected from etoposide, doxorubicin hydrochloride, or SN38. In some embodiments, the topoisomerase inhibitor is selected from etoposide.

In some embodiments, the tubulin inhibitor is selected from paclitaxel or docetaxel.

In some embodiments, the tubulin inhibitor is selected from docetaxel.

In some embodiments, the tubulin inhibitor is selected from paclitaxel.

In some embodiments, the present disclosure provides a combination for preventing or treating cancer, which comprises a compound of formula (A) or a pharmaceutically acceptable salt thereof or a pharmaceutical composition thereof, and a PARP inhibitor.

In some embodiments, the present disclosure provides a combination for preventing or treating cancer, which comprises compound I, compound II, compound III, or a pharmaceutically acceptable salt thereof or a pharmaceutical composition thereof, and a PARP inhibitor.

In some embodiments, the present disclosure provides a combination for preventing or treating cancer, which comprises a compound of formula (A) or a pharmaceutically acceptable salt thereof or a pharmaceutical composition thereof, and radiotherapy.

In some embodiments, the present disclosure provides a combination for preventing or treating cancer, which comprises compound I, compound II, compound III, or a pharmaceutically acceptable salt thereof or a pharmaceutical composition thereof, and radiotherapy.

In some embodiments, the present disclosure provides a combination for preventing or treating cancer, which comprises a compound of formula (A), compound I, compound II, compound III, or a pharmaceutically acceptable salt thereof or a pharmaceutical composition thereof and a PARP inhibitor selected from olaparib, niraparib, talazoparib, or AZD5305, an ATR inhibitor selected from RP3500, a DNA-PK inhibitor selected from AZD7648, a topoisomerase inhibitor selected from etoposide, doxorubicin hydrochloride, or SN38, a tubulin inhibitor selected from paclitaxel, or a platinum derivative selected from carboplatin or cisplatin.

In some embodiments, the present disclosure provides a combination for preventing or treating cancer selected from colorectal cancer, lung cancer, ovarian cancer, breast cancer, pancreatic cancer, or prostate cancer, which comprises a compound of formula (A), compound I, compound II, compound III, or a pharmaceutically acceptable salt thereof or a pharmaceutical composition thereof and a PARP inhibitor selected from olaparib, niraparib, talazoparib, or AZD5305, an ATR inhibitor selected from RP3500, a DNA-PK inhibitor selected from AZD7648, a topoisomerase inhibitor selected from etoposide, doxorubicin hydrochloride, or SN38, a tubulin inhibitor selected from paclitaxel, or a platinum derivative selected from carboplatin or cisplatin.

In some embodiments, the present disclosure provides a combination for preventing or treating cancer selected from colorectal cancer, gastric cancer, lung cancer, prostate cancer, ovarian cancer, or breast cancer, which comprises a compound of formula (A), compound I, compound II, compound III, or a pharmaceutically acceptable salt thereof or a pharmaceutical composition thereof, and radiotherapy.

In some embodiments, the compound of formula (A) or the pharmaceutically acceptable salt thereof or the pharmaceutical composition thereof and the at least one anti-cancer therapeutic agent in the combination may be packaged separately or together.

In some embodiments, compound I, compound II, compound III, or the pharmaceutically acceptable salt thereof or the pharmaceutical composition thereof and the at least one anti-cancer therapeutic agent in the combination may be packaged separately or together.

In some embodiments, in the use or the method, the compound of formula (A) or the pharmaceutically acceptable salt thereof and the at least one anti-cancer therapeutic agent are each in the form of a pharmaceutical composition and may be administered simultaneously, sequentially, or at intervals.

In some embodiments, in the use or the method, compound I, compound II, compound III, or the pharmaceutically acceptable salt thereof and the at least one anti-cancer therapeutic agent are each in the form of a pharmaceutical composition and may be administered simultaneously, sequentially, or at intervals.

In some embodiments, in the use or the method, the compound of formula (A) or the pharmaceutically acceptable salt thereof or the pharmaceutical composition thereof and the at least one anti-cancer therapeutic agent are each administered at intervals.

In some embodiments, in the use or the method, compound I, compound II, compound III, or the pharmaceutically acceptable salt thereof or the pharmaceutical composition thereof and the at least one anti-cancer therapeutic agent are each administered at intervals.

In some embodiments, in the use or the method, the compound of formula (A) or the pharmaceutically acceptable salt thereof or the pharmaceutical composition thereof and the at least one anti-cancer therapeutic agent are each administered according to the same or different administration regimens.

In some embodiments, in the use or the method, compound I, compound II, compound III, or the pharmaceutically acceptable salt thereof or the pharmaceutical composition thereof and the at least one anti-cancer therapeutic agent are each administered according to the same or different administration regimens.

In some embodiments, in the use or the method, the compound of formula (A) or the pharmaceutically acceptable salt thereof or the pharmaceutical composition thereof and the at least one anti-cancer therapeutic agent are each administered according to different administration regimens.

In some embodiments, in the use or the method, compound I, compound II, compound III, or the pharmaceutically acceptable salt thereof or the pharmaceutical composition thereof and the at least one anti-cancer therapeutic agent are each administered according to different administration regimens.

In some embodiments, in the use or the method, the compound of formula (A), compound I, compound II, compound III, or the pharmaceutically acceptable salt thereof may be administered at a frequency of once daily; a dose administered per day is 0.01-100 mg/kg based on the free base form.

In some embodiments, in the use or the method, the at least one anti-cancer agent may be administered at a frequency of once daily.

In some embodiments, in the use or the method, the radiotherapy is at an irradiation dose of 1-6 gray (Gy).

Administration of the combination comprising the compound of formula (A) or the pharmaceutically acceptable salt thereof or the pharmaceutical composition thereof, and the at least one anti-cancer therapeutic agent or the radiotherapy described in the present disclosure contributes to a better therapeutic effect in reducing growth of a tumor or even eliminating a tumor, or provides a smaller amount of administration compared to administration of the compound of formula (A) or the pharmaceutically acceptable salt thereof or the pharmaceutical composition thereof or the at least one anti-cancer therapeutic agent alone, or the radiotherapy alone.

## Terminology and Definitions

Unless otherwise stated, the terms used in the present disclosure have the following meanings, and the definitions of groups and terms described in the present disclosure, including definitions thereof as examples, exemplary definitions, preferred definitions, definitions documented in tables, definitions of specific compounds in the examples, and the like, may be arbitrarily combined and incorporated with each other. A certain term, unless otherwise specifically defined, should not be considered indefinite or unclear, but should be understood according to its common meaning in the field. When referring to a trade name, it is intended to refer to its corresponding commercial product or its active ingredient.

The term "substituted" means that any one or more hydrogen atoms on a specific atom are replaced by substituents, as long as the valence of the specific atom is normal and the compound resulting from the substitution is stable. When a substituent is oxo (i.e., =O), it means that two hydrogen atoms are substituted.

The term "optional" or "optionally" means that the subsequently described event or circumstance may or may not occur. The description includes instances where the event or circumstance occurs and instances where the event or circumstance does not. For example, ethyl being "optionally" substituted with halogen means that the ethyl may be unsubstituted ($CH_2CH_3$), monosubstituted ($CH_2CH_2F$, $CH_2CH_2Cl$, or the like), polysubstituted ($CHFCH_2F$, $CH_2CHF_2$, $CHFCH_2Cl$, $CH_2CHCl_2$, or the like), or fully substituted ($CF_2CF_3$, $CF_2CCl_3$, $CCl_2CCl_3$, or the like). It will be understood by those skilled in the art that for any group comprising one or more substituents, no substitution or substituting pattern that is spatially impossible and/or cannot be synthesized will be introduced.

When any variable (e.g., $R^a$ or $R^b$) occurs more than once in the constitution or structure of a compound, the variable is independently defined in each case. For example, if a group is substituted with two $R^b$, the definition of each $R^b$ is independent.

When a bond of a substituent is cross-linked to two atoms on a ring, the substituent can be bonded to any atom on the ring. For example, the structural unit

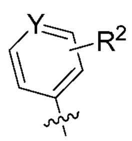

indicates that $R^2$ may be substituted at any position on the Y-containing aromatic ring, and the number of the substituent $R^2$

may be 1, 2, 3, 4, or 5.

The term "alkyl" refers to a hydrocarbyl group with a general formula of $C_nH_{2n+1}$. The alkyl may be linear or branched. The term "$C_1$-$C_6$ alkyl" may be understood to represent an alkyl group having 1, 2, 3, 4, 5, or 6 carbon atoms. Specific examples include, but are not limited to, methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, and the like. The term "$C_1$-$C_3$ alkyl" may be understood to represent a linear or branched saturated alkyl group having 1, 2, or 3 carbon atoms.

The term "alkoxy" refers to a group derived from a linear or branched alcohol by loss of a hydrogen atom from hydroxy, and may be understood as "alkyloxy" or "alkyl-O-". The term "$C_1$-$C_6$ alkoxy" may be understood as "$C_1$-$C_6$ alkyloxy" or "$C_1$-$C_6$ alkyl-O-". The term "$C_1$-$C_3$ alkoxy" may be understood as "$C_1$-$C_3$ alkyloxy" or "$C_1$-$C_3$ alkyl-O-".

The term "alkynyl" refers to a linear or branched unsaturated aliphatic hydrocarbyl group consisting of carbon atoms and hydrogen atoms and having at least one triple bond. The term "$C_2$-$C_6$ alkynyl" may be understood to represent a linear or branched unsaturated hydrocarbyl group comprising one or more triple bonds and having 2, 3, 4, 5, or 6 carbon atoms. Examples of "$C_2$-$C_6$ alkynyl" include, but are not limited to, ethynyl ($-C\equiv CH$), prop-1-ynyl ($-C\equiv CCH_3$), prop-2-ynyl ($-CH_2C\equiv CH$), but-1-ynyl, but-2-ynyl, or but-3-ynyl.

The term "cycloalkyl" refers to a fully saturated carbon ring that exists in the form of a monocyclic ring, a fused ring, a bridged ring, a spiro ring, or the like. Unless otherwise indicated, the carbon ring is generally a 3-6 membered ring. The term "$C_3$-$C_6$ cycloalkyl" may be understood to represent a saturated monocyclic or bicyclic hydrocarbyl ring having 3, 4, 5, or 6 carbon atoms. Specific examples include, but are not limited to, cyclopropyl, cyclobutyl, cyclopentyl, cyclohexyl, or the like.

The term "halogen" refers to fluorine, chlorine, bromine, or iodine.

The term "cyano" refers to a -CN group.

The term "in combination with" or "combined use" refers to a combination of two or more active ingredients administered simultaneously or sequentially (in the form of the respective active ingredients themselves or their pharmaceutical compositions, or in the form of their respective pharmaceutically acceptable salts, esters, or other derivatives, prodrugs, or their pharmaceutical compositions), or to the active ingredients administered simultaneously or sequentially (in the form of the respective active ingredients themselves or their pharmaceutical compositions, or in the form of their respective pharmaceutically acceptable salts, esters, or other derivatives, prodrugs, or their pharmaceutical compositions) and radiotherapy, etc. In some embodiments, "in combination with" or "combined use" means that two or more active substances may be administered to an individual in need thereof either simultaneously as single formulations, or sequentially in any order as single formulations. In some embodiments, "in combination with" or "combined use" means that the active substance as a single formulation and radiotherapy may be administered to an individual in need thereof either simultaneously, or sequentially in any order. The term "pharmaceutical composition" refers to a mixture of the active ingredient described in the present disclosure and a pharmaceutically acceptable excipient, which can be prepared by combining the active ingredient described in the present disclosure with a pharmaceutically acceptable excipient. For example, a pharmaceutical composition comprising a compound of formula (A) or a pharmaceutically acceptable salt thereof and at least one anti-cancer therapeutic agent may refer to a single formulation comprising a compound of formula (A) or a pharmaceutically acceptable salt thereof and at least one anti-cancer therapeutic agent as active ingredients and prepared by combining the active ingredients with a pharmaceutically acceptable excipient.

The term "combination" encompasses both "in combination with" or "combined use" and "pharmaceutical composition". In some embodiments, "combination" refers to "in combination with" or "combined use". In some embodiments, "combination" refers to "pharmaceutical composition".

The term "pharmaceutical combination" means a product that results from the mixing or combining of more than one active ingredient, including both a fixed combination and a non-fixed combination of the active ingredients. The term "fixed combination" means that the active ingredients (e.g., the compound and the anti-cancer therapeutic agent of the present disclosure) are both administered to a patient simultaneously in the form of a single entity or a single dosage form. The term "non-fixed combination" means that the active ingredients (e.g., the compound and the anti-cancer therapeutic agent of the present disclosure) are both administered to a patient as separate entities either simultaneously, concurrently, or sequentially with no specific time limits, wherein such administration provides therapeutically effective levels of the two compounds in the patient. The latter also applies to cocktail therapy, e.g., the administration of three or more active ingredients.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" includes a salt formed by a basic ion with a free acid or a salt formed by an acidic ion with a free base, and for example, a metal salt, an ammonium salt, a salt formed with an organic base, a salt formed with an inorganic acid, a salt formed with an organic acid, a salt formed with a basic or acidic amino acid, etc. may be mentioned.

The term "a compound of formula (A) or a pharmaceutically acceptable salt thereof" refers to an anhydrate and/or a solvate (e.g., hydrate) of the compound of formula (A), or an anhydrate and/or a solvate of a pharmaceutically acceptable salt of the compound of formula (A).

The dose of compound I, compound II, or compound III or a pharmaceutically acceptable salt thereof involved in the present disclosure is calculated based on the molecular weight of compound I, compound II, or compound III in free form, unless otherwise specified.

The pharmaceutical composition of the present disclosure may be administered by various suitable routes, or the components of the combination drug may each be administered independently by various suitable routes. Typical routes include, but are not limited to, oral, rectal, topical, inhalation, parenteral, sublingual, intravaginal, intranasal, intraocular, intraperitoneal, intramuscular, subcutaneous, subcuticular, subcapsular, subarachnoid, intravenous, intraarterial, intrathecal, intralymphatic, intralesional, intracapsular, intraorbital, intracardiac, intradermal, intraarticular, intraspinal, transtracheal, epidural, and intrasternal administration.

The term "pharmaceutically acceptable excipient" refers to those that do not have a significant irritating effect on an organism and do not impair the biological activity and properties of the active compound. Suitable excipients are well known to those skilled in the art, for example, carbohydrate, wax, water-soluble and/or water-swellable polymers, hydrophilic or hydrophobic material, gelatin, oil, solvent, or water.

The term "individual" is a mammal. In some embodiments, the individual is a mouse. In some embodiments, the individual is a human.

The components of the combination drug in the present disclosure may each independently be present in the form of a pharmaceutical composition.

The components of the combination drug in the present disclosure may each be independently, or some or all of them are together in a suitable dosage form, including, but not limited to, oral forms such as tablets, pills, lozenges, dragees, capsules, liquids, gels, slurries, or suspensions, or parenteral forms such as sterile solutions, suspensions, or lyophilized products.

The word "comprise" and variations thereof such as "comprises" or "comprising" should be understood in an open, non-exclusive sense, i.e., "including but not limited to".

The term "treat" generally refers to obtaining desired pharmacological and/or physiological effects. The effects may be therapeutic, as they partially or completely stabilize or cure a disease and/or the side effects caused by the disease. "Treatment" in the present disclosure encompasses any treatment of a disease in a patient, including: (a) inhibiting a symptom of the disease, i.e., arresting its development; or (b) relieving a symptom of the disease, i.e., causing the disease or symptom to regress.

The term "effective amount" refers to an amount of the compound of the present disclosure for (i) treating a specific disease, condition, or disorder; (ii) alleviating, ameliorating, or eliminating one or more symptoms of a specific disease, condition, or disorder, or (iii) delaying the onset of the one or more symptoms of the specific disease, condition, or disorder described herein. The amount of the compound of the present disclosure constituting the "therapeutically effective amount" varies depending on the compound, the disease state and its severity, the administration regimen, and the age of the mammal to be treated, but can be determined routinely by those skilled in the art in accordance with their knowledge and the present disclosure.

The compounds of the present disclosure can be prepared by a variety of synthetic methods well known to those skilled in the art, including the specific embodiments listed below, embodiments formed by combinations of the specific embodiments with other chemical synthetic methods, and equivalents thereof well known to those skilled in the art. The preferred embodiments include, but are not limited to, the examples disclosed herein.

The chemical reactions of the specific embodiments of the present disclosure are conducted in a suitable solvent that must be suitable for the chemical changes in the present disclosure and the reagents and materials required. In order to obtain the compounds of the present disclosure, it is sometimes necessary for those skilled in the art to modify or select a synthesis procedure or a reaction process based on the existing embodiments.

Unless otherwise defined herein, scientific and technical terms used in correlation with the present disclosure shall have the meanings that are commonly understood by those skilled in the art.

The following abbreviations are used in the present disclosure:

| Abbreviation | Chemical name | Abbreviation | Chemical name |
|---|---|---|---|
| HATU | O-(7-Azabenzotriazol-1-yl)-*N,N,N',N'*-tetramethyluronium hexafluorophosphate | DIEA or DIPEA | N,N-Diisopropylethylamine |
| $Pd(dppf)Cl_2$ or $PdCl_2(dppf)$ | 1,1'-Bis(diphenylphosphino)ferrocene dichloropalladium(II) or 1,1-bis(diphenylphosphine)ferrocene palladium chloride | $Pd(dtbpf)Cl_2$ | 1,1-Bis(tert-butylphosphine)ferrocene palladium chloride |

(continued)

| Abbreviation | Chemical name | Abbreviation | Chemical name |
|---|---|---|---|
| HOBt | 1-Hydroxybenzotriazole | DMAP | 4-Dimethylaminopyridine |

## BRIEF DESCRIPTION OF THE DRAWINGS

FIG. 1 shows matrix diagrams of combined use of compound I and the sodium salt thereof, compound II and the sodium salt thereof, and compound III of the present disclosure with a combination drug, separately.
FIG. 2 shows matrix diagrams of combined use of the sodium salt of compound I with irradiation.
FIG. 3 shows curve graphs of the tumor volume (A) and the body weight change rate (B) for each group in the DLD-1 BRCA2-/- xenograft tumor model.
FIG. 4 shows curve graphs of the tumor volume (A) and the body weight change rate (B) for each group in the MDA-MB-436 xenograft tumor model.

## EXAMPLES

The present disclosure is described in detail below by way of examples. However, this is by no means disadvantageously limiting the scope of the present disclosure. Although the present disclosure has been described in detail herein and specific embodiments thereof have also been disclosed, it will be apparent to those skilled in the art that various changes and modifications can be made to the specific embodiments of the present disclosure without departing from the spirit and scope of the present disclosure. All reagents used in the present disclosure are commercially available and can be used without further purification.

Compounds are named either manually or by ChemDraw® software, and suppliers' catalog names are given for commercially available compounds.

The structures of the compounds are determined by nuclear magnetic resonance (NMR) and/or mass spectrometry (MS). NMR shifts are given in $10^{-6}$ (ppm). The solvents for NMR determination are deuterated dimethyl sulfoxide, deuterated chloroform, deuterated methanol, and the like, and the internal standard is tetramethylsilane (TMS); "$IC_{50}$" refers to the half inhibitory concentration, which is the concentration at which half of the maximal inhibitory effect is achieved.

In the following purification using high-performance liquid chromatography, "%" for the amount of the acid or base used in the mobile phase A means a volume fraction unless otherwise specified. For example, "water (0.05% formic acid)" means that the volume of formic acid is 0.05% of the total volume of formic acid and water. B% represents the ratio of the volume of mobile phase B to the total volume of mobile phase A and mobile phase B during gradient elution, and "B%: 50%-70%" represents that the ratio of the volume of mobile phase B to the total volume of mobile phase A and mobile phase B changes from 50% to 70% during gradient elution.

### Example 1. 4-(5-Chloro-2-methoxyphenyl)-N-[6-(4-cyanophenyl)thiazolo[4,5-b]pyrazin-2-yl]-6-methylnicotinamide (Compound I)

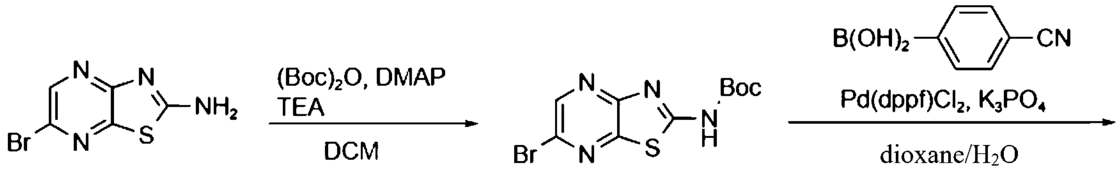


I-1 I-2

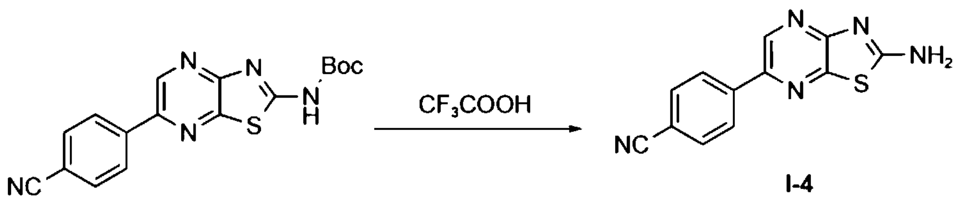


I-3 I-4

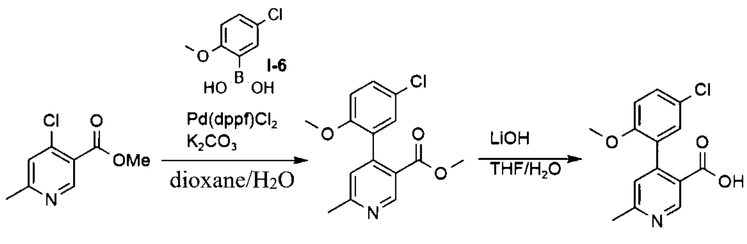


I-5 I-7 I-8

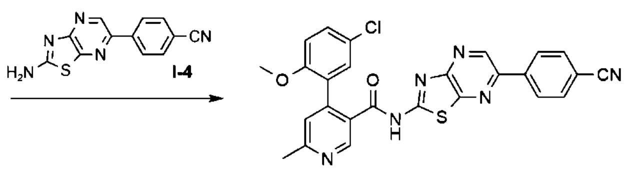


I

**Step 1: Synthesis of tert-butyl (6-bromothiazolo[4,5-*b*]pyrazin-2-yl)carbamate (intermediate I-2)**

**Starting material I-1** (790 mg), triethylamine (691.90 mg), and DMAP (41.77 mg) were dissolved in dichloromethane (10 mL), $(Boc)_2O$ (820.78 mg) was added dropwise to the reaction mixture, and the mixture was stirred at room temperature for 16 h. Then, the reaction mixture was washed with saturated brine, the organic phase was dried over anhydrous sodium sulfate, filtered, and the filtrate was concentrated under reduced pressure to give the title compound (1 g). MS m/z (ESI): 331.2/333.2 $[M+H]^+$.

**Step 2: Synthesis of tert-butyl (6-(4-cyanophenyl)thiazolo[4,5-*b*]pyrazin-2-yl)carbamate (intermediate I-3)**

**Intermediate I-2** (1 g), 4-cyanobenzeneboronic acid (887.34 mg), Pd(dppf)$Cl_2$ (220.93 mg), and potassium phosphate (1.28 g) were added to dioxane (10 mL) and water (2 mL), and the mixture was stirred at 80 °C for 4 h. The reaction mixture was poured into water, and the mixture was extracted with ethyl acetate (10 mL × 3). The organic phase was dried over anhydrous sodium sulfate and filtered, and the filtrate was concentrated to dryness under reduced pressure. The residue was slurried with ethyl acetate and filtered, and the filter cake was dried to give the title compound (0.5 g). MS m/z (ESI): 354.0 $[M+H]^+$.

**Step 3: Synthesis of 4-(2-aminothiazolo[4,5-*b*]pyrazin-6-yl)benzonitrile (intermediate I-4)**

**Intermediate I-3** (0.48 g) was dissolved in trifluoroacetic acid (2 mL), and the mixture was stirred at room temperature for 1 h. Ethyl acetate was added to the reaction mixture, and the mixture was filtered. The filtrate was concentrated to dryness under reduced pressure to give the title compound (230 mg). MS m/z (ESI): 254.0 $[M+H]^+$.

**Step 4: Synthesis of methyl 4-(5-chloro-2-methoxyphenyl)-6-methylpyridine-3-carboxylate (intermediate 1-7)**

Under a nitrogen atmosphere, **intermediate I-6** (1 g) was dissolved in dioxane (20 mL) and water (5 mL), and **intermediate I-5** (995.75 mg), $Pd(dppf)Cl_2$ (349.65 mg), and potassium carbonate (1.48 g) were added to the reaction mixture. Then, the reaction mixture was stirred at 90 °C for 2 h under a nitrogen atmosphere. After the reaction was completed, water (50 mL) was added to the reaction mixture. The mixture was extracted with ethyl acetate (50 mL × 3 times). The organic phases were combined and dried over anhydrous sodium sulfate. After filtration, the organic phase was concentrated under reduced pressure to remove the solvent, and the residue was purified by silica gel column chromatography (ISCO®; 20 g SepaFlash® Silica Flash chromatographic column, gradient: 0-50% ethyl acetate/petroleum ether, flow rate: 20 mL/min) to give the title compound (870 mg). MS m/z (ESI): 292.1 $[M+H]^+$.

**Step 5: Synthesis of 4-(5-chloro-2-methoxyphenyl)-6-methylpyridine-3-carboxylic acid (intermediate 1-8)**

**Intermediate I-7** (870 mg) was added to tetrahydrofuran (8 mL) and water (4 mL), lithium hydroxide (157.13 mg) was added to the reaction mixture, and then the reaction mixture was stirred at 25 °C for 16 h. After the reaction was completed, the pH of the reaction mixture was adjusted to 3, and the solvent was removed by concentration under reduced pressure. The residue was washed with a mixed solvent of dichloromethane/methanol (10/1, 20 mL) and filtered, and the filtrate was concentrated to dryness under reduced pressure to give the title compound (1.2 g). MS m/z (ESI): 277.9 $[M+H]^+$.

**Step 6: Synthesis of 4-(5-chloro-2-methoxyphenyl)-*N*-[6-(4-cyanophenyl)thiazolo[4,5-b]pyrazin-2-yl]-6-methylnicotinamide (compound I)**

Under a nitrogen atmosphere, **intermediate I-8** (300 mg) was added to N,N-dimethylformamide (5 mL), and **intermediate I-4** (273.62 mg), HATU (410.76 mg), and N,N-diisopropylethylamine (279.24 mg) were added to the reaction mixture. Then, the reaction mixture was stirred at 25 °C for 2 h under a nitrogen atmosphere. After the reaction was completed, the reaction mixture was purified by preparative high-performance liquid chromatography (chromatographic column: Boston Prime C18 150 × 30 mm × 5 μm; mobile phase: [A: water (0.225% formic acid), B: acetonitrile]; B%: 48%-68%, 11 min) to give the title compound (51.96 mg). MS m/z (ESI): 513.1 $[M+H]^+$. **$^1$H NMR (400MHz, DMSO-*d*$_6$)** δ 13.40 (brs, 1H), 9.27 (s, 1H), 8.84 (s, 1H), 8.36 (d, *J* = 8.4 Hz, 2H), 8.00 (d, *J* = 8.3 Hz, 2H), 7.50-7.42 (m, 2H), 7.36 (s, 1H), 7.03-6.99 (m, 1H), 3.52 (s, 3H), 2.59 (s, 3H).

**Example 2. Synthesis of Sodium Salt of 4-(5-Chloro-2-methoxyphenyl)-N-[6-(4-cyanophenyl)thiazolo[4,5-b]pyrazin-2-yl]-6-methylnicotinamide (Sodium Salt of Compound I)**

9.5 g of compound I was suspended in 190 mL of absolute ethanol, and a solution of sodium hydroxide (742 mg) in absolute ethanol (190 mL) was added. After the addition, the mixture was stirred at room temperature for 3 h. The reaction mixture was filtered, and the filter cake was washed with absolute ethanol (40 mL). The filter cake was collected and dried under vacuum at 50 °C for 12 h to give a sodium salt of compound I. $^1$H NMR (400 MHz, DMSO-d6) δ 9.02 (s, 1H), 8.96 (s, 1H), 8.28 (d, J = 8.5 Hz, 2H), 7.92 (d, J = 8.5 Hz, 2H), 7.37 (m, 1H), 7.24 (d, J = 2.7 Hz, 1H), 7.13 (s, 1H), 6.99 (d, J = 8.9 Hz, 1H), 3.52 (s, 3H), 2.53 (s, 3H).

**Example 3. *N*-(6-(4-Cyanophenyl)thiazolo[4,5-b]pyrazin-2-yl)-5'-methoxy-2',6-dimethyl-[4,4'-bipyridinyl]-3-carboxamide (Compound II)**

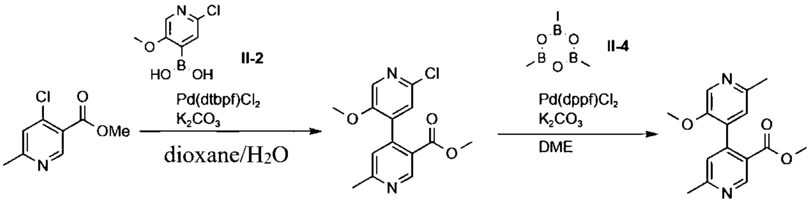


II-1 II-3 II-5

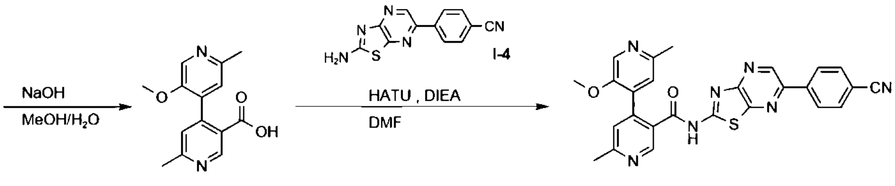


II-6 II

**Step 1: Synthesis of methyl 2'-chloro-5'-methoxy-6-methyl-[4,4'-bipyridine]-3-carboxylate (intermediate II-3)**

At 20 °C, **intermediate II-1** (3.96 g) was dissolved in a dioxane (40 mL)/water (8 mL) solution, and **intermediate II-2** (4 g), potassium carbonate (5.90 g), and 1,1-bis(tert-butylphosphine)ferrocene palladium chloride (Pd(dtbpf)$Cl_2$) (1.39 g) were added to the mixture. The reaction mixture was stirred at 80 °C for 1 h under a nitrogen atmosphere. After the reaction was completed, water (100 mL) was added to the mixture. The mixture was extracted with ethyl acetate (50 mL × 3 times). The organic phases were combined and dried over anhydrous sodium sulfate. After filtration, the organic phase was concentrated under reduced pressure to remove the solvent. The residue was purified by silica gel column chromatography (petroleum ether/ethyl acetate = 1/1) to give the title compound (3.2 g). MS m/z (ESI): = 292.9 $[M+H]^+$.

**Step 2: Synthesis of methyl 5'-methoxy-2',6-dimethyl-[4,4'-bipyridine]-3-carboxylate (intermediate II-5)**

**Intermediate II-3** (250 mg) and **intermediate II-4** (321.64 mg) were dissolved in 1,2-dimethoxyethane (2 mL), and potassium carbonate (295.10 mg) was added to the mixture. Then Pd(dppf)$Cl_2$ (62.49 mg) was added to the reaction mixture. The reaction mixture was stirred at 115 °C for 2 h under a nitrogen atmosphere. After the reaction was completed, the reaction mixture was filtered, and the filtrate was concentrated under reduced pressure to remove the solvent. The residue was purified by flash silica gel column chromatography (ISCO®; 12 g SepaFlash® flash silica gel column, gradient: 0-40% petroleum ether/ethyl acetate, flow rate: 50 mL/min) to give the title compound (200.0 mg). MS m/z (ESI): 273.0 $[M+H]^+$.

**Step 3: Synthesis of 5'-methoxy-2',6-dimethyl-[4,4'-bipyridine]-3-carboxylic acid (intermediate II-6)**

**Intermediate II-5** (200 mg) was dissolved in anhydrous methanol (4 mL), sodium hydroxide (88.13 mg) and water (1 mL) were added to the mixture, and the reaction mixture was stirred at 25 °C for 2 h. After the reaction was completed, the reaction mixture was concentrated under reduced pressure, and the pH of the residue was adjusted to 3 with an appropriate amount of hydrochloric acid. The reaction mixture was concentrated to dryness under reduced pressure, and the residue was stirred with 10 mL of dichloromethane:methanol (10:1) for 10 min and filtered. The filtrate was concentrated under reduced pressure to remove the solvent to give the title compound (120.0 mg). MS m/z (ESI): 259.0 $[M+H]^+$.

**Step 4: Synthesis of *N*-(6-(4-cyanophenyl)thiazolo[4,5-b]pyrazin-2-yl)-5'-methoxy-2',6-dimethyl-[4,4'-bipyridinyl]-3-carboxamide (compound II)**

**Intermediate II-6** (50.00 mg) was dissolved in anhydrous *N,N*-dimethylformamide (1 mL), HATU (77.29 mg) and *N,N*-diisopropylethylamine (75.06 mg) were added to the mixture, and the reaction mixture was stirred at 25 °C for 30 min under a nitrogen atmosphere. Then **intermediate I-4** (53.94 mg) was added to the mixture, and the reaction mixture was stirred at 25 °C for 2 h under a nitrogen atmosphere. After the reaction was completed, the reaction mixture was filtered, and the filtrate was purified by preparative high-performance liquid chromatography (Boston Green ODS C18 column, 5 μm silica, 30 mm in diameter, 150 mm in length; a mixture of water (containing 0.225% formic acid) and acetonitrile with decreasing polarity used as the eluent; acetonitrile gradient: 15%-45%, elution time: 12 min) to give the title compound (12

mg). MS m/z (ESI): 494.0 [M+H]+. **$^1$H NMR (400MHz, DMSO-$d_6$)** $\delta$ = 13.56 (brs, 1H), 9.37 (s, 1H), 8.88 (s, 1H), 8.39 (d, *J* = 8.4 Hz, 2H), 8.24 (s, 1H), 8.02 (d, *J* = 8.4 Hz, 2H), 7.45 (s, 1H), 7.43 (s, 1H), 3.62 (s, 3H), 2.63 (s, 3H), 2.54 (s, 3H).

**Example 4. 4-(5-Chloro-2-methoxyphenyl)-*N*-[6-(5-cyclopropylpyridin-2-yl)thiazolo[4,5-*b*]pyrazin-2-yl]-6-methylpyridine-3-carboxamide (Compound III)**

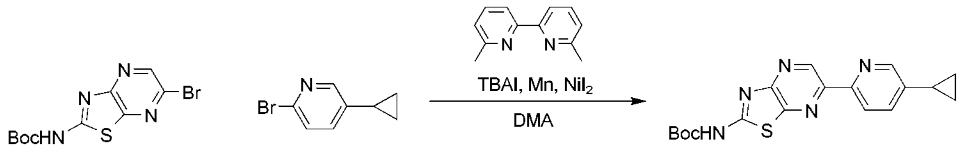


I-2 III-1

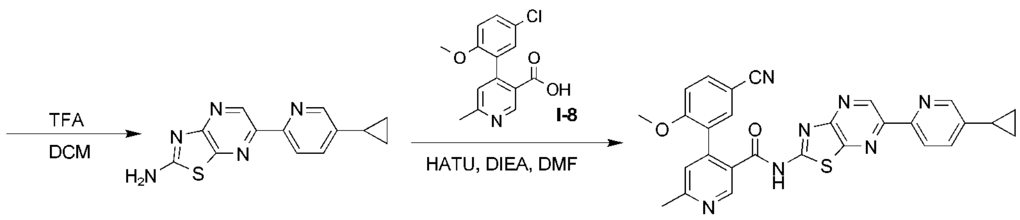


III-2 III

**Step 1: Synthesis of tert-butyl (6-(5-cyclopropylpyridin-2-yl)thiazolo[4,5-b]pyrazin-2-yl)carbamate (intermediate III-1)**

**Intermediate I-2** (500 mg), 2-bromo-5-cyclopropylpyridine (299.01 mg), 6,6'-dimethyl-2,2'-bipyridine (27.81 mg), tetrabutylammonium iodide (836.46 mg), manganese powder (331.76 mg), and nickel iodide (47.18 mg) were dissolved in *N,N*-dimethylacetamide (20 mL), and the reaction mixture was stirred at 90 °C for 16 h under a nitrogen atmosphere. After the reaction was completed, the mixture was filtered, and water (60 mL) was added to the filtrate. The mixture was extracted with ethyl acetate (30 mL $\times$ 2 times). The organic phases were combined and dried over anhydrous sodium sulfate. After filtration, the organic phase was concentrated under reduced pressure to remove the solvent. The residue was purified by flash silica gel column chromatography (ISCO®; 8 g SepaFlash® flash silica gel column, gradient: 0-34% ethyl acetate/petroleum ether, flow rate: 20 mL/min) to give the title compound (140 mg). MS m/z (ESI): = 370.2 [M+H]+.

**Step 2: Synthesis of 6-(5-cyclopropylpyridin-2-yl)thiazolo[4,5-*b*]pyrazin-2-amine (intermediate III-2)**

**Intermediate III-1** (180 mg) was dissolved in dichloromethane (2 mL) and trifluoroacetic acid (2 mL). The reaction mixture was stirred at 20 °C for 4 h. After the reaction was completed, the reaction mixture was concentrated to dryness under reduced pressure to give the title compound (80 mg). MS m/z (ESI): = 270.0 [M+H]+.

**Step 3: Synthesis of 4-(5-chloro-2-methoxyphenyl)-N-[6-(5-cyclopropylpyridin-2-yl)thiazolo[4,5-b]pyrazin-2-yl]-6-methylpyridine-3-carboxamide (compound III)**

At 20 °C, **intermediate III-2** (150 mg) was dissolved in *N,N*-dimethylformamide (2.5 mL), and **intermediate I-8** (154.67 mg), HATU (211.77 mg), and DIEA (143.96 mg) were added to the mixture. The reaction mixture was stirred at 20 °C for 6 h. After the reaction was completed, the reaction mixture was purified by high-performance liquid chromatography (chromatographic column: Boston Green ODS 150 $\times$ 30 mm $\times$ 5 $\mu$m; mobile phase: A: water (0.225% formic acid), B: acetonitrile; B%: 55%-85%, 14 min) to give the title compound (91 mg). **$^1$H NMR(400 MHz, DMSO-$d_6$)** $\delta$ = 13.42(s, 1H), 9.49(s, 1H), 8.80(s, 1H), 8.54(s, 1H), 8.22(d, *J* = 7.7 Hz, 1H), 7.60(d, *J* = 7.5 Hz, 1H), 7.54-7.39(m, 3H), 7.01(d, *J* = 8.6 Hz, 1H), 3.52(s, 3H), 2.61(s, 3H), 2.05-1.98(m, 1H), 1.09-1.01(m, 2H), 0.91-0.86(s, 2H); MS m/z(ESI): = 529.1 [M+H]+.

A sodium salt of compound II and a sodium salt of compound III were prepared by referring to the method in Example 2.

## Test Examples of Biological Activity and Related Properties

The compounds in the following test examples were prepared according to the methods in the above examples disclosed in the present disclosure.

## Test Example 1. POLQ Enzyme Activity Inhibition Experiment

**Brief introduction to experimental principle:** After the N-terminal active peptide segment of POLQ with ATPase activity (M1-N899) is co-incubated with the compound, this peptide segment reacts with substrate dT50 under the action of ATP to generate ADP. The generated ADP then participates in the subsequent NADH oxidation-coupled enzymatic reaction, catalyzing the conversion of NADH to $NAD^+$. The reduction in the OD value of NADH at 340 nm was measured using the Envision microplate reader from Perkin Elmer to reflect enzyme activity. **Experimental instruments:** Labcyte Echo 650 pipetting system; Perkin Elmer Envision microplate reader; Eppendorf 5810R centrifuge; Boxun BSD-YX3400 thermostatic shaker.

**Experimental materials:**

| Reagent | Brand |
| --- | --- |
| POLQ (N) | Synthesized by Pharmaron |
| ATP | Sigma |
| dT50 | Synthesized by GenScript |
| NADH | Roche |
| PEP | Sigma |
| Lactate dehydrogenase | Sigma |
| Pyruvate kinase | Sigma |
| 384-well plate | Greiner Bio-One |

**Experimental methods:** The POLQ enzyme was diluted to 100 nM with a reaction buffer (20 mM Tris HCl (pH 7.80), 80 mM KCl, 10 mM $MgCl_2$, 1 mM DTT, 0.01% w/v bovine serum albumin, 0.01% v/v Tween-20, 5% v/v glycerol). The test compound was diluted to different concentrations with dimethyl sulfoxide (DMSO) using an Echo 650 pipetting system and transferred to a 384-well plate, 20 μL/well of 100 nM POLQ was added, and the mixture was incubated at room temperature for 15 min. A reaction mixture solution was prepared with the concentrations of each component in the reaction mixture solution as follows: 100 μM ATP, 300 nM dT50, 300 μM NADH, 6 mM PEP, 10 U/mL lactate dehydrogenase, and 20 U/mL pyruvate kinase. 20 μL/well of the reaction mixture solution was added to start the enzyme reaction. In the reaction system, the compound was serially diluted 3-fold from the final concentration of 10 μM, covering a concentration range from 10 μM to 0.0005 μM, and the final concentration of DMSO in the system was 0.2% v/v. After the 384-well plate was reacted at room temperature for 20 min, the OD value at 340 nm was read using an Envision microplate reader.

**Data analysis:**

Inhibition rates were calculated and fitted using XLfit software to give the $IC_{50}$ of the compound.

A blank group and a DMSO group were set in the experiment. The reaction system of the blank group was 0.2% v/v DMSO and the reaction mixture solution, and the inhibition rate was considered to be 100% under this condition. The reaction system of the DMSO group was 0.2% v/v DMSO, POLQ (N) (100 nM), and the reaction mixture solution, and the inhibition rate was considered to be 0 under this condition.

$$\text{Inhibition rate} = (100 - 100 \times (OD_{max} - OD_{compound})/(OD_{max} - OD_{min}))\%$$

wherein $OD_{max}$ refers to the OD value of the well containing the reactant mixture solution and 0.2% v/v DMSO, and $OD_{compound}$ refers to the OD value of the well containing the compound, the enzyme, and the reactant mixture solution.

$OD_{min}$ refers to the OD value of the well containing the enzyme, the reactant mixture solution, and 0.2% v/v DMSO.
The biological activity of the compounds of the present disclosure was determined by the above test, and the measured $IC_{50}$ values are shown in Table 1 below.

Table 1. $IC_{50}$ of example compounds on inhibition of POLQ enzyme activity

| Example compound number | $IC_{50}$ (nM) |
|---|---|
| Compound I | ++++ |
| Compound II | ++++ |
| Compound III | ++++ |

In the above table, the symbols used to indicate the inhibitory activity represent the following meanings:

"++++" indicates that the $IC_{50}$ range of the test compound for enzyme inhibitory activity is: $IC_{50}$ < 100 nM.
"+++" indicates that the $IC_{50}$ range of the test compound for enzyme inhibitory activity is: 100 ≤ $IC_{50}$ < 500 nM.
"++" indicates that the $IC_{50}$ range of the test compound for enzyme inhibitory activity is: 500 ≤ $IC_{50}$ < 1000 nM.

**Test Example 2. Inhibitory Experiment of Compounds on Tumor Cell Proliferation**

**Brief introduction to experimental principle:** After the compound was co-incubated with tumor cells for 7 days, the ATP in viable cells was quantified using the CTG kit from Promega to reflect the effect of the compound on tumor cell proliferation.
**Experimental instruments:** Perkin Elmer Envision microplate reader; Eppendorf 5810R centrifuge; Countstar automatic cell counter.

**Experimental materials:**

| Reagent | Brand | Catalog No. |
|---|---|---|
| DLD-1 parent cells | Horizon | HD PAR-008 |
| DLD-1 BRCA2 (-/-) cell line | Horizon | HD 105-007 |
| RPMI 1640 medium | Gibco | A10491-01 |
| FBS | Gibco | 10099-141C |
| 0.25% Trypsin-EDTA | Gibco | 25200-172 |
| PBS | HyClone | SH30256.01 |
| 96-well plate | Corning | 3610 |
| CelltiterGlo assay kit (CTG) | Promega | G7573 |

**Experimental methods:** DLD-1 parent cells or DLD-1 BRCA2 (-/-) cells were diluted with an RPMI 1640 medium containing 10% FBS and then added to a 96-well plate (90 μL/well), with the number of cells being 600/well or 1200/well, respectively, and incubated overnight in an incubator at 37 °C with 5% $CO_2$. The test compound was diluted to different concentrations with dimethyl sulfoxide (DMSO) and then added to a 96-well plate, such that, in the reaction system, the compound was serially diluted 4-fold from the final concentration of 25 μM. The concentration range of the compound was from 25 μM to 0.0004 μM, and the final concentration of DMSO was 0.25% v/v. After incubation for 7 days, 50 μL/well of CTG was added, and the mixture was incubated at room temperature for 10 min. The luminescence (Lum) signal value was read using an Envision microplate reader, and the inhibition rate and half inhibitory concentration ($IC_{50}$) were calculated.

**Data analysis:**

Inhibition rates were calculated and fitted using XLfit software to give the $IC_{50}$ of the compound.
A Blank well and a DMSO well were set in the experiment. The blank well contained 100 μL of an RPMI Medium

1640 medium containing 10% FBS, and the inhibition rate of the compound on tumor cell growth was considered to be 100% under this condition. For the DMSO well, 0.25% v/v DMSO was added to the cell-containing well, and the inhibition rate of the compound on tumor cell growth was considered to be 0 under this condition.

$$\text{Inhibition rate} = 100 \times (Lum_{max} - Lum_{compound})/(Lum_{max} - Lum_{min})\%$$

wherein $Lum_{max}$ refers to the luminescence signal value of the well containing cells and 0.25% v/v DMSO, and $Lum_{compound}$ refers to the luminescence signal value of the well containing the compound and cells. $Lum_{min}$ refers to the luminescence signal value of the well containing the medium and 0.25% v/v DMSO.

The growth inhibition of the compounds of the present disclosure on tumor cells was determined by the above test, and an $IC_{50}$ value was determined.

Table 2. $IC_{50}$ of example compounds on inhibition of tumor cell growth

| Example compound number | DLD-1 BRCA2 (-/-) $IC_{50}$ (nM) | DLD-1 parent cells $IC_{50}$ (nM) |
|---|---|---|
| Compound I | ++++ | - |
| Compound II | ++++ | - |
| Compound III | ++++ | |

In the above table, the symbols used to indicate the inhibitory activity represent the following meanings:

"++++" indicates that the $IC_{50}$ range of the test compound for cell inhibitory activity is: $IC_{50}$ < 200 nM.
"+++" indicates that the $IC_{50}$ range of the test compound for cell inhibitory activity is: 200 nM ≤ $IC_{50}$ < 500 nM.
"++" indicates that the $IC_{50}$ range of the test compound for cell inhibitory activity is: 500 nM ≤ $IC_{50}$ < 1000 nM.
"+" indicates that the $IC_{50}$ range of the test compound for cell inhibitory activity is: 1000 nM ≤ $IC_{50}$ < 10000 nM.
"-" indicates that the $IC_{50}$ range of the test compound for cell inhibitory activity is: $IC_{50}$ ≥ 10000 nM.

As tested, the compounds of the present disclosure have good inhibitory effect on tumor cells with BRCA2 mutation, and have good selectivity.

**Test Example 3. Experiment on Inhibition of Compounds on Cellular MMEJ Pathway**

**Brief introduction to experimental principle:** POLQ is a key protein in the repair process of cellular MMEJ. The NanoLuciferase MMEJ repair reporter system was transferred into HEK293T cells. When the MMEJ repair pathway was normally performed in the cells, the NanoLuciferase reporter protein was correctly expressed, and the cell luminescence signal could be detected. The decrease in cell luminescence was measured using a BMG multifunctional microplate reader from BMG LABTECH to reflect the inhibition of the compound on the cellular MMEJ pathway.

**Experimental instruments:** ESCD Incucyte viable cell imaging system, Labcyte Echo 655 pipetting system, BMG LABTECH BMG multifunctional microplate reader, and Invitrogen Neon transfection system.

**Experimental materials:**

| Reagent | Brand | Catalog No. |
|---|---|---|
| MMEJ luciferase substrate | Synthesized by ICE Bioscience | / |
| HEK293T cell | ATCC | CRL-3216 |
| Nano-Glo Luciferase Assay kit | Promega | N1130 |
| DMEM | Gibco | 11995-065 |
| DMSO | Solarbio | D8371 |
| DPBS | Gibco | 14190250 |
| TrypL Express | Gibco | 25200-072 |

(continued)

| Reagent | Brand | Catalog No. |
|---|---|---|
| 384-well plate | Corning | 3764 |
| Neon™ Transfection System 100 μL Kit | Invitrogen | MPK10096 |

**Experimental methods:** The test compound was diluted to different concentrations with dimethyl sulfoxide (DMSO) using an Echo 655 pipetting system and transferred to a 384-well plate. In the reaction system, the compound was serially diluted 3-fold from the final concentration of 10 μM. The final concentration of DMSO was 0.1%. HEK293T cells were collected, an MMEJ luciferase substrate was transferred to the cells using a Neon transfection system, and the transfected HEK293T cells at 4000 cells/well were diluted with a DMEM medium containing 10% FBS and added to a 384-well plate (25 μL/well). After the compound and the cells were incubated in an incubator at 37 °C with 5% $CO_2$ for 24 h, 40 μL/well of a NanoGlo substrate buffer was added to measure the growth inhibition of the compound on tumor cells, and the inhibition rate and half inhibitory concentration ($IC_{50}$) were calculated.

**Data analysis:**

The compound inhibition rate was calculated and fitted using XLfit software to give the $IC_{50}$ of the compounds.

A Blank well and a DMSO well were set in the experiment. 10 μM positive compound ART558 (doi: 10.1038/s41467-021-23463-8) was added to the blank well, and the compound inhibition rate was considered to be 100% under this condition. 0.1% DMSO was added to the DMSO well, and the compound inhibition rate was considered to be 0 under this condition.

$$\text{Compound inhibition rate (\%)} = (100 \times (\text{DMSO well - test compound well})/(\text{DMSO well - blank well}))\%$$

As tested, the compounds of the examples of the present disclosure have strong inhibitory activity on the POLQ-mediated MMEJ pathway in cells, and the compounds are expected to effectively inhibit the target POLQ and the related pathway MMEJ in tumors, thereby exerting corresponding pharmacological effects.

**Test Example 4. Determination of Metabolic Stability of Compounds in Hepatocytes**

The metabolic stability of the compounds of the present disclosure in hepatocytes was determined by the following test method.

I. Experimental materials and instruments

1. Caucasian human hepatocytes (Biopredic BQHPCH10), cynomolgus monkey hepatocytes (RILD HP-SXH-02M), Beagle dog hepatocytes (BioIVT M00205), SD rat hepatocytes (BioIVT M00005), and CD-1 mouse hepatocytes (BioIVT M00505)
2. AOPI stain (Nexcelom 200710-01-01)
3. Dexamethasone (NIFDC 100129-201506)
4. DPBS (10×) (Gibco by Life Technologies 2060570)
5. Fetal bovine serum (FBS) (Corning 35081001)
6. GlutaMAXTM-1 (100×) (Gibco by Life Technologies 2186980)
7. HEPES (Sigma RNBJ1276)
8. Human recombinant insulin (Gibco by Life Technologies 2090407)
9. Isotonic Percoll (GE Healthcare 10288259)
10. Verapamil (Sigma MKBV4993V)
11. Williams' Medium E (Sigma RNBJ3314)
12. AB Sciex API4000 liquid chromatography-mass spectrometry system

II. Experimental procedures

1. The hepatocyte thawing medium was prepared according to the information in the table below. 49.5 mL of Williams' Medium E and 0.5 mL of GlutaMAXTM-1 (100×) were mixed as an incubation medium. The hepatocyte thawing medium and incubation medium were pre-heated in a water bath at 37 °C for at least 15 min prior to use. A tube of hepatocytes stored at an ultra-low temperature was taken, and the hepatocytes were ensured to be at a frozen state at a low temperature prior to thawing. The hepatocytes were quickly placed in a water bath at 37 °C and gently shaken until all ice crystals were completely dispersed. The hepatocytes were sprayed with 70% ethanol and transferred into a biological safety cabinet. The contents of the tube of hepatocytes were poured into a centrifuge tube containing 50 mL of the thawing medium and centrifuged at 100 g for 10 min. After centrifugation, the thawing medium was aspirated, and a sufficient amount of the incubation medium was added to give a cell suspension with a cell density of about $1.5 \times 10^6$ cells/mL. Cellometer Vision was used to count hepatocytes and determine viable cell density; the viability of hepatocytes must be greater than 75%. The hepatocyte suspension was diluted by using the incubation medium to a viable cell density of $0.5 \times 10^6$ viable cells/mL.

| Reagent | Initial concentration | Final concentration | Volume of reagent |
|---|---|---|---|
| Williams' Medium E | - | - | 31.2 mL |
| Isotonic Percoll | - | 30% | 13.5 mL |
| DPBS (10×) | - | - | 1.5 mL |
| GlutaMAXTM-1(100×) | 200 mM/100× | 2 mM | 500 μL |
| HEPES | 1 M | 15 mM | 750 μL |
| FBS | - | 5% | 2.5 mL |
| Human recombinant insulin | 4 mg/mL | 4 μg/mL | 50 μL |
| Dexamethasone | 10 mM | 1 μM | 5 μL |

2. 247.5 μL of the viable cell suspension or medium was transferred to a 96-well deep-well plate, and the deep-well plate was vortexed and placed in an incubator and preheated for 10 min. Incubation of all the samples was performed in parallel duplicate. 2.5 μL of the 100 μM test compound or control drug verapamil was added to each well to start the reaction, and the deep-well plate was placed back in the vortex incubator. 25 μL of the incubation sample was taken at 0 min, 15 min, 30 min, 60 min, 90 min, and 120 min, separately, and 125 μL of acetonitrile containing an internal standard was added to terminate the reaction. The mixture was vortexed for 10 min and centrifuged at 3220 g at 4 °C for 30 min. After the centrifugation was completed, 100 μL of the supernatant was transferred to an injection plate, and 150 μL of pure water was added. The mixture was mixed well for LC-MS/MS analysis.

All data were calculated by Microsoft Excel software. The peak area was detected through an extracted ion chromatogram. The *in vitro* half-life ($t_{1/2}$) of the compound was determined by performing linear fitting of the natural logarithm of elimination percent of the compound and time.

*In vitro* half-life ($t_{1/2}$) was calculated by the slope:

*in vitro* $t_{1/2} = 0.693/k$

The *in vitro* intrinsic clearance (unit: μL/min/mg protein) was calculated using the following formula:

$$\textit{in vitro}\ CL_{int} = k \times \text{volume of incubation } (\mu L)/\text{amount of proteins (mg)}$$

$CL_{int}$ is the intrinsic clearance rate; *k* is an elimination rate constant; volume of incubation is the incubation volume (μL); amount of proteins is the amount of the protein (mg).

As tested, the compounds of the examples of the present disclosure are metabolically stable in hepatocytes of various species, are expected to be relatively stable *in vivo* through liver metabolism, and are relatively less affected by the first-pass effect of the liver.

**Test Example 5. Combined Use of Compounds of the Present Disclosure with Platinum Derivatives, DNA Damage Repair Pathway Inhibitors, Tubulin Inhibitors, or Topoisomerase Inhibitors**

**Experimental materials and instruments:**

0.25% trypsin, fetal bovine serum, IMDM medium, 1640 medium, McCoy's 5A (modified) medium, and DMEM medium purchased from Gibco; NCI-H82, SHP-77, and OVCAR3 cells purchased from ATCC; DLD-1 BRCA2 (-/-) and DLD1 cells purchased from Horizon Discovery; A2780, LNCAP, MDA-MB-436, SKOV3, KURAMOCHI, Capan-1, NCI-H23, and A549 cells purchased from Nanjing Cobioer; bovine insulin purchased from Yeasen Biotechnology; DMSO purchased from Sigma; 96-well non-transparent sidewall sterile culture plate purchased from Corning; Cell-Titer Glo

reagent purchased from Promega; olaparib, niraparib, talazoparib, cisplatin, carboplatin, SN-38, etoposide, paclitaxel, doxorubicin hydrochloride, AZD5305, and AZD7648 (7,9-dihydro-7-methyl-2-[(7-methyl[1,2,4]triazolo[1,5-A]pyridin-6-yl) amino]-9-(tetrahydro-2H-pyran-4-yl)-8H-purin-8-one)
purchased from MCE (MedChemExpress); RP3500 (Camonsertib) purchased from Selleck; Thermo Fisher thermostatic cell incubator; Eppendorf centrifuge ; Envision microplate reader.

**Cell culture:**

DLD-1 colorectal adenocarcinoma cells, DLD-1 BRCA2 (-/-) colorectal adenocarcinoma cells, SHP-77 human small cell lung cancer cells, NCI-H82 human small cell lung cancer cells, LNCAP prostate cancer cells, KURAMOCHI human ovarian cancer cells, A2780 human ovarian cancer cells, and NCI-H23 non-small cell lung cancer cells were cultured in a 1640 medium containing 10% fetal bovine serum. SKOV3 human ovarian cancer cells were cultured in a McCoy's 5A (modified) medium containing 10% fetal bovine serum. OVCAR3 ovarian cancer cells were cultured in a 1640 medium containing 20% fetal bovine serum and 10 μg/mL bovine insulin. MDA-MB-436 breast cancer cells (natural BRCA1 mutation) and A549 non-small cell lung cancer cells were cultured in a DMEM medium containing 10% fetal bovine serum. Capan-1 human pancreatic cancer cells (natural BRCA2 mutation) were cultured in an IMDM medium containing 20% fetal bovine serum. The cells were all cultured at 37 °C with 5% $CO_2$. Cells at the logarithmic growth phase were available for the experiment.

**Experimental methods:**

100 μL of the cell medium was added to rows A and H and columns 1 and 12 of a 96-well cell culture plate. Cultured cells were resuspended (adherent cells were digested with trypsin) and counted to give information on cell density and viability. The dilution ratio of the resuspended cells required for inoculating a fixed number of cells into 80 μL of the medium in each culture well was calculated based on the cell density information. After the resuspended cells were diluted with the corresponding medium, 80 μL of the diluted cells were transferred to columns 2-11 of rows B-G of a 96-well cell culture plate using an Eppendorf multi-channel pipette (60 wells in total for each plate). The cell culture plate was incubated overnight in an incubator at 37 °C with 5% $CO_2$.

The test compound and the combination compound were dissolved in DMSO to give a 10 mM solution for later use. In the experiment, the compound was further diluted to the corresponding concentration according to the concentration designed in the experiment, and based on this concentration, serial gradient dilution was performed. Matrix study was performed at 8 or 7 dose concentrations of the test compound (the lowest dose concentration was 0, and other specific concentration values were shown in FIG. 1) and 7 or 8 dose concentrations of the combination compound (the lowest dose concentration was 0, and other specific concentration values were shown in FIG. 1). 10 μL of each of the diluted combination compound and test compound was added to the corresponding cell plate well, such that the final concentration of DMSO was 0.25%. The test compound and the cells were incubated in an incubator at 37 °C with 5% $CO_2$ for another 7 days, Cell Titer Glo was added at 50 μL/well, and the luminescence value of each well was read using an Envision microplate reader.

The luminescence value of the well containing only the cell medium was taken as the background ($RLU_{background}$, corresponding cell survival rate to be 0%), the cell well containing only 0.25% DMSO without the compound was taken as the maximum signal ($RLU_{DMSO}$, corresponding cell survival rate to be 100%), and the luminescence values of the remaining wells containing cells and compounds at different concentrations were taken as $RLU_{sample}$. The survival rate of each cell well was calculated as follows:

$$\text{Survival rate (\%)} = (RLU_{sample} - RLU_{background})/(RLU_{DMSO} - RLU_{background}) \times 100\%$$

The interaction between drugs (synergistic, independent, or antagonistic) was statistically analyzed by the Bliss Independence model using Combenefit software (Bliss, C.I., Bacteriol. Rev., 1956, 20, 243-258). The model assumes that each drug acts independently. The theoretical expected action curve of the combination was calculated from the following equation:

$$\text{Bliss effect} = \text{effect A} + \text{effect B} - \text{effect A} \times \text{effect B}$$

wherein effect A and effect B are the effects of drugs A and B alone at specific concentrations.

The Bliss effect is the expected effect when the combination of the two drugs is added precisely. A negative Bliss score ($\delta$ = observed effect - Bliss effect) indicates an antagonistic effect between the drugs if the observed effect (i.e., the experimentally measured combined effect) is less than the Bliss effect. A positive Bliss score indicates a synergistic effect between the drugs if the observed effect is greater than the Bliss effect. A Bliss score equal to zero indicates independence

of the drugs if the observed effect is equal to the Bliss effect.

**Experimental results:**

As shown in Table 3 and FIG. 1, compound I showed a synergistic effect when used in combination with PARP inhibitors; the sodium salt of compound I showed a synergistic effect when used in combination with PARP inhibitors, the DNA-PK inhibitor, ATR inhibitors, platinums, tubulin inhibitors, or topoisomerase inhibitors, respectively; compound II or the sodium salt of compound II showed a synergistic effect when used in combination with PARP inhibitors; compound III showed a synergistic effect when used in combination with the PARP inhibitor.

Table 3. Effects of combined use of compounds of the present disclosure with DNA damage repair pathway inhibitors, platinums, tubulin inhibitors, or topoisomerase inhibitors

| Compound | Type of combination drug | Name of combination drug | Cell model | Combined effect |
|---|---|---|---|---|
| Compound I | PARP inhibitor | Olaparib | DLD-1 BRCA2 (-/-) | Synergistic |
| Sodium salt of compound I | PARP inhibitor | Niraparib | DLD-1 BRCA2 (-/-) | Synergistic |
| Sodium salt of compound I | PARP inhibitor | AZD5305 | DLD-1 BRCA2 (-/-) | Synergistic |
| Sodium salt of compound I | PARP inhibitor | Olaparib | MDA-MB-436 | Synergistic |
| Compound I | PARP inhibitor | Niraparib | MDA-MB-436 | Synergistic |
| Sodium salt of compound I | PARP inhibitor | Niraparib | MDA-MB-436 | Synergistic |
| Sodium salt of compound I | PARP inhibitor | AZD5305 | MDA-MB-436 | Synergistic |
| Sodium salt of compound I | DNA-PK inhibitor | AZD7648 | MDA-MB-436 | Synergistic |
| Sodium salt of compound I | PARP inhibitor | Olaparib | Capan-1 | Synergistic |
| Sodium salt of compound I | PARP inhibitor | Niraparib | Capan-1 | Synergistic |
| Sodium salt of compound I | PARP inhibitor | Talazoparib | Capan-1 | Synergistic |
| Sodium salt of compound I | ATR inhibitor | RP3500 | Capan-1 | Synergistic |
| Sodium salt of compound I | ATR inhibitor | RP3500 | NCIH23 | Synergistic |
| Sodium salt of compound I | PARP inhibitor | Talazoparib | NCIH23 | Synergistic |
| Sodium salt of compound I | PARP inhibitor | Olaparib | OVCAR3 | Synergistic |
| Sodium salt of compound I | PARP inhibitor | Niraparib | OVCAR3 | Synergistic |
| Sodium salt of compound I | PARP inhibitor | Talazoparib | OVCAR3 | Synergistic |
| Sodium salt of compound I | Platinum derivative | Carboplatin | OVCAR3 | Synergistic |
| Sodium salt of compound I | Platinum derivative | Cisplatin | A549 | Synergistic |

(continued)

| Compound | Type of combination drug | Name of combination drug | Cell model | Combined effect |
|---|---|---|---|---|
| Sodium salt of compound I | Topoisomerase II inhibitor | Etoposide | A549 | Synergistic |
| Sodium salt of compound I | Topoisomerase II inhibitor | Etoposide | A2780 | Synergistic |
| Sodium salt of compound I | Topoisomerase II inhibitor | Etoposide | NCI-H82 | Synergistic |
| Sodium salt of compound I | Topoisomerase I inhibitor | SN38 | DLD1 | Synergistic |
| Sodium salt of compound I | Topoisomerase II inhibitor | Doxorubicin hydrochloride | A2780 | Synergistic |
| Sodium salt of compound I | Topoisomerase II inhibitor | Doxorubicin hydrochloride | SKOV3 | Synergistic |
| Sodium salt of compound I | Topoisomerase II inhibitor | Doxorubicin hydrochloride | KURAMOCHI | Synergistic |
| Sodium salt of compound I | Tubulin inhibitor | Paclitaxel | A2780 | Synergistic |
| Sodium salt of compound I | Tubulin inhibitor | Paclitaxel | SKOV3 | Synergistic |
| Sodium salt of compound I | Tubulin inhibitor | Paclitaxel | KURAMOCHI | Synergistic |
| Compound II | PARP inhibitor | Olaparib | DLD-1 BRCA2 (-/-) | Synergistic |
| Sodium salt of compound II | PARP inhibitor | Olaparib | MDA-MB-436 | Synergistic |
| Compound II | PARP inhibitor | Niraparib | MDA-MB-436 | Synergistic |
| Sodium salt of compound II | PARP inhibitor | Niraparib | MDA-MB-436 | Synergistic |
| Compound III | PARP inhibitor | Olaparib | DLD-1 BRCA2 (-/-) | Synergistic |

**Test Example 6. Combined Use of Compounds of the Present Disclosure with Radiotherapy Experimental materials and instruments:**

0.25% trypsin, fetal bovine serum, 1640 medium, and DMEM medium purchased from Gibco; MDA-MB-436 (natural BRCA1 mutation), SHP-77, and OVCAR3 cells purchased from ATCC; DLD-1 cells purchased from Horizon Discovery; NUGC4, LNCAP, and NCI-H460 cells purchased from Nanjing Cobioer; bovine insulin purchased from Yeasen Biotechnology; DMSO purchased from Sigma; 96-well non-transparent sidewall sterile culture plate purchased from Corning; Cell-Titer Glo reagent purchased from Promega; X-ray irradiator CellRad purchased from Precision X-Ray; Thermo Fisher thermostatic cell incubator ; Eppendorf centrifuge ; Envision microplate reader .

**Cell culture:**

DLD-1 colorectal adenocarcinoma cells, NUGC4 gastric cancer cells, NCI-H460 non-small cell lung cancer cells, SHP-77 small cell lung cancer cells, and LNCAP prostate cancer cells were cultured in a 1640 medium containing 10% fetal bovine serum. MDA-MB-436 breast cancer cells were cultured in a DMEM medium containing 10% fetal bovine serum. OVCAR3 ovarian cancer cells were cultured in a 1640 medium containing 20% fetal bovine serum and 10 μg/mL bovine insulin. The cells were all cultured at 37 °C with 5% $CO_2$. Cells at the logarithmic growth phase were available for the experiment.

**Experimental methods:**

100 μL of the cell medium was added to rows A and H and columns 1 and 12 of a 96-well cell culture plate. Cultured cells were resuspended (adherent cells were digested with trypsin) and counted to give information on cell density and viability. The dilution ratio of the resuspended cells required for inoculating a fixed number of cells into 90 μL of the medium in each culture well was calculated based on the cell density information. After the resuspended cells were diluted with the corresponding medium, 90 μL of the diluted cells were transferred to columns 2-11 of rows B-G of a 96-well cell culture plate using an Eppendorf multi-channel pipette (60 wells in total for each plate). The cell culture plate was incubated overnight in an incubator at 37 °C with 5% $CO_2$.

The test compound was dissolved in DMSO to give a 10 mM stock solution for later use. In the experiment, the compound was further diluted to the corresponding concentration according to the concentration designed in the experiment, and based on this concentration, 4-fold serial gradient dilution was performed to give a total of 10 dose concentrations (the lowest dose was 0, and other specific concentration values were shown in FIG. 2). 10 μL of the diluted compound was added to the corresponding cell plate well, such that the final concentration of DMSO was 0.25%. The compound was incubated with the cells in an incubator at 37 °C with 5% $CO_2$ for 4 h, and then the cell plate was transferred to Cellrad for irradiation (abbreviated as IR) at different doses (see FIG. 2 for specific dose values). After the irradiation was completed, the cell plate was incubated in an incubator at 37 °C with 5% $CO_2$ for another 7 days, Cell Titer Glo was added at 50 μL/well, and the luminescence value of each well was read using an Envision microplate reader.

The luminescence value of the well containing only the cell medium was taken as the background ($RLU_{background}$, corresponding cell survival rate to be 0%), the cell well containing only 0.25% DMSO without the compound and the irradiation treatment was taken as the maximum signal ($RLU_{DMSO}$, corresponding cell survival rate to be 100%), and the luminescence values of the remaining wells containing cells and compounds at different concentrations were taken as $RLU_{sample}$. The survival rate of each cell well was calculated as follows:

$$\text{Survival rate (\%)} = (RLU_{sample} - RLU_{background})/(RLU_{DMSO} - RLU_{background}) \times 100\%$$

The interaction between the drug and irradiation (synergistic, independent, or antagonistic) was statistically analyzed by the Bliss Independence model using Combenefit software (Bliss, C.I., Bacteriol. Rev., 1956, 20, 243-258). The model assumes that the drug and irradiation act independently. The theoretical expected action curve of the combination was calculated from the following equation:

$$\text{Bliss effect} = \text{effect A} + \text{effect B} - \text{effect A} \times \text{effect B}$$

wherein effect A is the effect of drug A at a particular concentration. Effect B is the effect of irradiation at a particular dose.

The Bliss effect is the expected effect when the combination of the drug and irradiation is administered precisely. A negative Bliss score ($\delta$ = observed effect - Bliss effect) indicates an antagonistic effect between the drug and irradiation if the observed effect (i.e., the experimentally measured combined effect) is less than the Bliss effect. A positive Bliss score indicates a synergistic effect between the drug and irradiation if the observed effect is greater than the Bliss effect. A Bliss score equal to zero indicates independence of the drug and irradiation if the observed effect is equal to the Bliss effect.

**Experimental results:**

As shown in Table 4 and FIG. 2, there was a significant synergistic effect when the sodium salt of compound I was used in combination with irradiation.

Table 4. Effects of compound of the present disclosure in combination with irradiation

| Compound | Combination mode | Cell model | Combined effect |
|---|---|---|---|
| Sodium salt of compound I | IR | DLD-1 | Synergistic |
| Sodium salt of compound I | IR | MDA-MB-436 | Synergistic |
| Sodium salt of compound I | IR | NCI-H460 | Synergistic |
| Sodium salt of compound I | IR | NUGC4 | Synergistic |
| Sodium salt of compound I | IR | OVCAR3 | Synergistic |
| Sodium salt of compound I | IR | LNCAP | Synergistic |
| Sodium salt of compound I | IR | SHP-77 | Synergistic |

**Test Example 7. Efficacy of Combined Use of Sodium Salt of Compound I with Olaparib in DLD1 BRCA2-/- Xenograft Tumor Model**

**Experimental materials and methods:**

The anti-tumor activity of the sodium salt of compound I used in combination with olaparib (MCE, 136925) was studied using a DLD1 BRCA2-/- subcutaneous tumor model. 32 female NU/NU nude mice (6-8 weeks old, Beijing Vital River Laboratory Animal Technology Co., Ltd.) were subcutaneously inoculated with $1 \times 10^7$ human colorectal adenocarcinoma DLD1 BRCA2-/- cells (Horizon- HD105-007). When the tumors grew to 80-100 $mm^3$, the mice were randomly divided into four groups with eight mice per group: a first group treated with vehicle control (QD) by intragastric administration, a second group treated with a 10 mg/kg solution of the sodium salt of compound I (QD) by intragastric administration, a third group treated with a 50 mg/kg olaparib solution (QD) by intragastric administration, and a fourth group treated with a 10 mg/kg solution of the sodium salt of compound I (QD) by intragastric administration and a 50 mg/kg olaparib solution (QD) by intragastric administration. The vehicle of olaparib was a solution containing 10% DMSO, 50% 2-hydroxypropyl-β-cyclodextrin (Aladdin-F2223139, 60% aqueous solution), and 40% water, the vehicle of the sodium salt of compound I was 10% (w/v) Vitamin E TPGS (Hanhong Chemical-BH-GN0100619-22 1101) aqueous solution, and the vehicle of the vehicle control group was a mixture of the vehicle of olaparib and the vehicle of the sodium salt of compound I (mixed at a volume ratio of 1:1). Mice had free access to food and water throughout the experiment.

Note: w/v refers to (mass of solute/volume of solvent) × 100%.

**Tumor measurement and experimental indices:**

Tumor diameters were measured twice weekly using a vernier caliper. The tumor volume (V) was calculated using the following formula: $V = 0.5a \times b^2$, wherein a and b represent the long diameter and short diameter of the tumor, respectively.

Mouse body weight was measured twice weekly.

Two way ANOVA was used to compare the differences in tumor growth trend among groups, and a significant difference was considered when $p < 0.05$.

**Experimental results:**

As shown in FIG. 3, the tumor volume of mice in the group of the sodium salt of compound I used in combination with olaparib was significantly lower than that in the single drug group of the sodium salt of compound I or the single drug group of olaparib (* indicates $p < 0.05$; ** indicates $p < 0.01$; *** indicates $p < 0.001$; **** indicates $p < 0.0001$). Mice were in good condition throughout the experiment. The experimental results showed that the sodium salt of compound I used in combination with olaparib has a strong synergistic anti-tumor effect on human colorectal adenocarcinoma DLD1 BRCA2-/- cells with good safety.

**Test Example 8. Efficacy of Combined Use of Sodium Salt of Compound I with Olaparib in MDA-MB-436 Xenograft Tumor Model**

**Experimental materials and methods:**

The anti-tumor activity of the sodium salt of compound I used in combination with olaparib (MCE, 136925) was studied using a MDA-MB-436 subcutaneous tumor model. 24 female NOD-SCID mice (6-7 weeks old, Beijing Vital River Laboratory Animal Technology Co., Ltd.) were subcutaneously inoculated with $1 \times 10^7$ human breast cancer MDA-MB-436 cells (natural BRCA1 mutation, Nanjing Cobioer). When the tumors grew to 80-120 $mm^3$, the mice were randomly divided into four groups with six mice per group: a first group treated with vehicle control (QD) by intragastric administration, a second group treated with a 30 mg/kg solution of the sodium salt of compound I (QD) by intragastric administration, a third group treated with a 10 mg/kg olaparib solution (QD) by intragastric administration, and a fourth group treated with a 30 mg/kg solution of the sodium salt of compound I (QD) by intragastric administration and a 10 mg/kg olaparib solution (QD) by intragastric administration. The vehicle of olaparib was a solution containing 10% DMSO, 50% 2-hydroxypropyl-β-cyclodextrin (Aladdin-F2223139, 60% aqueous solution), and 40% water, the vehicle of the sodium salt of compound I was 10% (w/v) Vitamin E TPGS (Hanhong Chemical-BH-GN0100619-22 1101) aqueous solution, and the vehicle of the vehicle control group was a mixture of the vehicle of olaparib and the vehicle of the sodium salt of compound I (mixed at a volume ratio of 1:1). Mice had free access to food and water throughout the experiment.

Note: w/v refers to (mass of solute/volume of solvent) × 100%.

**Tumor measurement and experimental indices:**

Tumor diameters were measured twice weekly using a vernier caliper. The tumor volume (V) was calculated using the following formula: $V = 0.5a \times b^2$, wherein a and b represent the long diameter and short diameter of the tumor, respectively.

Mouse body weight was measured twice weekly.

Two way ANOVA was used to compare the differences in tumor growth trend among groups, and a significant difference was considered when $p < 0.05$.

**Experimental results:**

As shown in FIG. 4, the tumor volume of mice in the group of the sodium salt of compound I used in combination with olaparib was significantly lower than that in the single drug group of the sodium salt of compound I or the single drug group of olaparib (* indicates $p < 0.05$; ** indicates $p < 0.01$; *** indicates $p < 0.001$; **** indicates $p < 0.0001$). Except that one mouse in the vehicle control group had a cold body and abdominal distension, and was euthanized on day 15 of administration, other mice were in good condition throughout the experiment. The experimental results showed that the sodium salt of compound I used in combination with olaparib has a synergistic anti-tumor effect on human breast cancer MDA-MB-436 cells with good safety.

## Claims

1. A combination for preventing or treating cancer, comprising a compound of formula (A) or a pharmaceutically acceptable salt thereof or a pharmaceutical composition thereof and at least one anti-cancer therapeutic agent or radiotherapy, wherein the compound of formula (A) has the following structure:

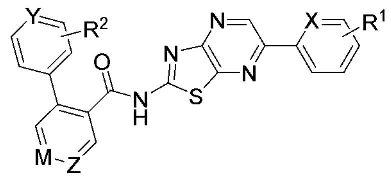

(A)

wherein X is selected from CH or N;
Y is selected from CH or N;
Z is selected from N or $CR^3$;
M is selected from N or $CR^4$; and
at least one of Z and M is N;
$R^3$ is selected from H or $CH_3$;
$R^4$ is selected from H or $CH_3$;
$R^1$ is selected from halogen, cyano, =O, $C_1$-$C_6$ alkyl, $C_1$-$C_6$ alkoxy, or $C_3$-$C_6$ cycloalkyl;
$R^2$ is selected from halogen, cyano, $C_1$-$C_6$ alkoxy, $C_1$-$C_6$ alkyl, $C_2$-$C_6$ alkynyl, or $C_3$-$C_6$ cycloalkyl, wherein the $C_1$-$C_6$ alkoxy, $C_1$-$C_6$ alkyl, $C_2$-$C_6$ alkynyl, or $C_3$-$C_6$ cycloalkyl is optionally substituted with $R^{2a}$; and
$R^{2a}$ is selected from halogen or deuterium.

2. The combination according to claim 1, wherein Z is N, M is $CR^4$, and $R^4$ is $CH_3$.

3. The combination according to any one of claims 1-2, wherein $R^1$ is selected from cyano or $C_3$-$C_6$ cycloalkyl; or $R^1$ is selected from cyano or cyclopropyl.

4. The combination according to any one of claims 1-3, wherein $R^2$ is selected from halogen, $C_1$-$C_3$ alkoxy, $C_1$-$C_3$ alkyl, or cyano, wherein the $C_1$-$C_3$ alkoxy or $C_1$-$C_3$ alkyl is optionally substituted with $R^{2a}$; or $R^2$ is selected from halogen, $C_1$-$C_3$ alkoxy, $C_1$-$C_3$ alkyl, or cyano; or $R^2$ is selected from chlorine, methoxy, methyl, or cyano.

5. The combination according to claim 1, wherein the compound of formula (A) or the pharmaceutically acceptable salt thereof is selected from the following compounds or pharmaceutically acceptable salts thereof:

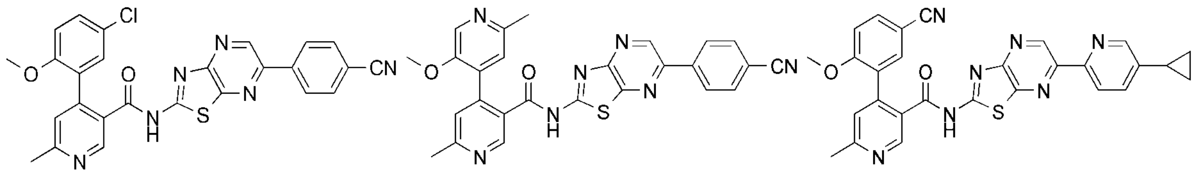

I II III .

6. The combination according to claim 5, wherein the compound of formula (A) or the pharmaceutically acceptable salt thereof is selected from the following compounds or pharmaceutically acceptable salts thereof:

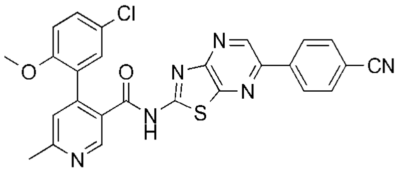

I .

7. The combination according to any one of claims 1-6, wherein the pharmaceutical composition comprises the compound of formula (A) or a pharmaceutically acceptable salt thereof and a pharmaceutically acceptable excipient.

8. The combination according to any one of claims 1-7, wherein the cancer is an HR-deficient cancer; or the cancer is cancer with reduced or absent BRCA gene expression, BRCA gene deficiency, or a reduced BRCA protein function; or the cancer is cancer with a BRCA mutation; or the cancer is cancer with BRCA1 and/or BRCA2 mutations; or the cancer is selected from colorectal cancer, lung cancer, ovarian cancer, breast cancer, gastric cancer, pancreatic cancer, or prostate cancer; or the cancer is selected from colorectal cancer, non-small cell lung cancer, small cell lung cancer, ovarian cancer, breast cancer, gastric cancer, pancreatic cancer, or prostate cancer; or the cancer is selected from colorectal adenocarcinoma, lung cancer, ovarian cancer, breast cancer, gastric cancer, pancreatic cancer, or prostate cancer; or the cancer is selected from colorectal adenocarcinoma, non-small cell lung cancer, small cell lung cancer, ovarian cancer, breast cancer, gastric cancer, pancreatic cancer, or prostate cancer.

9. The combination according to any one of claims 1-8, wherein the anti-cancer therapeutic agent is selected from one or more of a platinum derivative, a DNA damage repair pathway inhibitor, a topoisomerase inhibitor, or a tubulin inhibitor.

10. The combination according to claim 9, wherein the platinum derivative is selected from carboplatin, cisplatin, oxaliplatin, or satraplatin; or the platinum derivative is selected from carboplatin or cisplatin.

11. The combination according to claim 9, wherein the DNA damage repair pathway inhibitor is selected from a PARP inhibitor, an ATR inhibitor, or a DNA protein kinase (i.e., DNA-PK) inhibitor.

12. The combination according to claim 11, wherein the PARP inhibitor is selected from olaparib, niraparib, talazoparib, rucaparib, fluzoparib, pamiparib, veliparib, or AZD5305; or the PARP inhibitor is selected from olaparib, niraparib, talazoparib, or AZD5305; or the ATR inhibitor is selected from RP3500, ceralasertib, berzosertib, ART-0380, M-4344 (VX-803), M-1774, or elimusertib; or the ATR inhibitor is selected from RP3500; or the DNA protein kinase (i.e., DNA-PK) inhibitor is selected from AZD7648, CC-115, BR-101801, BR-2002, SRX-2523, or SN-39884; or the DNA protein kinase (i.e., DNA-PK) inhibitor is selected from AZD7648.

13. The combination according to claim 9, wherein the topoisomerase inhibitor is selected from irinotecan, etoposide, doxorubicin hydrochloride, or SN38; or the topoisomerase inhibitor is selected from etoposide, doxorubicin hydrochloride, or SN38.

14. The combination according to claim 9, wherein the tubulin inhibitor is selected from paclitaxel or docetaxel; or the tubulin inhibitor is selected from paclitaxel.

15. The combination according to any one of claims 1-14, wherein the compound of formula (A) or the pharmaceutically acceptable salt thereof or the pharmaceutical composition thereof and the at least one anti-cancer therapeutic agent

in the combination may be packaged separately or together.

**16.** Use of the combination comprising the compound of formula (A) or the pharmaceutically acceptable salt thereof or the pharmaceutical composition thereof and the at least one anti-cancer therapeutic agent or the radiotherapy according to any one of claims 1-15 in preventing or treating cancer.

**17.** Use of the combination of the compound of formula (A) or the pharmaceutically acceptable salt thereof or the pharmaceutical composition thereof and the at least one anti-cancer therapeutic agent or the radiotherapy according to any one of claims 1-15 in preparing a medicament for preventing or treating cancer.

**18.** A method for preventing or treating cancer in a mammal, comprising administering to a mammal, preferably a human, in need thereof the combination of the compound of formula (A) or the pharmaceutically acceptable salt thereof or the pharmaceutical composition thereof and the at least one anti-cancer therapeutic agent or the radiotherapy according to any one of claims 1-15.

**19.** The use according to claim 16 or 17 and the method according to claim 18, wherein the compound of formula (A) or the pharmaceutically acceptable salt thereof and the at least one anti-cancer therapeutic agent are each in the form of a pharmaceutical composition and may be administered simultaneously, sequentially, or at intervals; or the compound of formula (A) or the pharmaceutically acceptable salt thereof or the pharmaceutical composition thereof and the at least one anti-cancer therapeutic agent are each administered at intervals.

**20.** The use according to claim 16 or 17 and the method according to claim 18, wherein the compound of formula (A) or the pharmaceutically acceptable salt thereof or the pharmaceutical composition thereof and the at least one anti-cancer therapeutic agent are each administered according to the same or different administration regimens; or the compound of formula (A) or the pharmaceutically acceptable salt thereof or the pharmaceutical composition thereof and the at least one anti-cancer therapeutic agent are each administered according to different administration regimens.

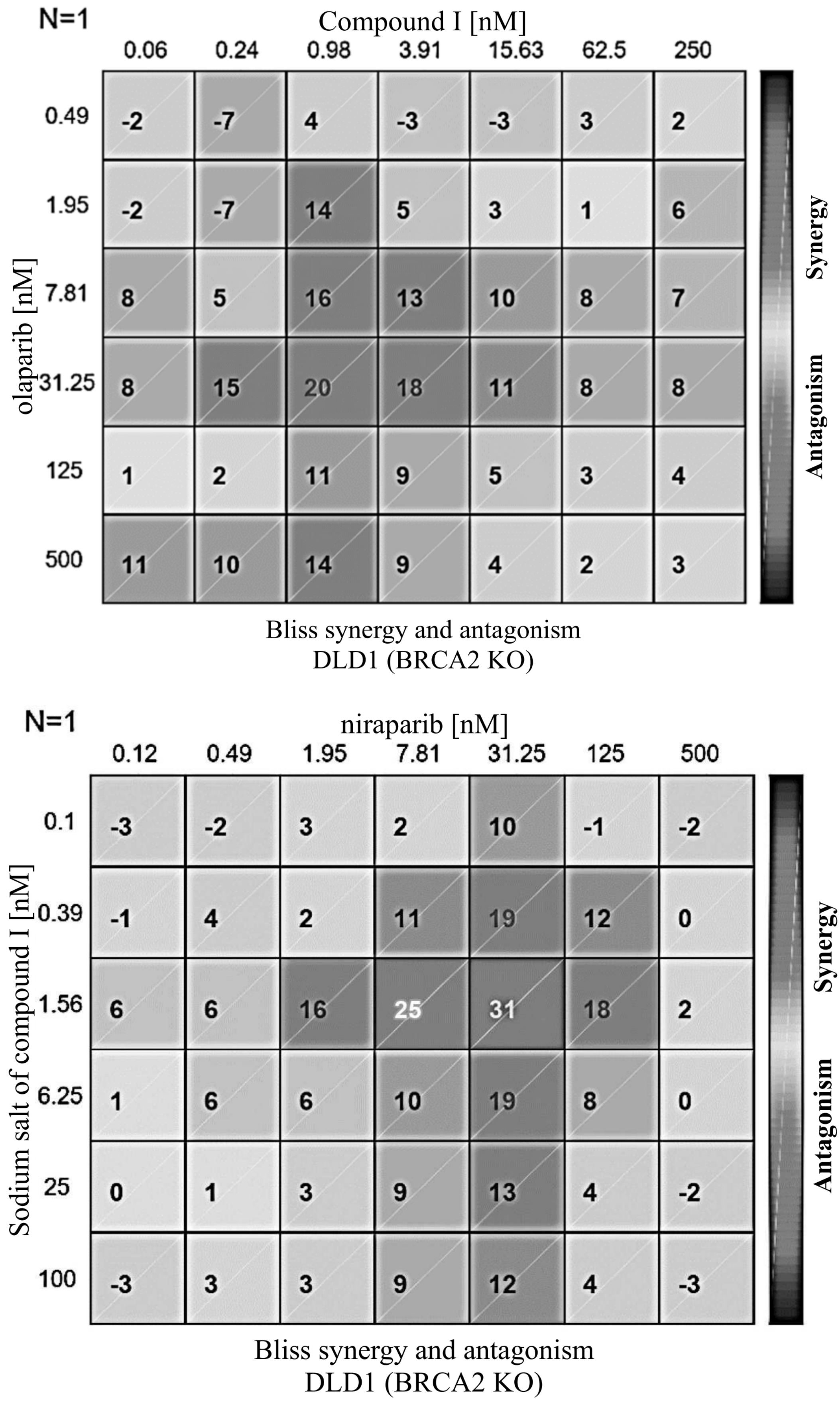
N=1
Compound I [nM]
0.06 0.24 0.98 3.91 15.63 62.5 250
olaparib [nM]
0.49: -2 -7 4 -3 -3 3 2
1.95: -2 -7 14 5 3 1 6
7.81: 8 5 16 13 10 8 7
31.25: 8 15 20 18 11 8 8
125: 1 2 11 9 5 3 4
500: 11 10 14 9 4 2 3
Synergy
Antagonism
Bliss synergy and antagonism
DLD1 (BRCA2 KO)
N=1
niraparib [nM]
0.12 0.49 1.95 7.81 31.25 125 500
Sodium salt of compound I [nM]
0.1: -3 -2 3 2 10 -1 -2
0.39: -1 4 2 11 19 12 0
1.56: 6 6 16 25 31 18 2
6.25: 1 6 6 10 19 8 0
25: 0 1 3 9 13 4 -2
100: -3 3 3 9 12 4 -3
Synergy
Antagonism
Bliss synergy and antagonism
DLD1 (BRCA2 KO)

FIG. 1

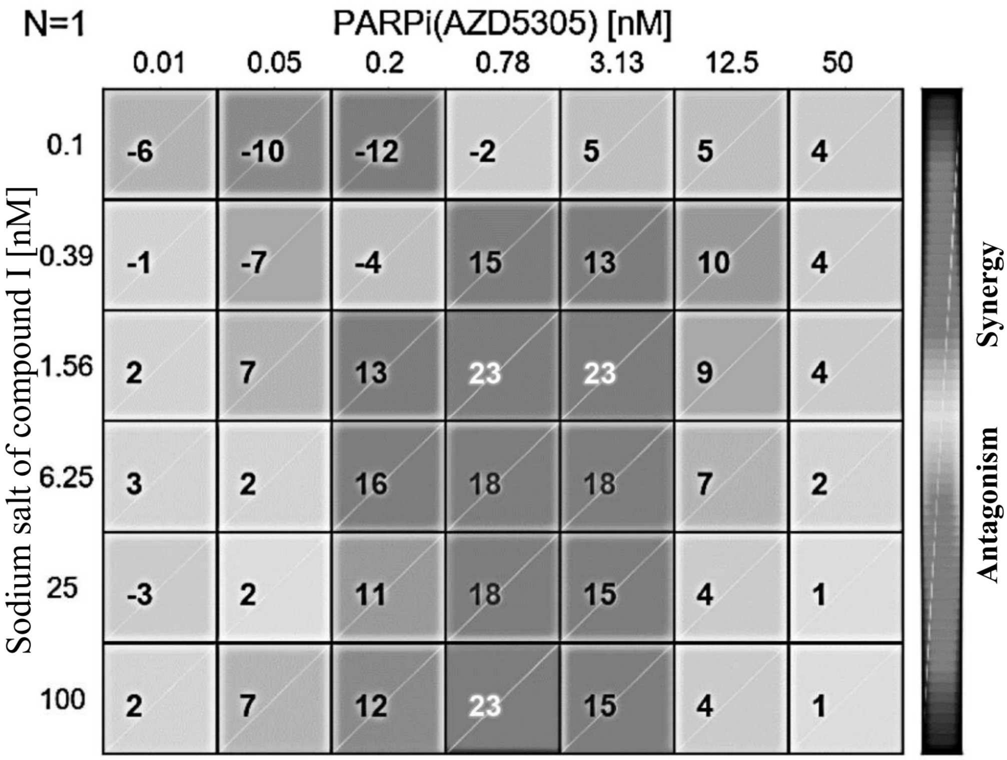


Bliss synergy and antagonism
DLD1 (BRCA2 KO)

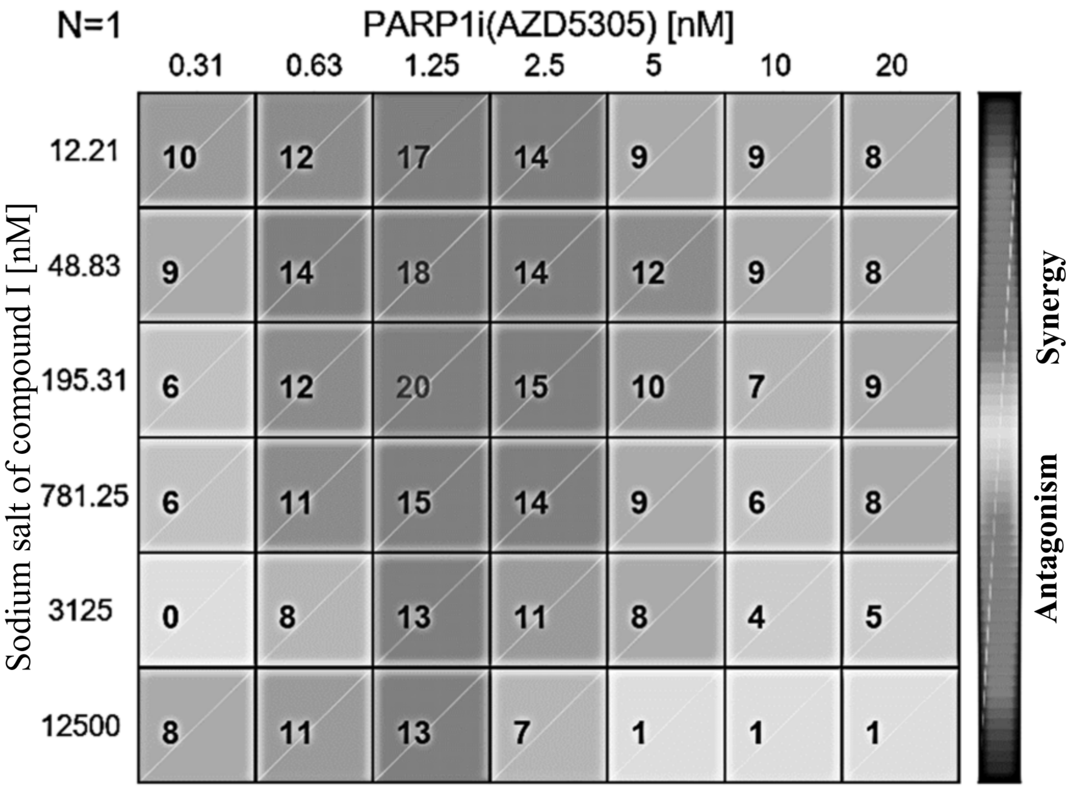


Bliss synergy and antagonism
MDAMB436

**FIG. 1 (Continued)**

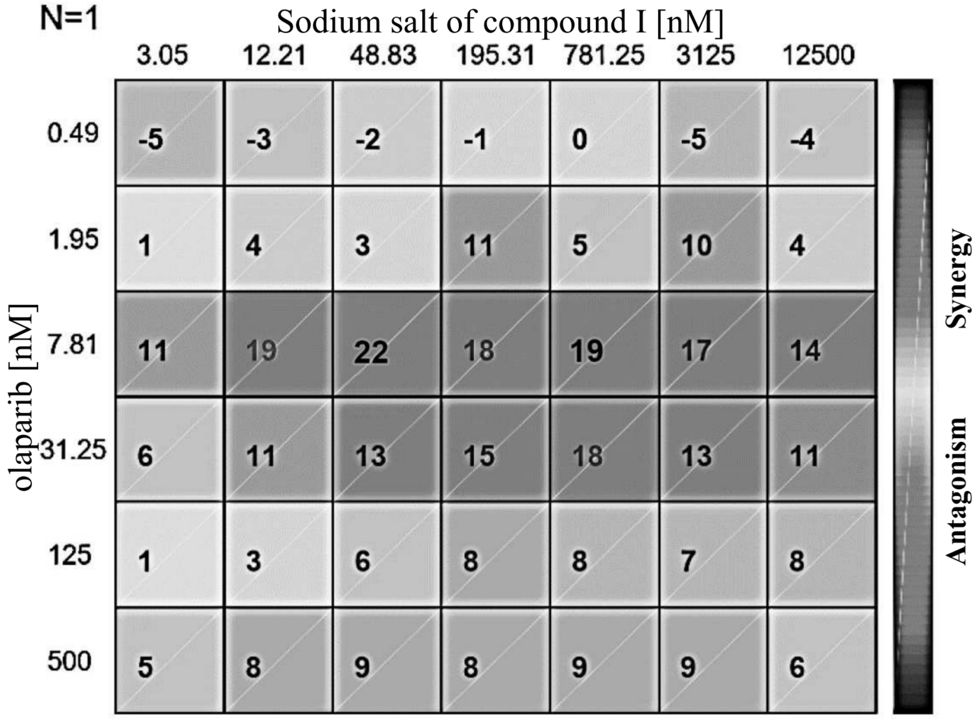


Bliss synergy and antagonism
MDA-MB-436

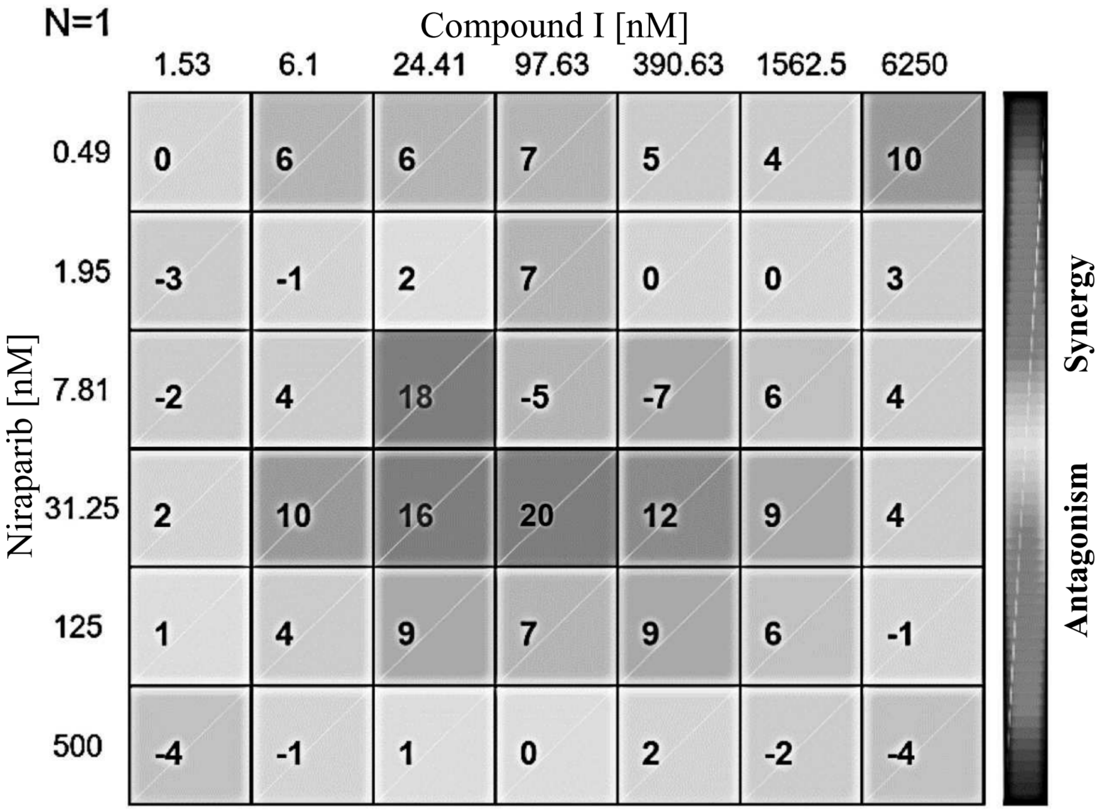


Bliss synergy and antagonism
MDA-MB-436

**FIG. 1 (Continued)**

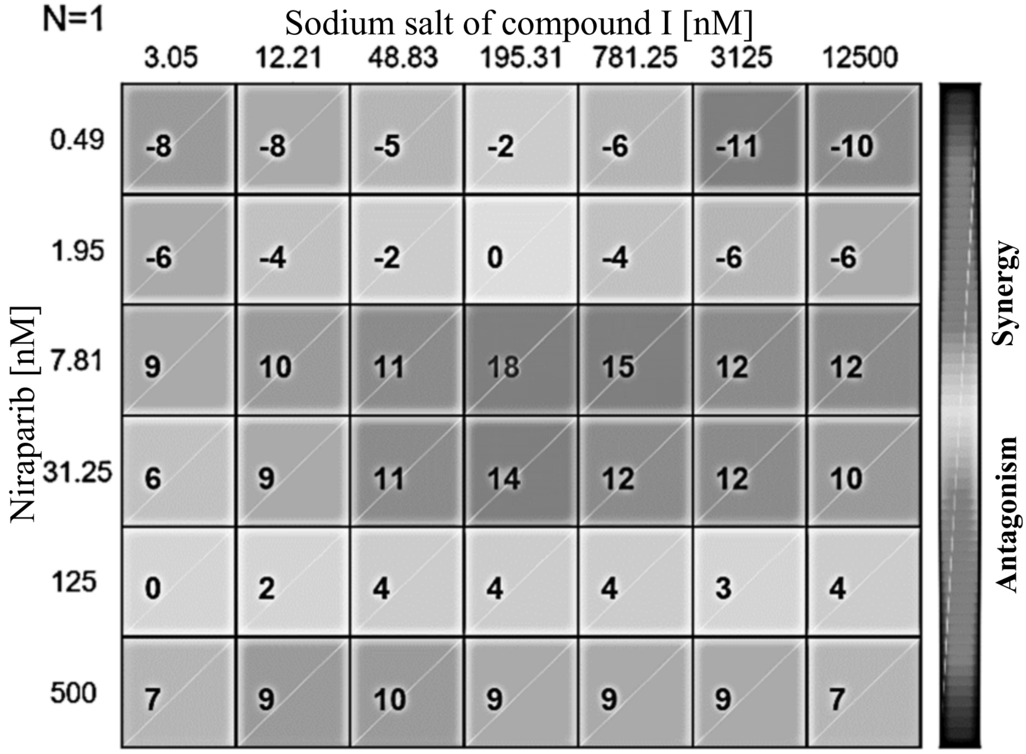


Bliss synergy and antagonism
MDA-MB-436

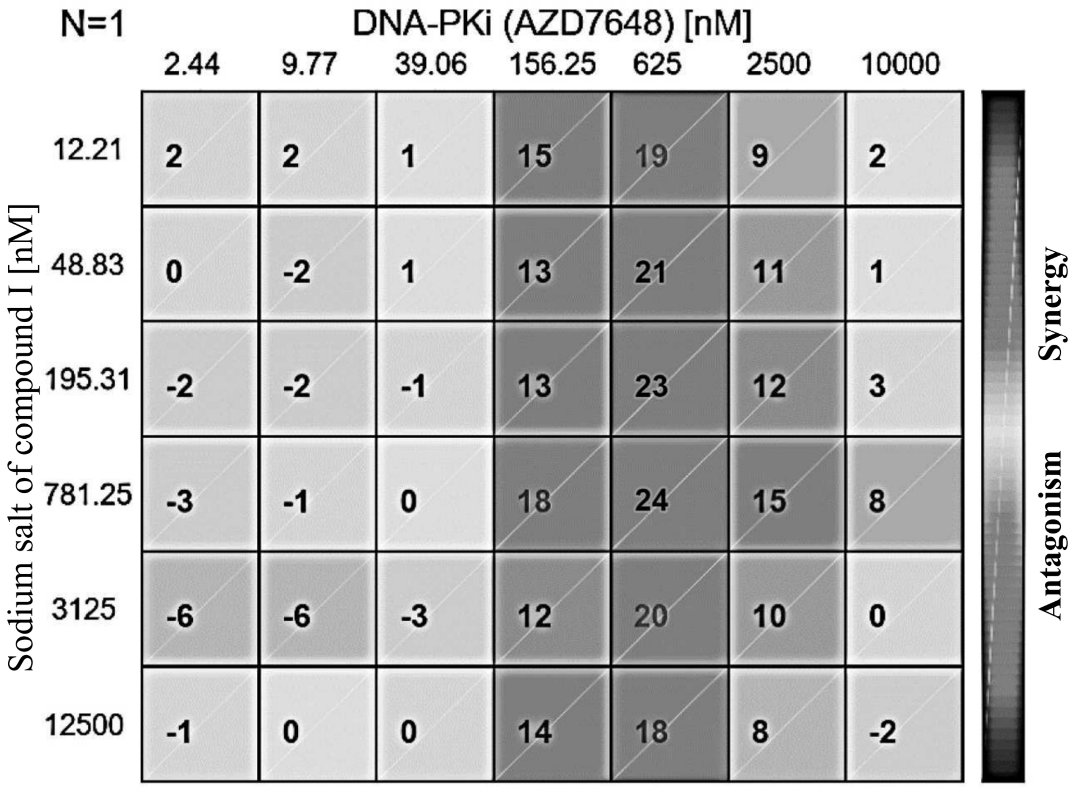


Bliss synergy and antagonism
MDA-MB-436

**FIG. 1 (Continued)**

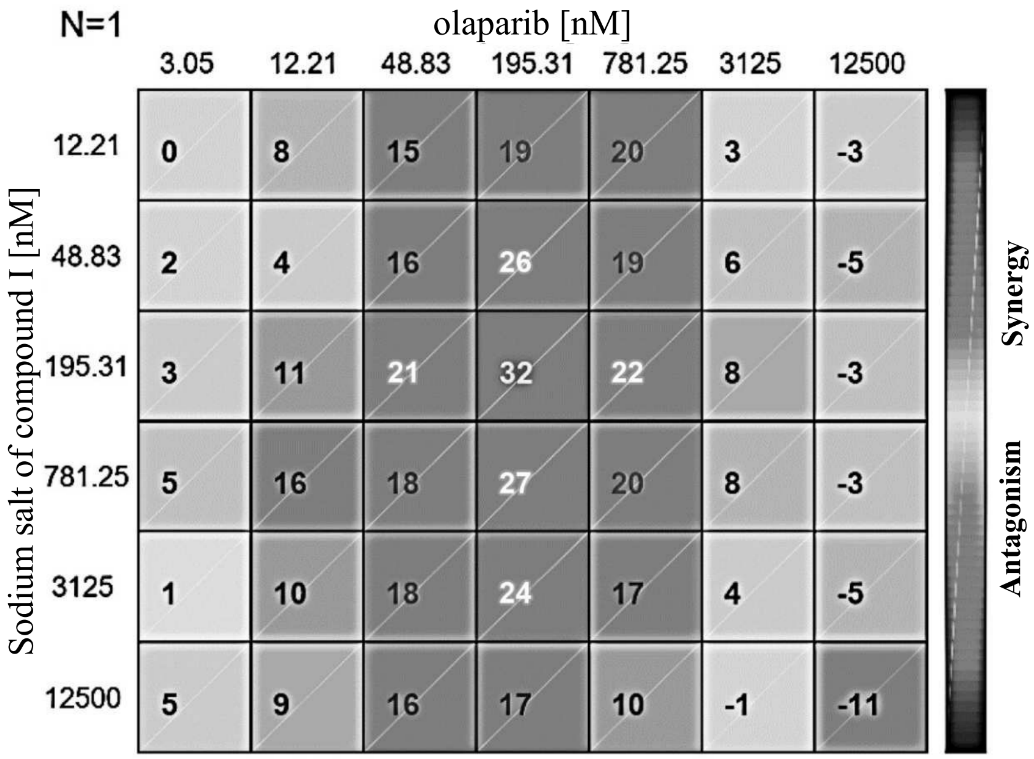


Bliss synergy and antagonism
Capan1

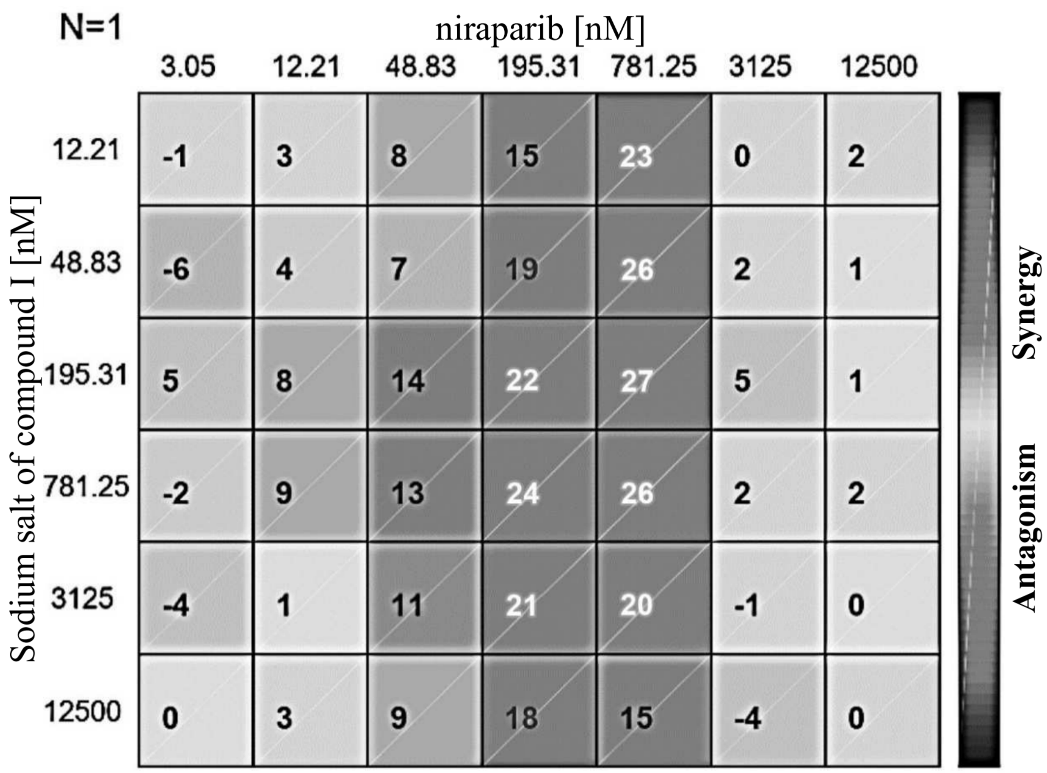


Bliss synergy and antagonism
Capan1

**FIG. 1 (Continued)**

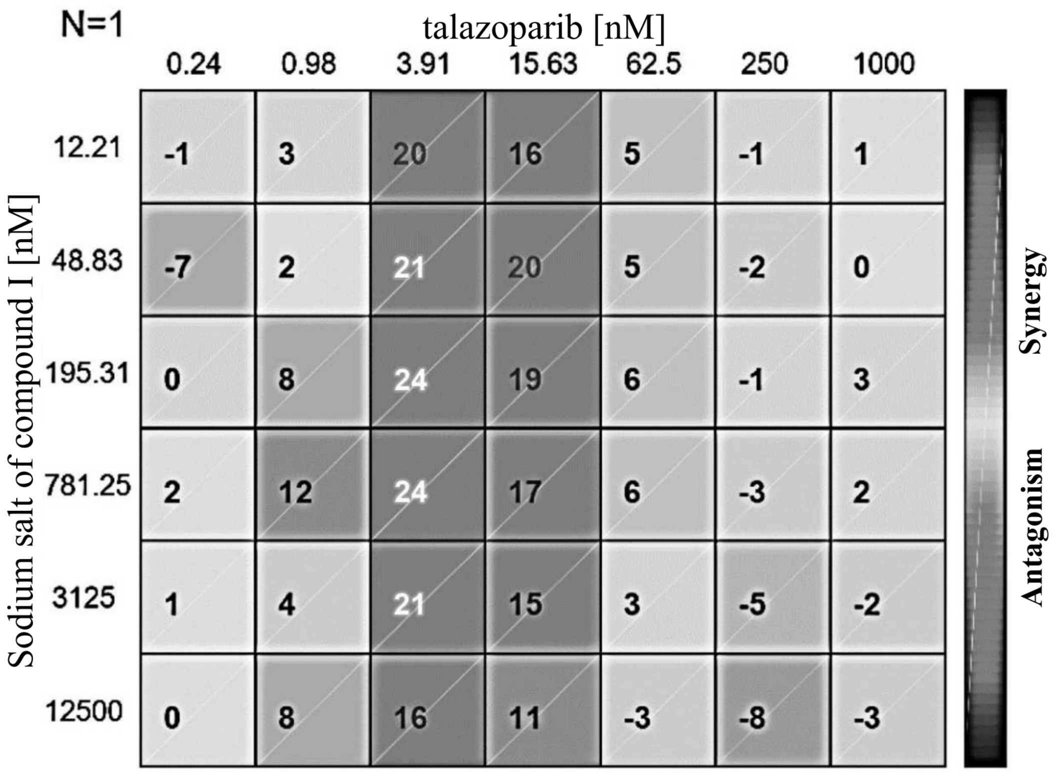


Bliss synergy and antagonism
Capan1

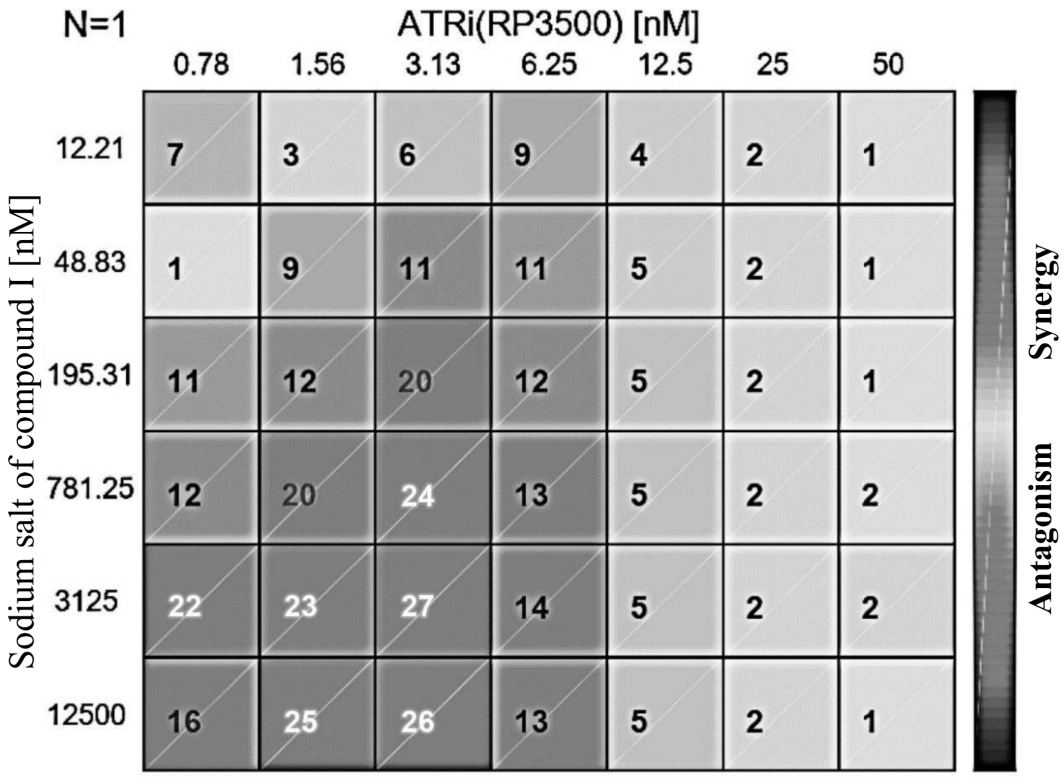


Bliss synergy and antagonism
NCIH23

**FIG. 1 (Continued)**

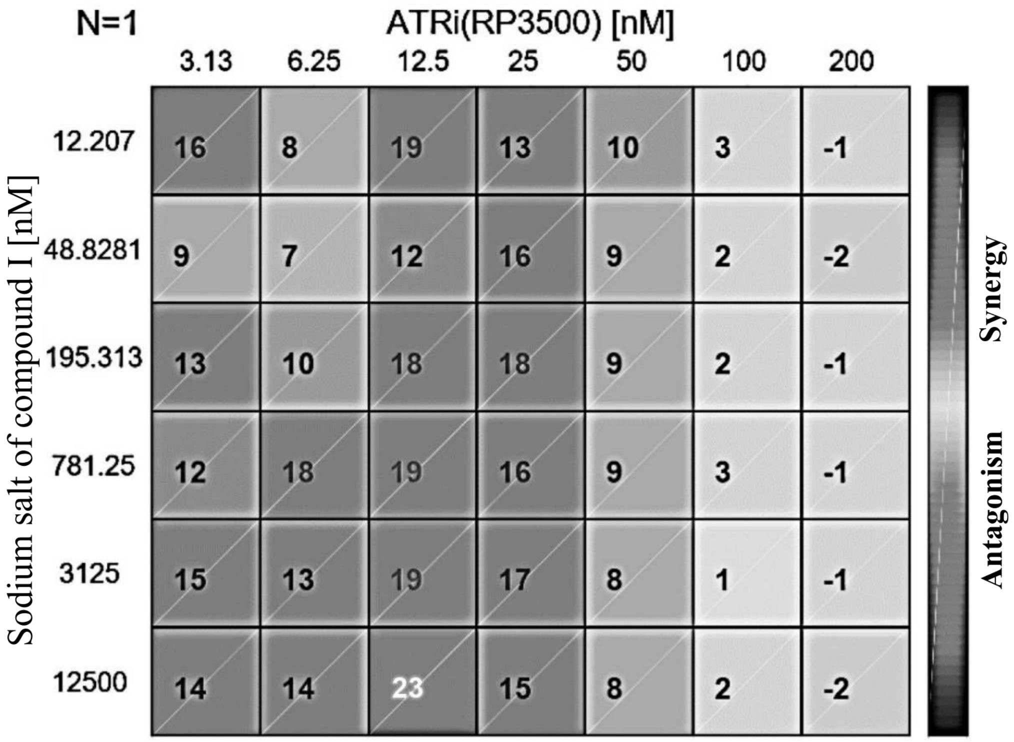


Bliss synergy and antagonism
Capan1

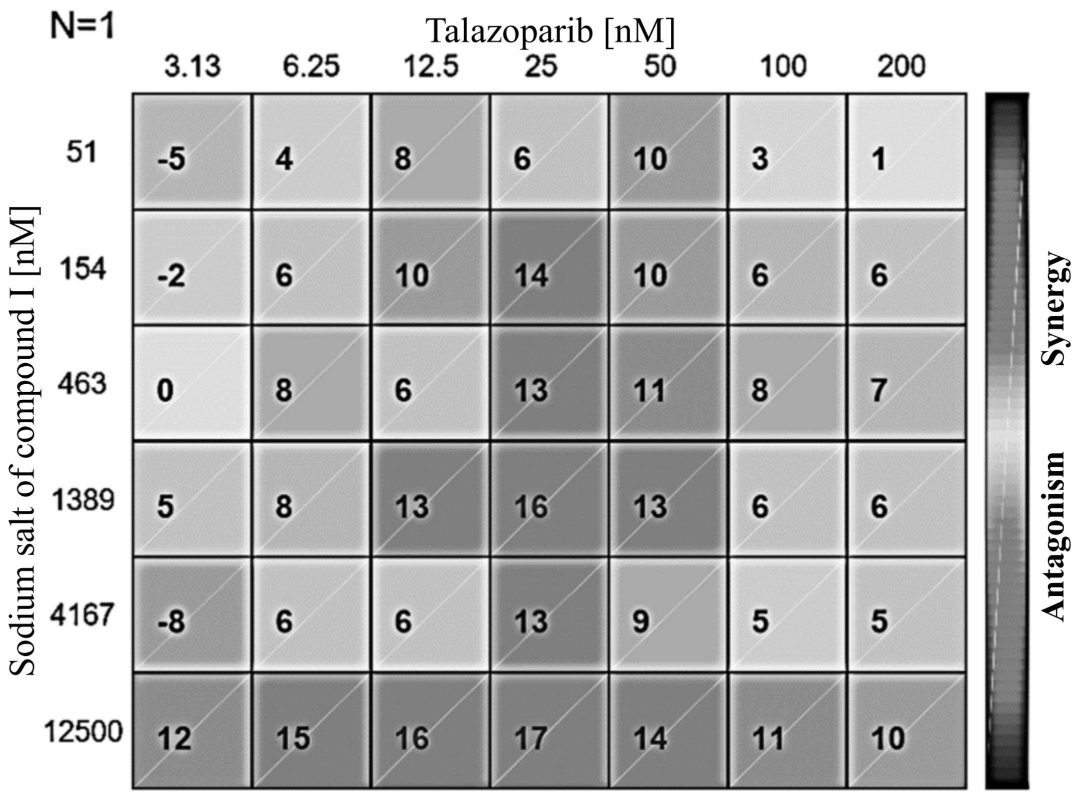


Bliss synergy and antagonism
NCIH23

**FIG. 1 (Continued)**

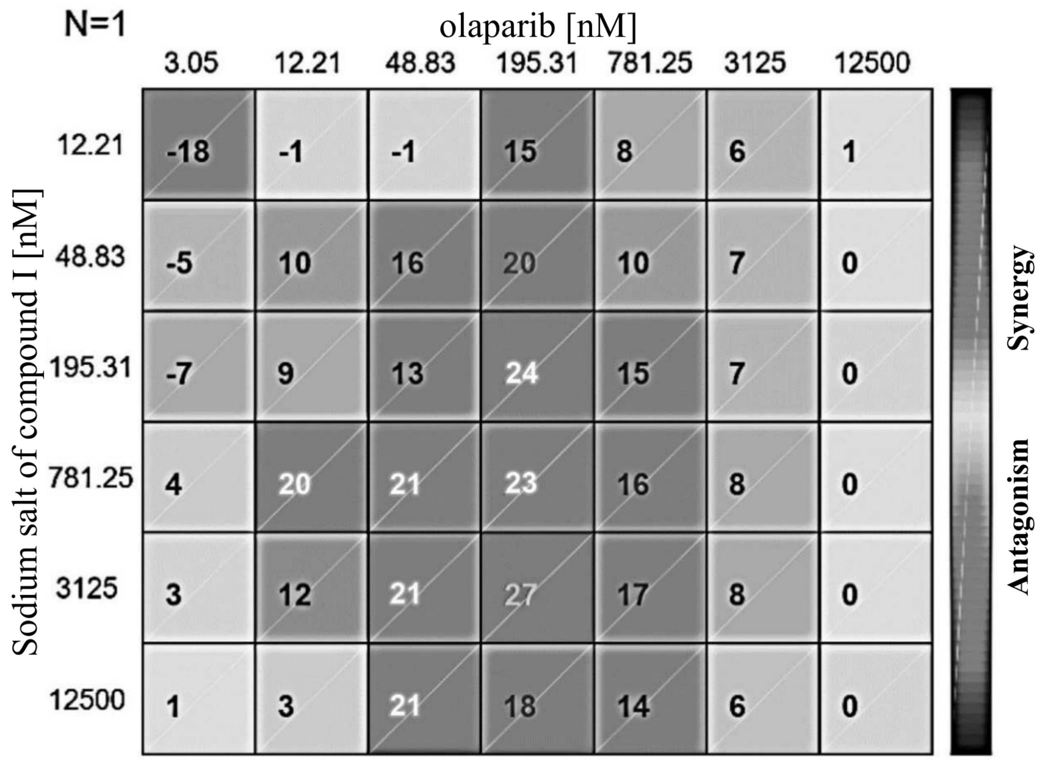


Bliss synergy and antagonism
OVCAR3

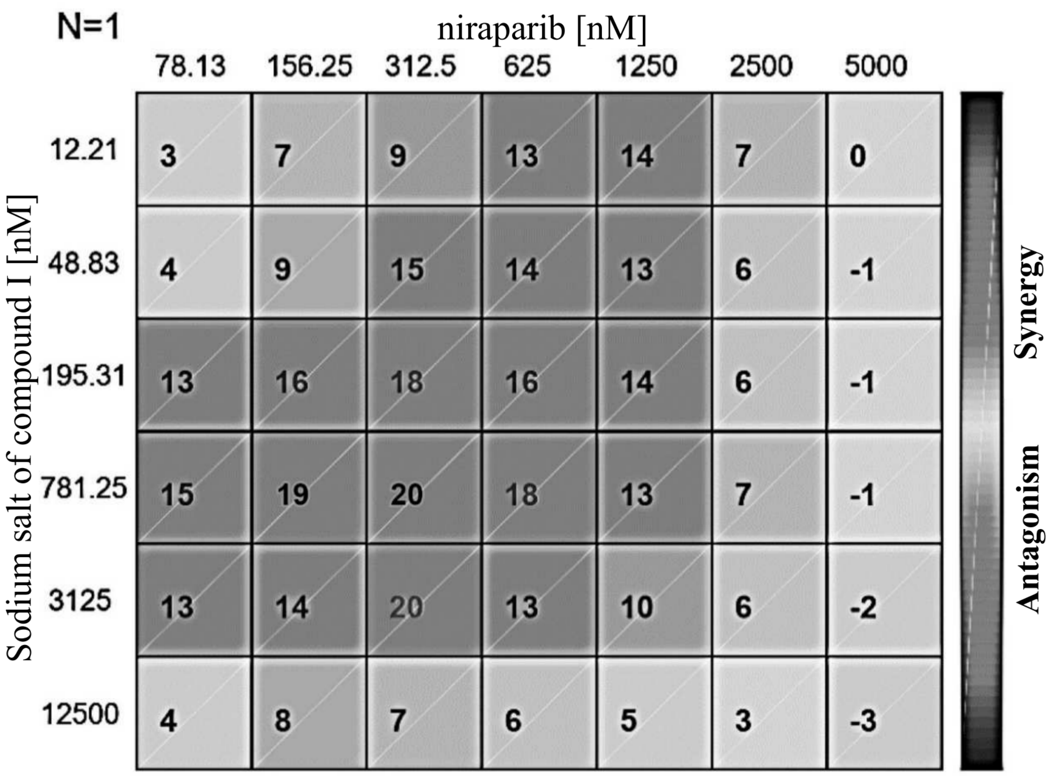


Bliss synergy and antagonism
OVCAR3

**FIG. 1 (Continued)**

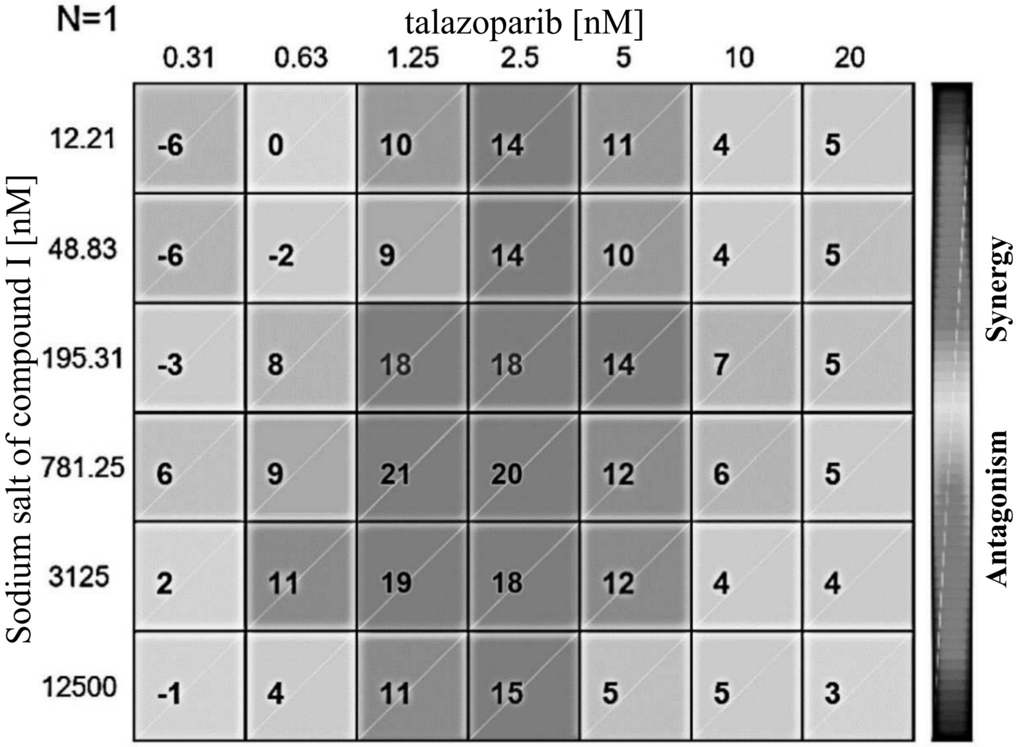


Bliss synergy and antagonism
OVCAR3

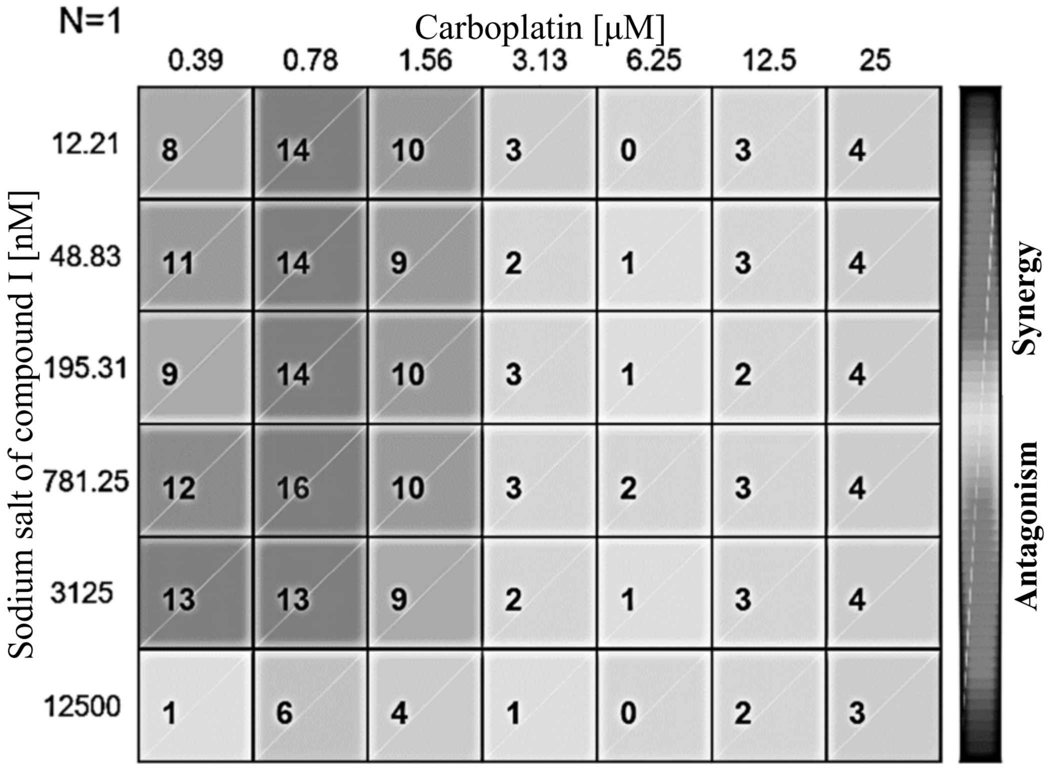


Bliss synergy and antagonism
OVCAR3

**FIG. 1 (Continued)**

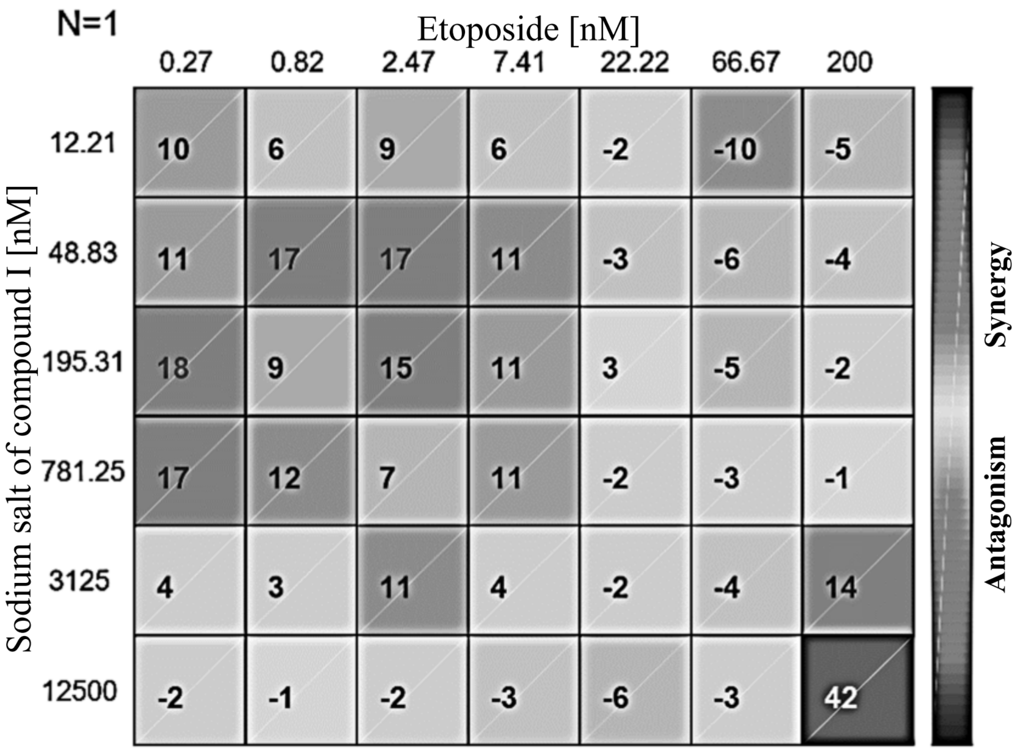


Bliss synergy and antagonism
A549

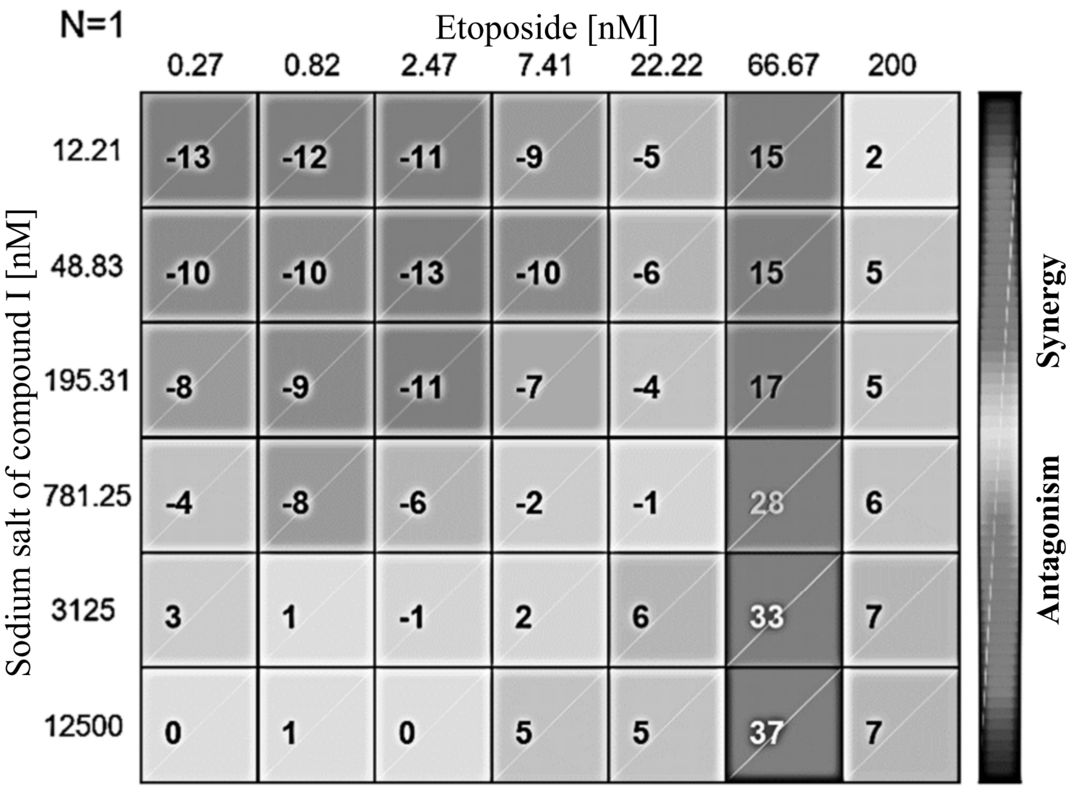


Bliss synergy and antagonism
A2780

FIG. 1 (Continued)

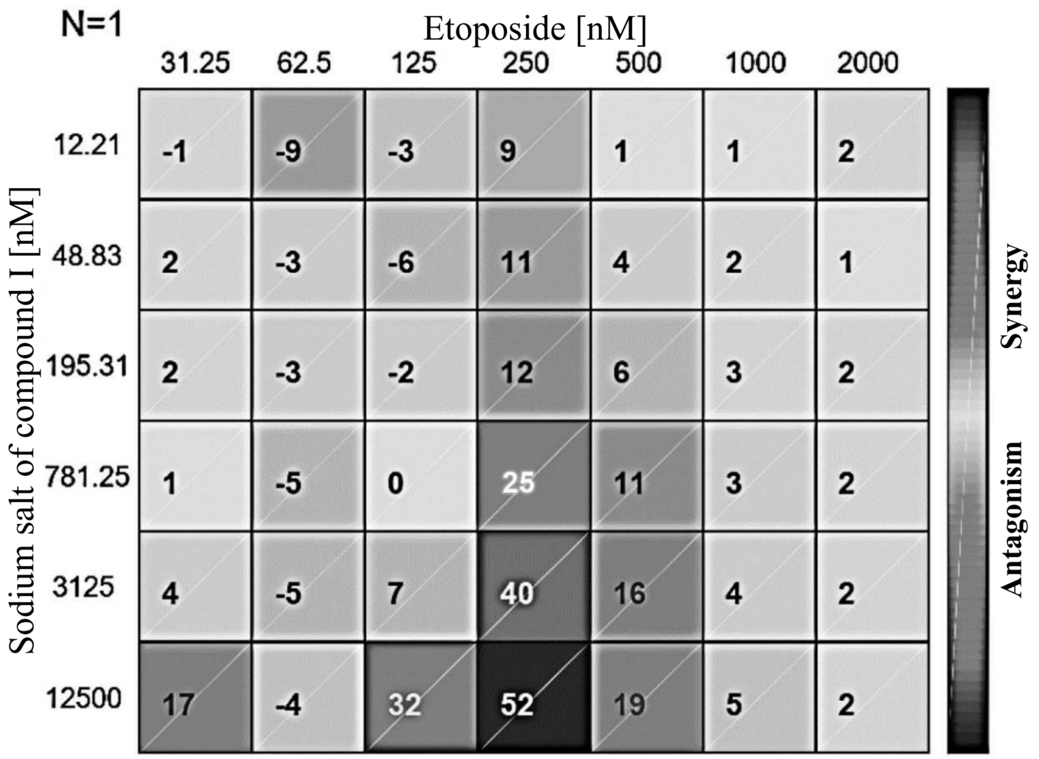


Bliss synergy and antagonism
NCI-H82

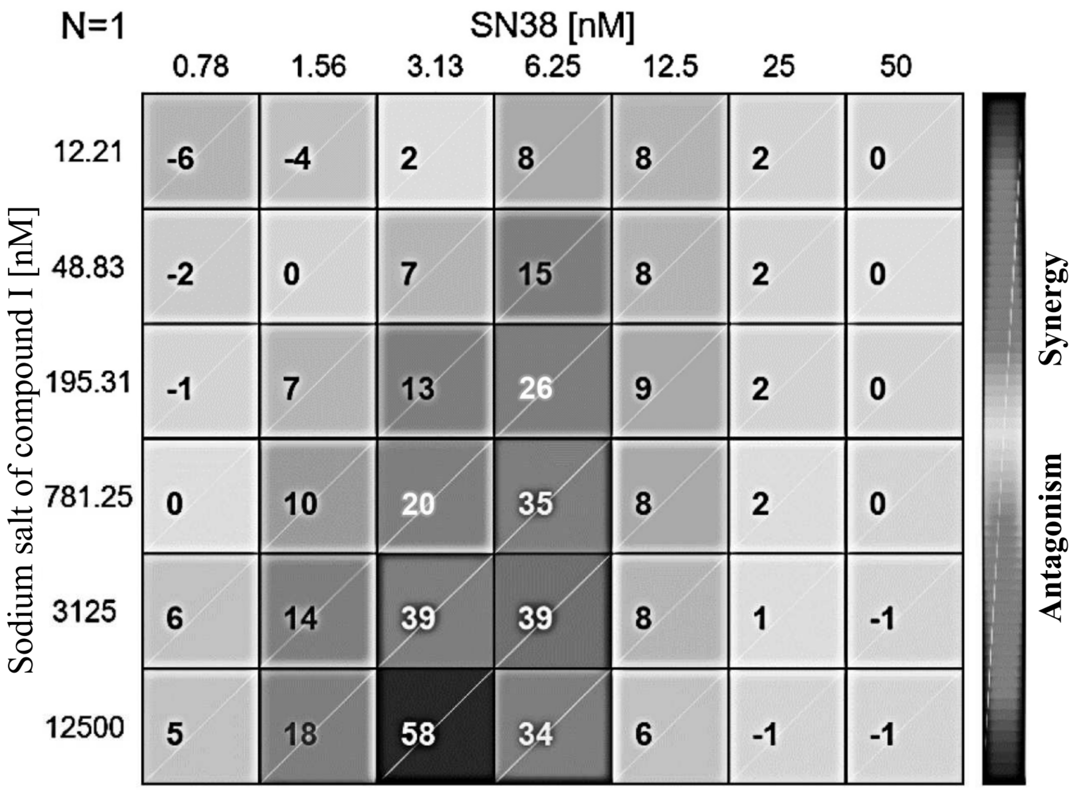


Bliss synergy and antagonism
DLD1

**FIG. 1 (Continued)**

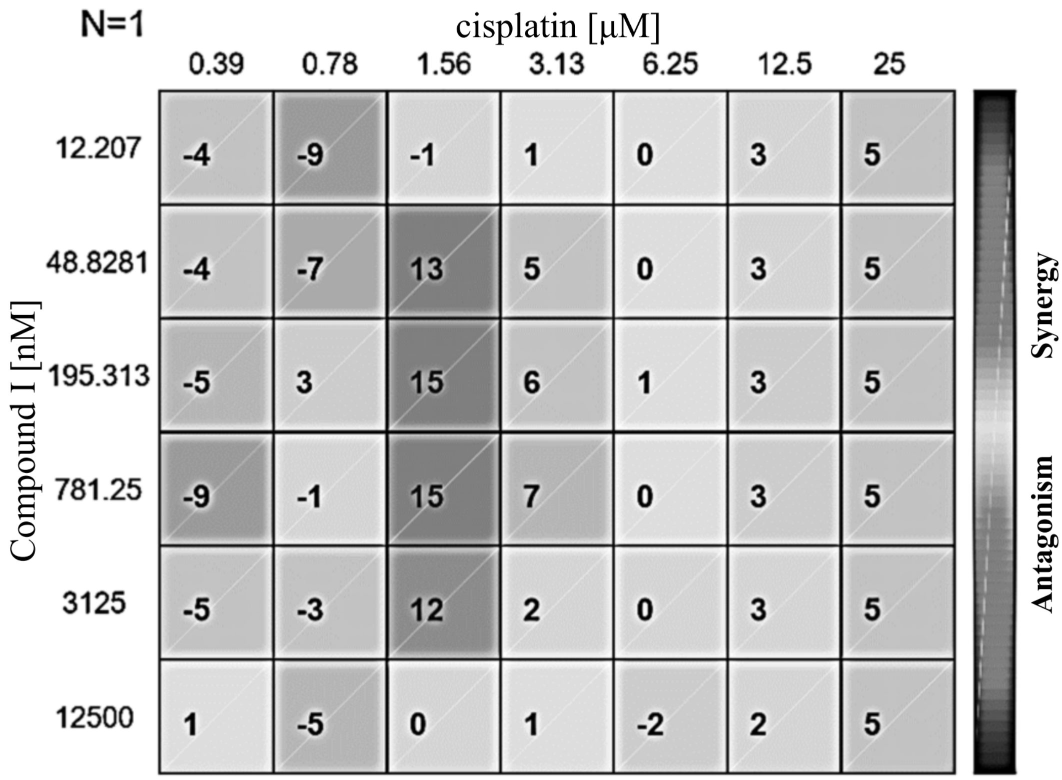


Bliss synergy and antagonism
A549

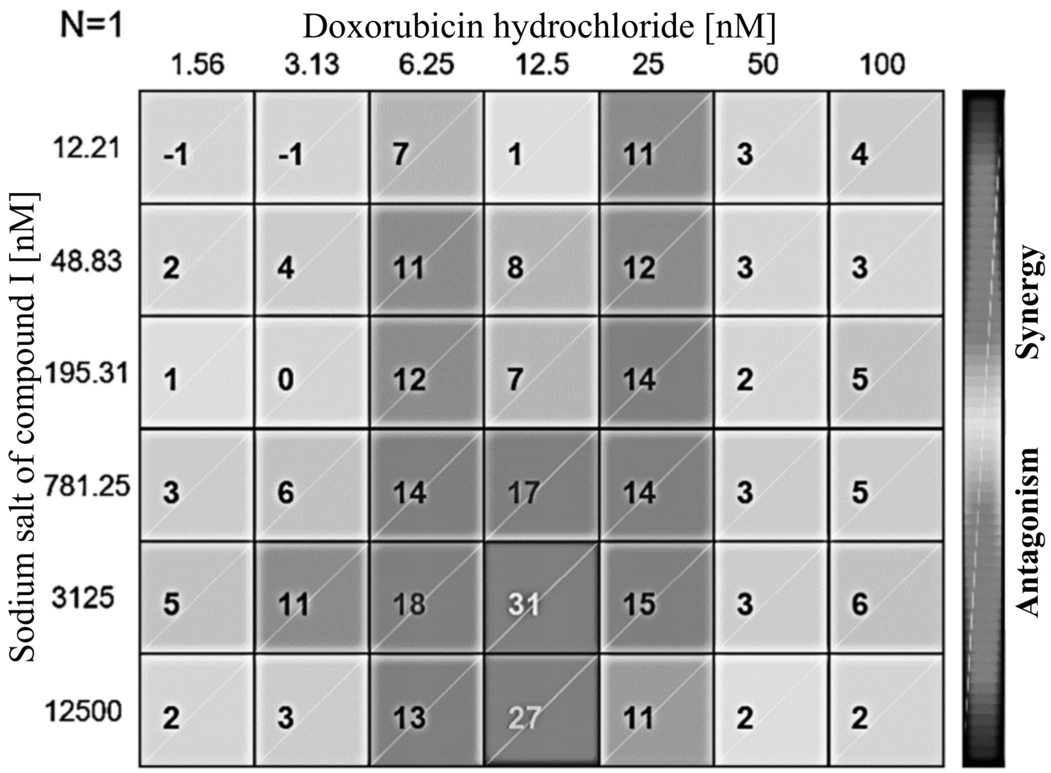


Bliss synergy and antagonism
A2780

**FIG. 1 (Continued)**

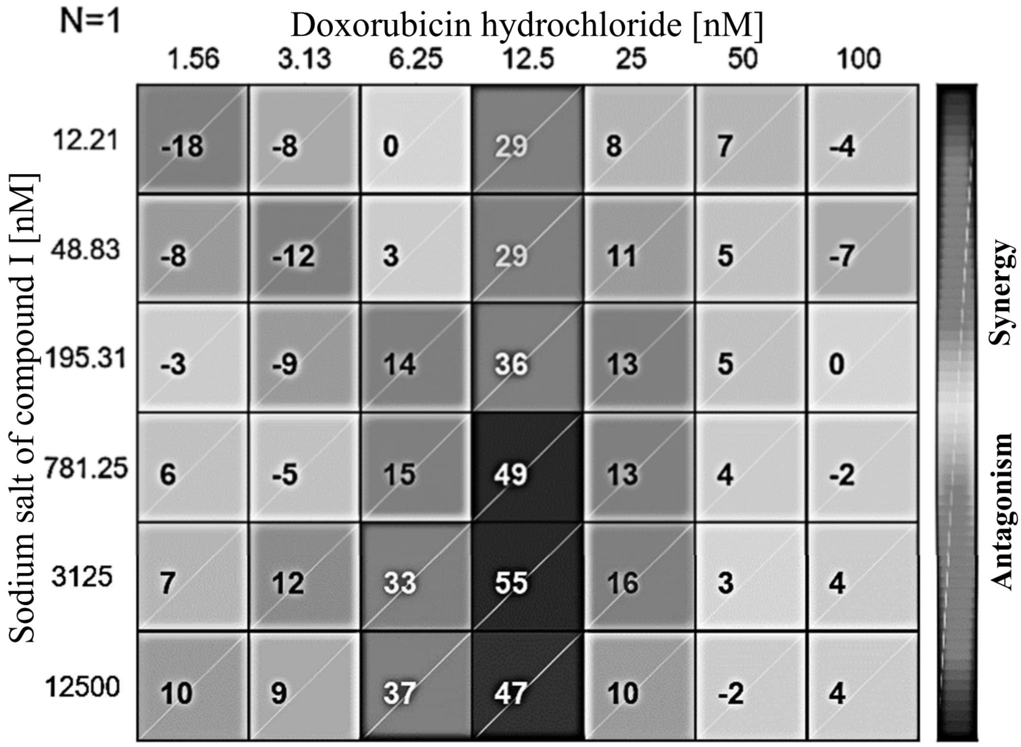


Bliss synergy and antagonism
kuramochi

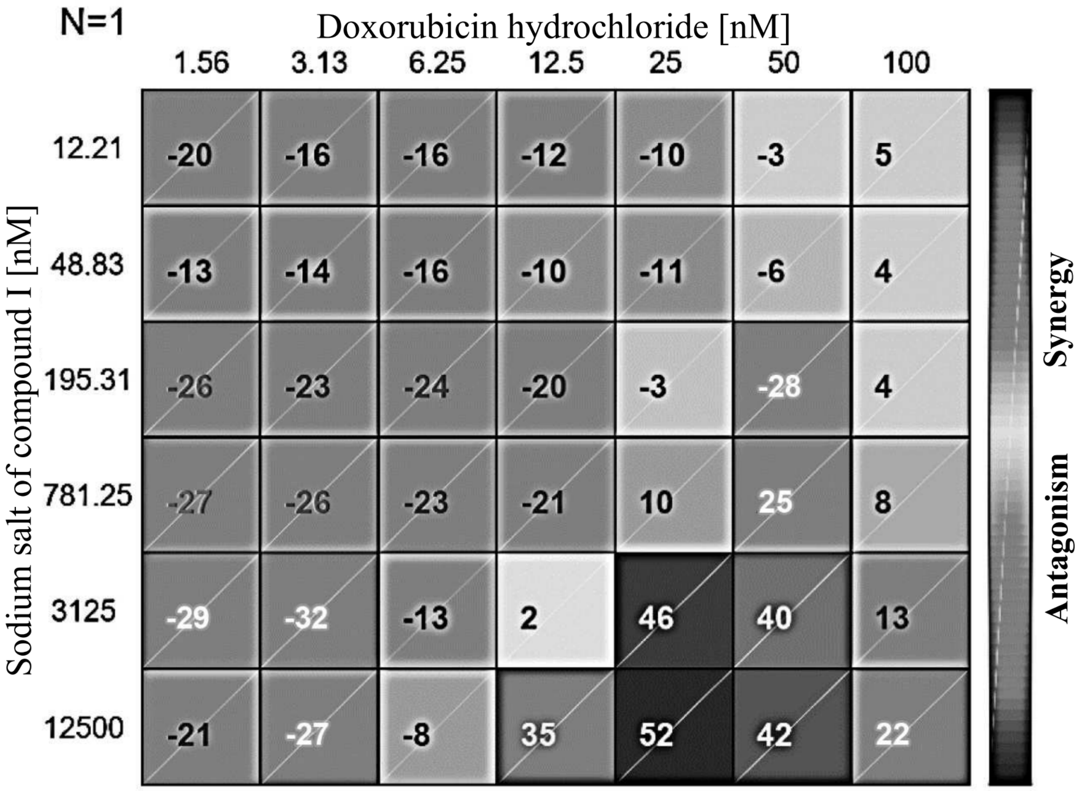


Bliss synergy and antagonism
SKOV3

**FIG. 1 (Continued)**

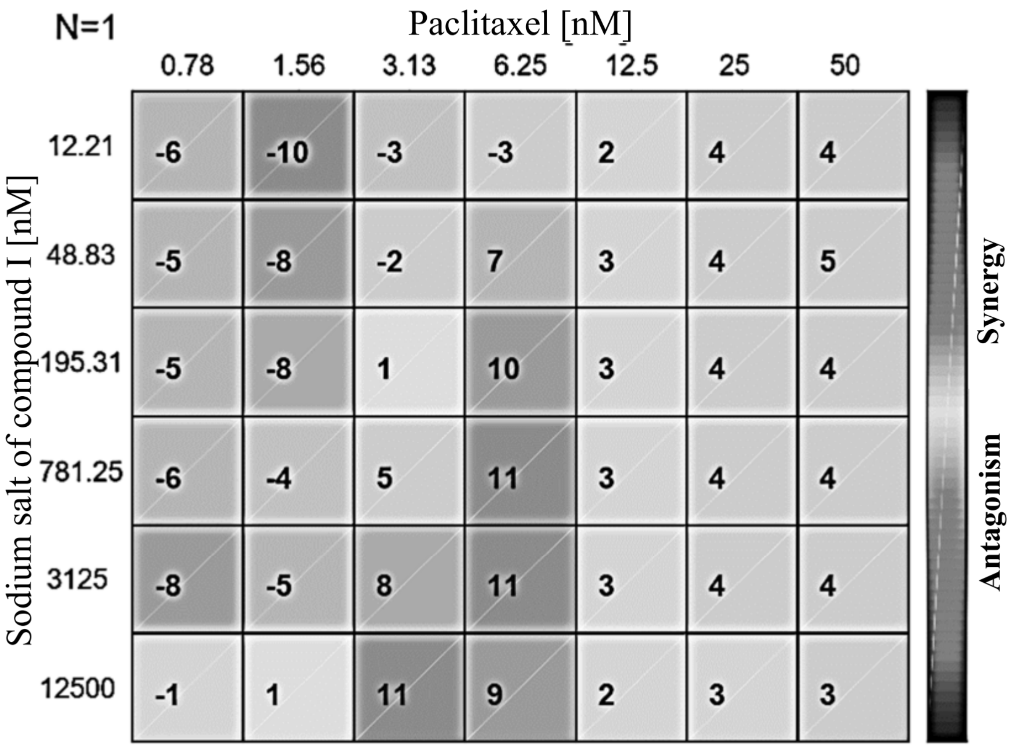


Bliss synergy and antagonism
A2780

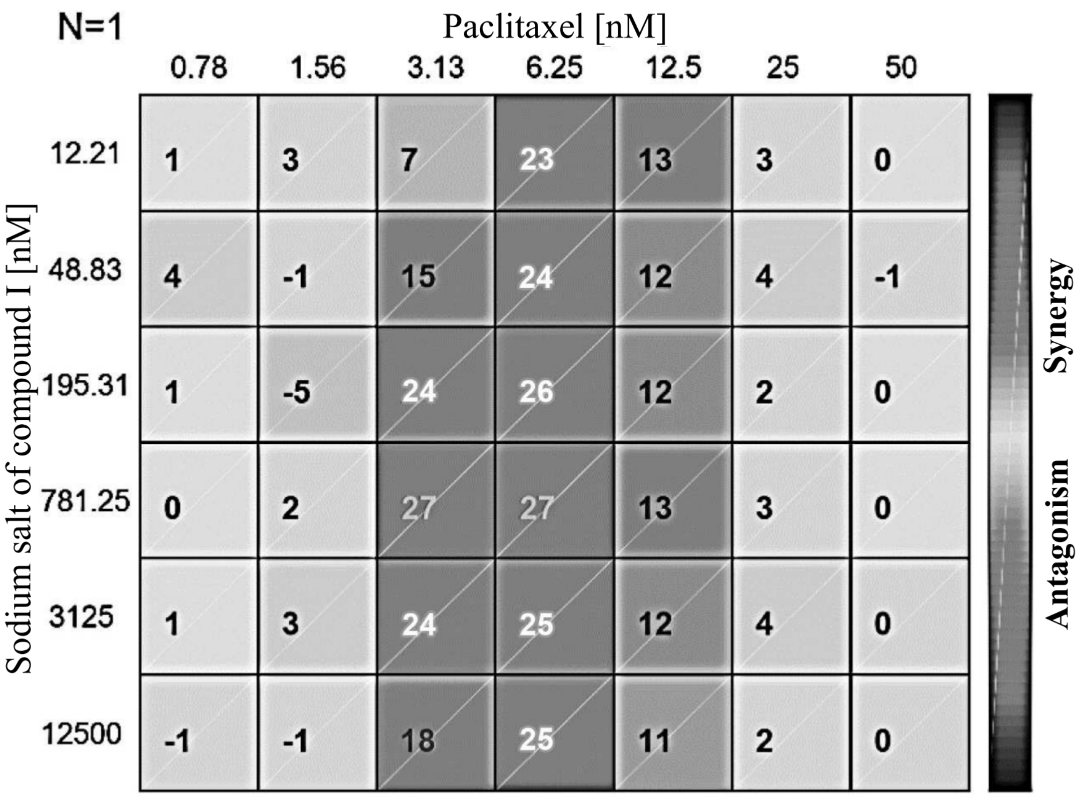


Bliss synergy and antagonism
kuramochi

**FIG. 1 (Continued)**

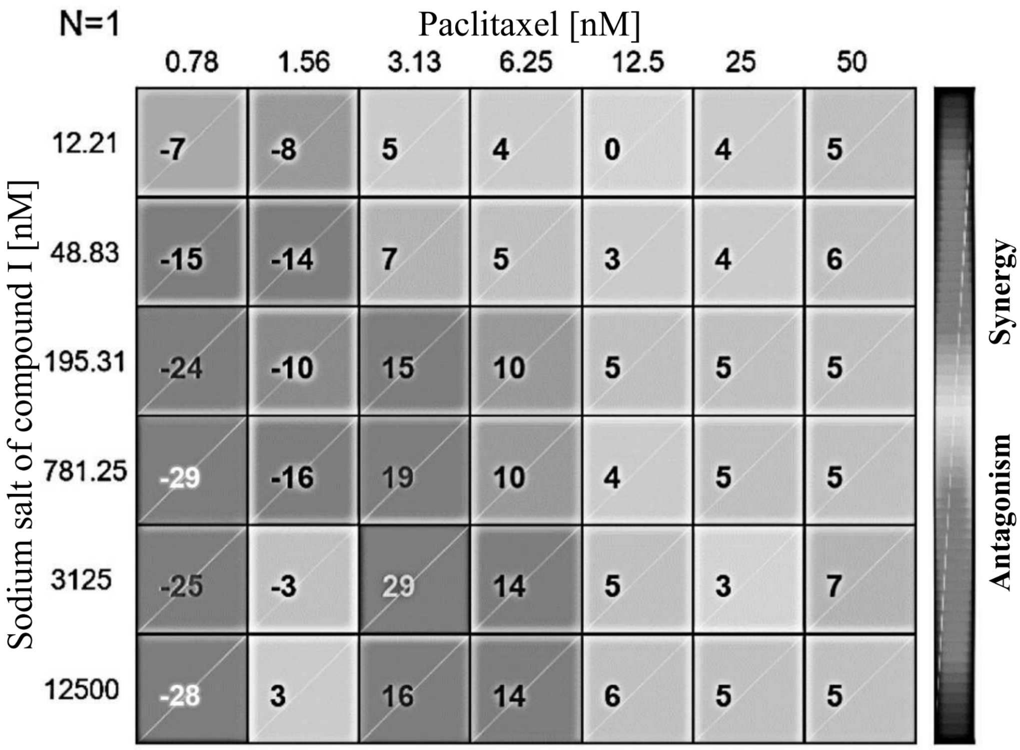


Bliss synergy and antagonism
SKOV3

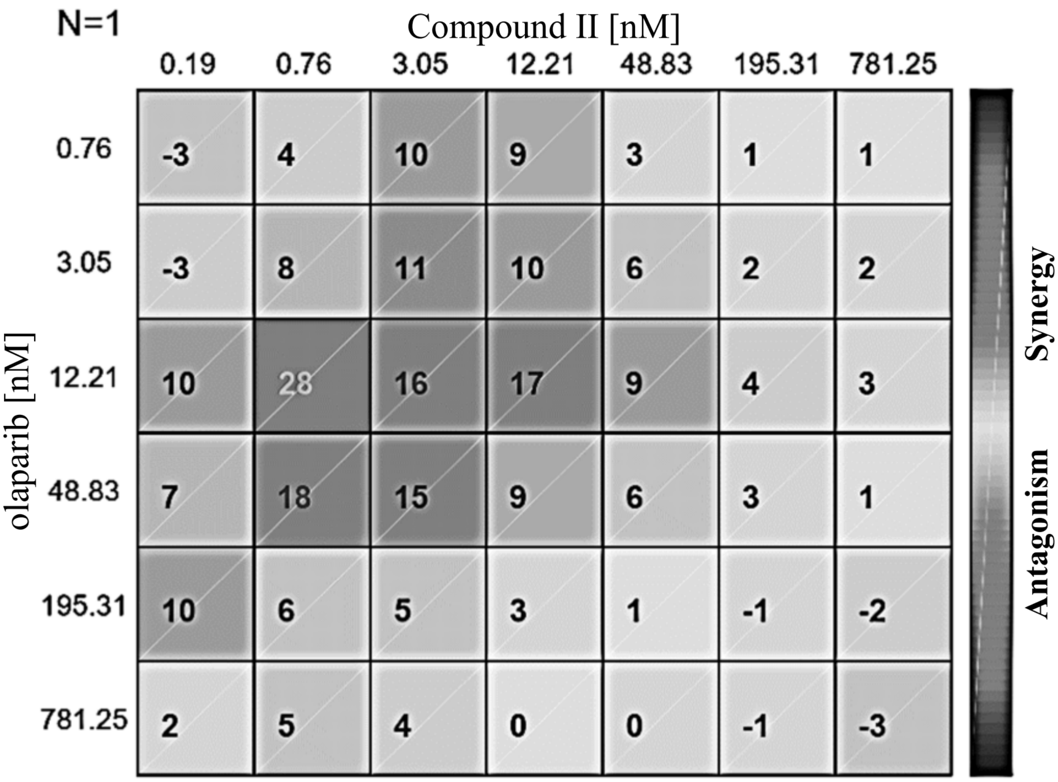


Bliss synergy and antagonism
DLD1 (BRCA2 KO)

**FIG. 1 (Continued)**

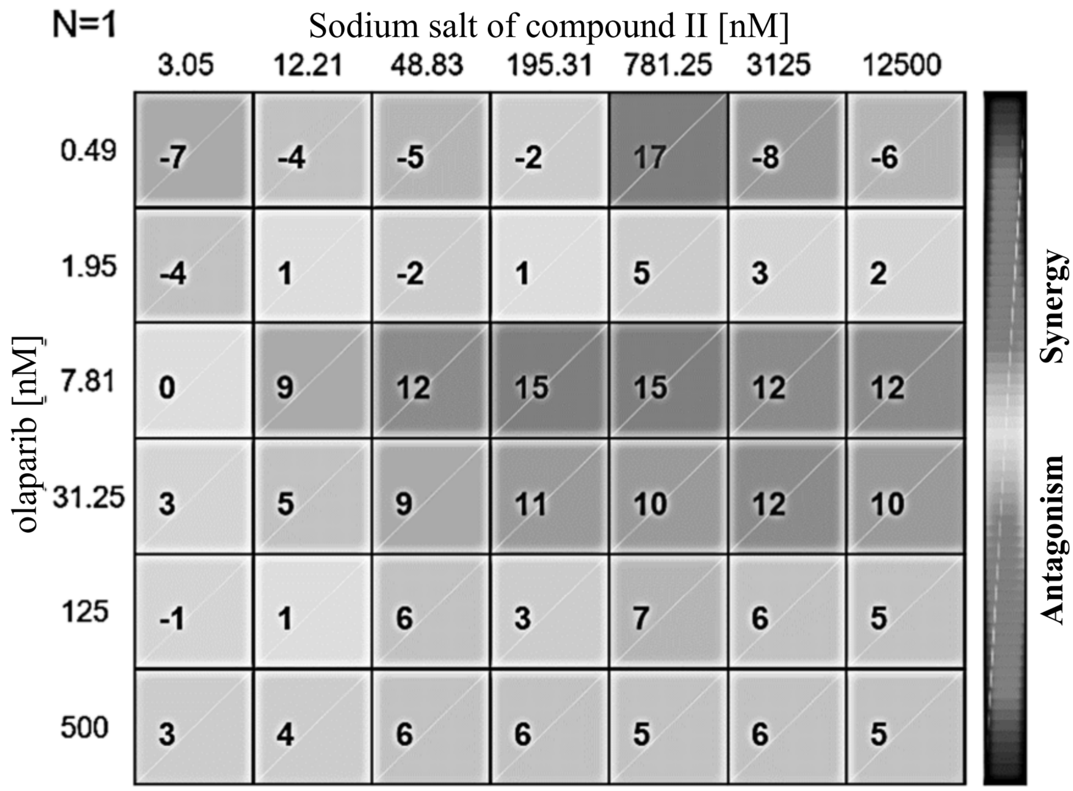


Bliss synergy and antagonism
MDA-MB-436

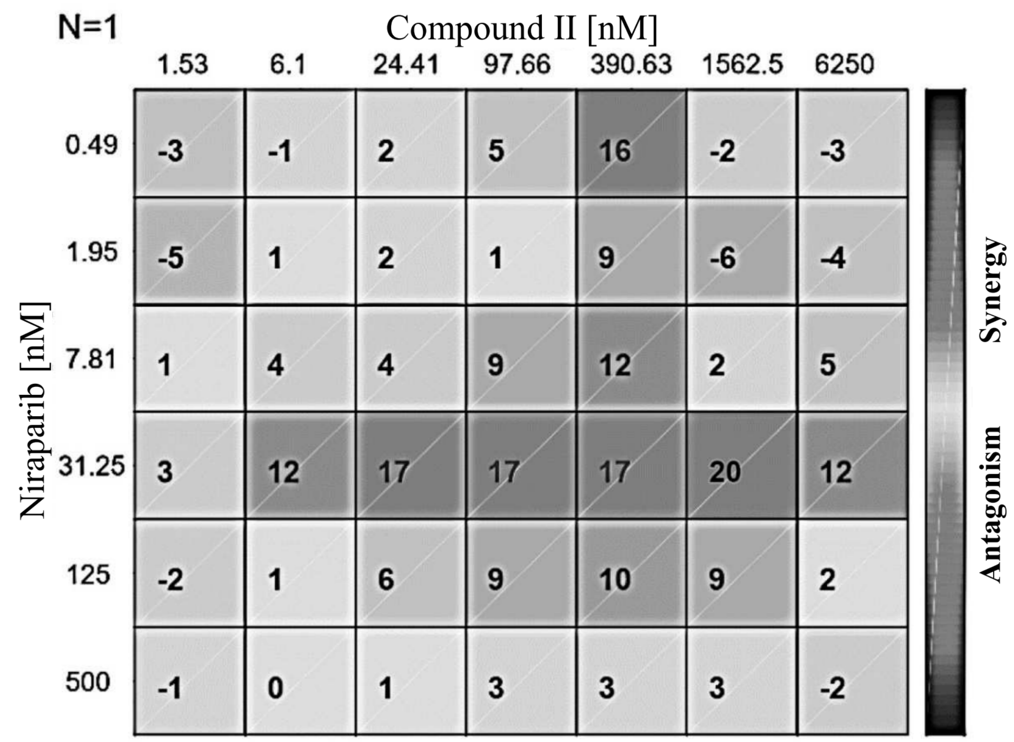


Bliss synergy and antagonism
MDA-MB-436

**FIG. 1 (Continued)**

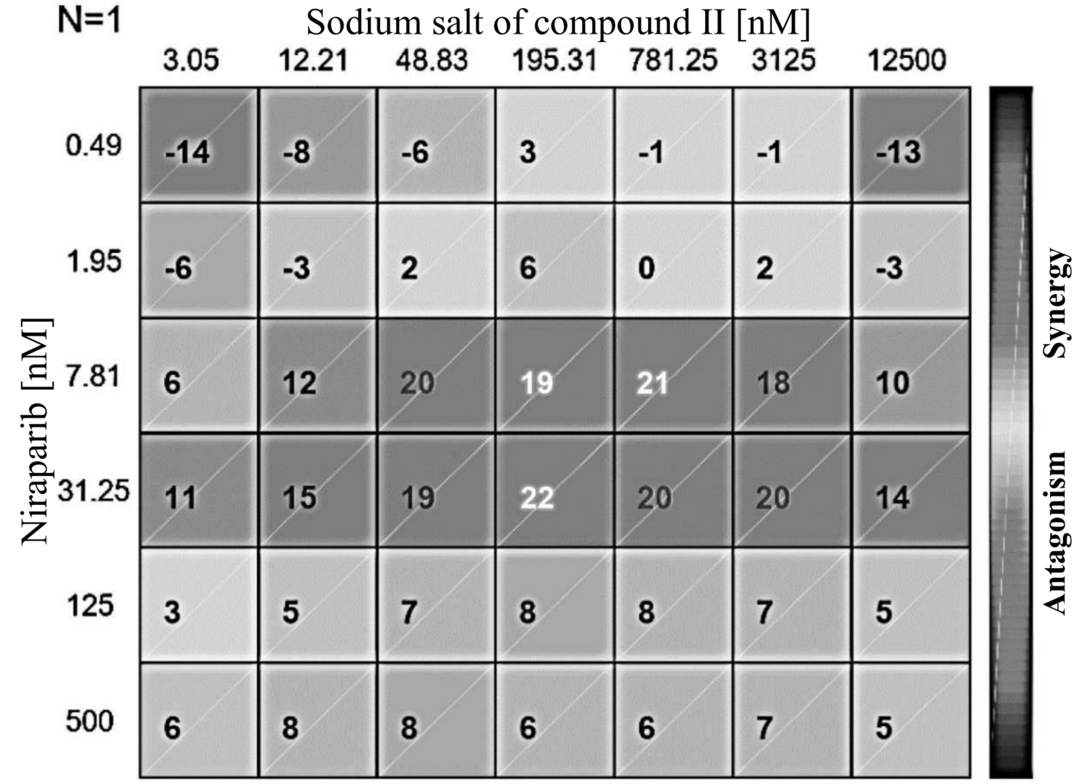


Bliss synergy and antagonism
MDA-MB-436

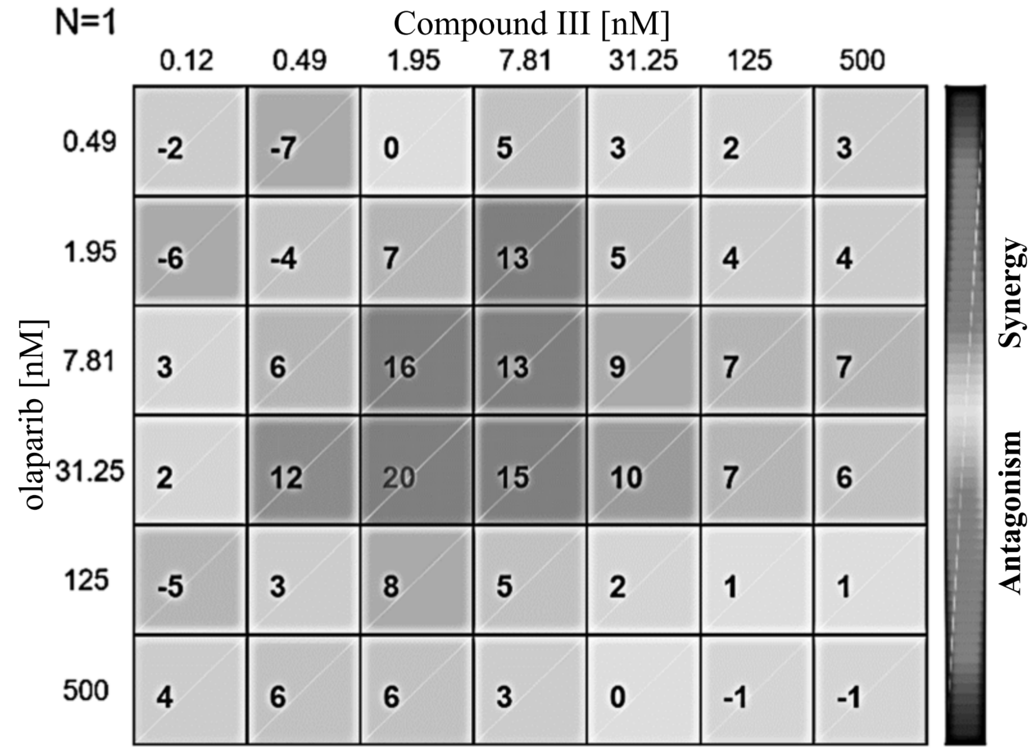


Bliss synergy and antagonism
DLD1 (BRCA2 KO)

**FIG. 1 (Continued)**

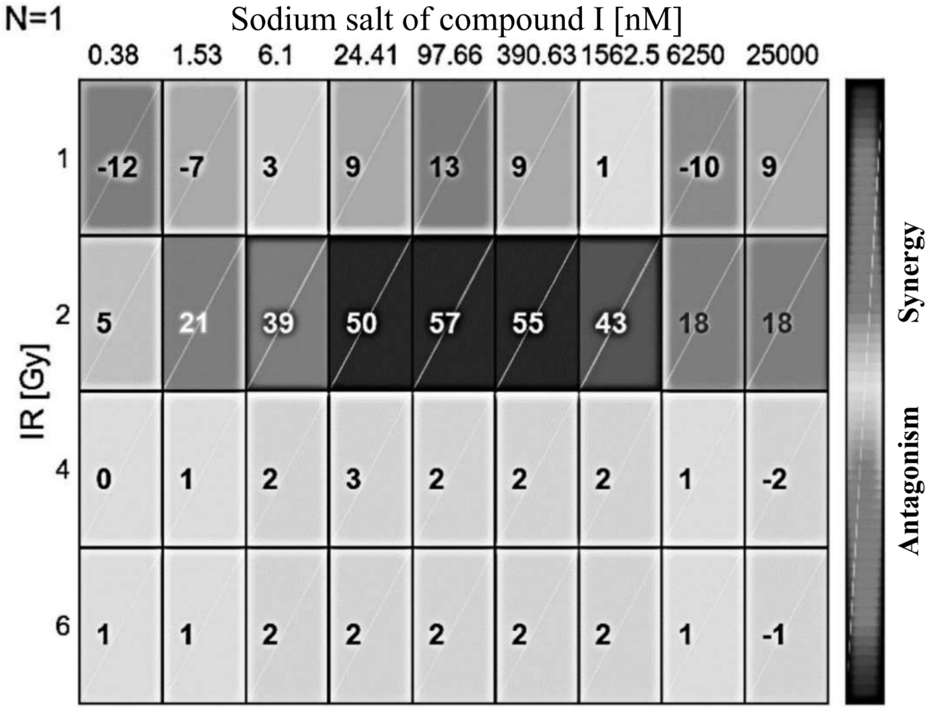


Bliss synergy and antagonism
DLD1

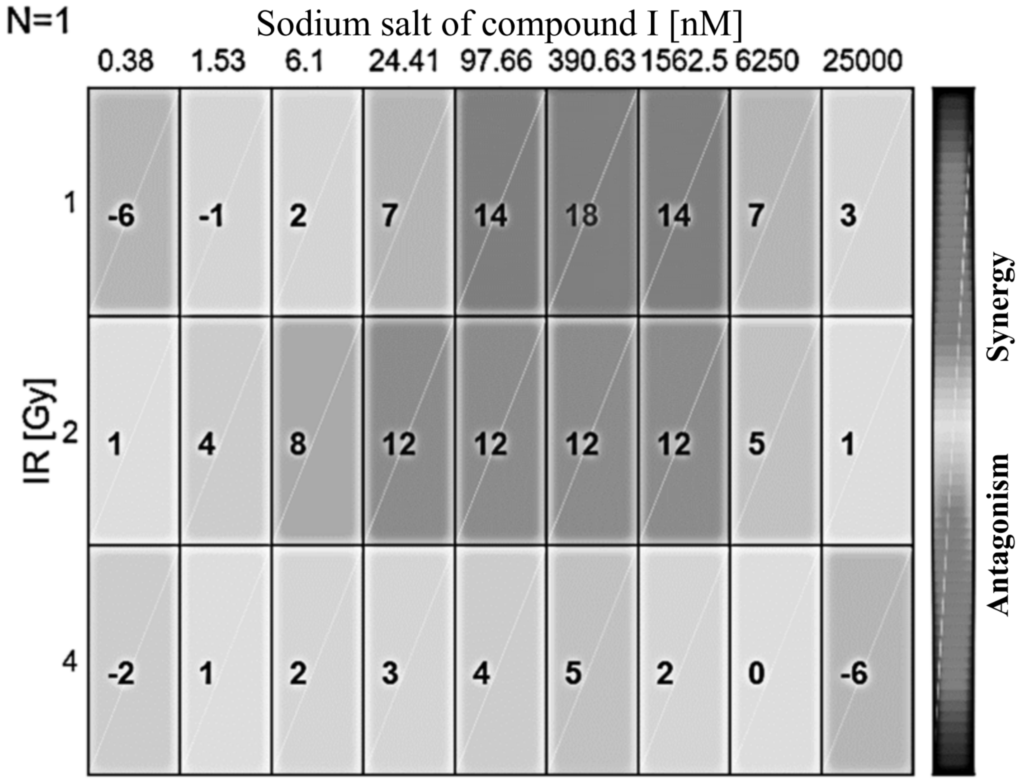


Bliss synergy and antagonism
MDA-MB-436

**FIG. 2**

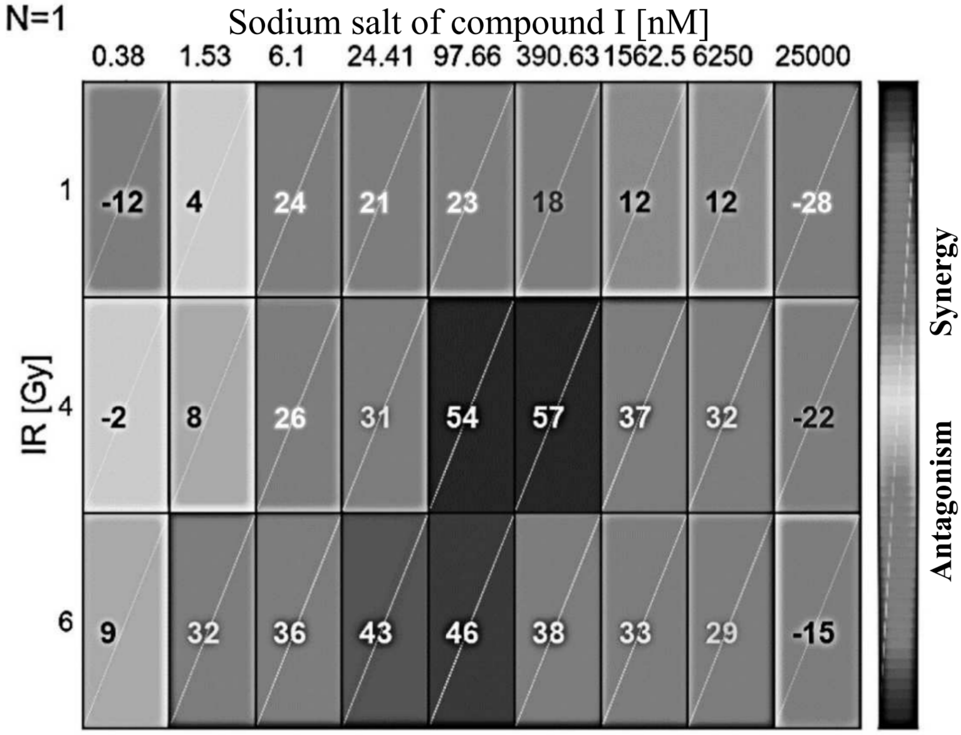


Bliss synergy and antagonism
NCIH460

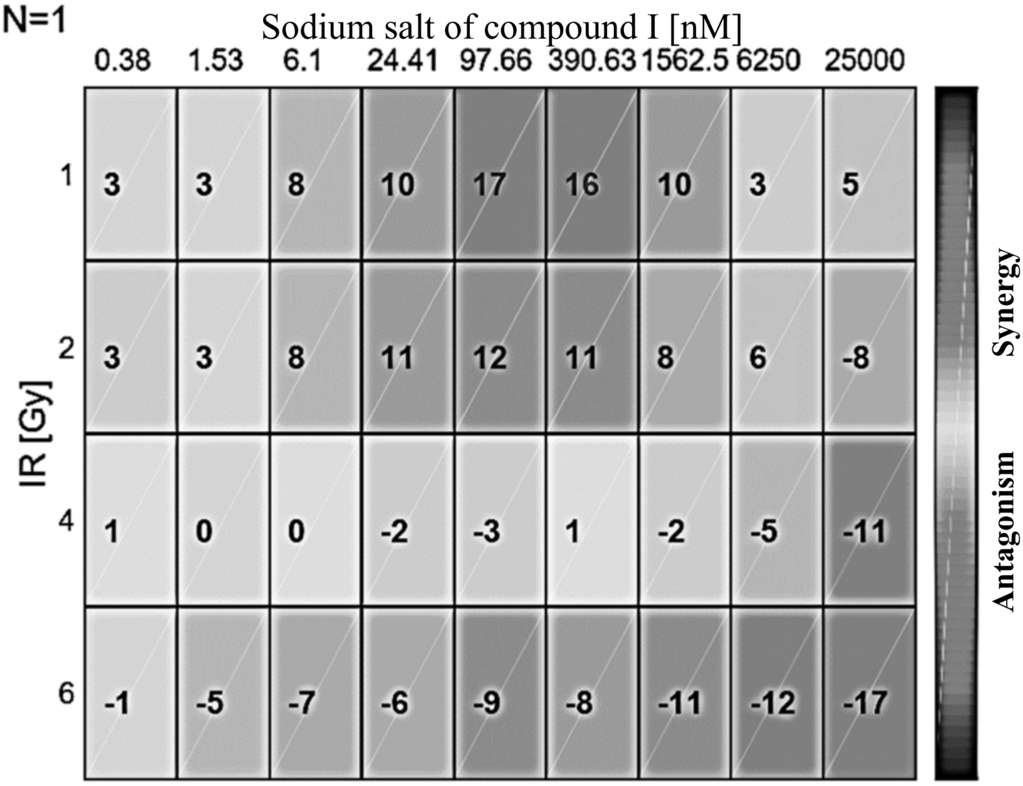


Bliss synergy and antagonism
NUGC4

**FIG. 2 (Continued)**

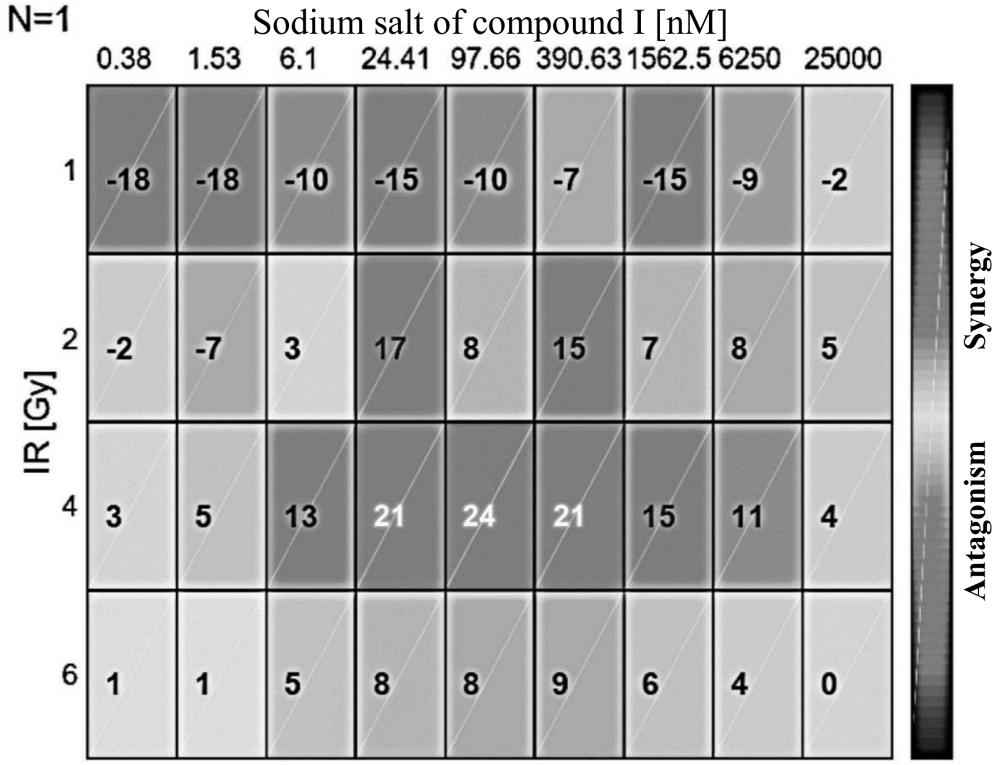


Bliss synergy and antagonism
OVCAR3

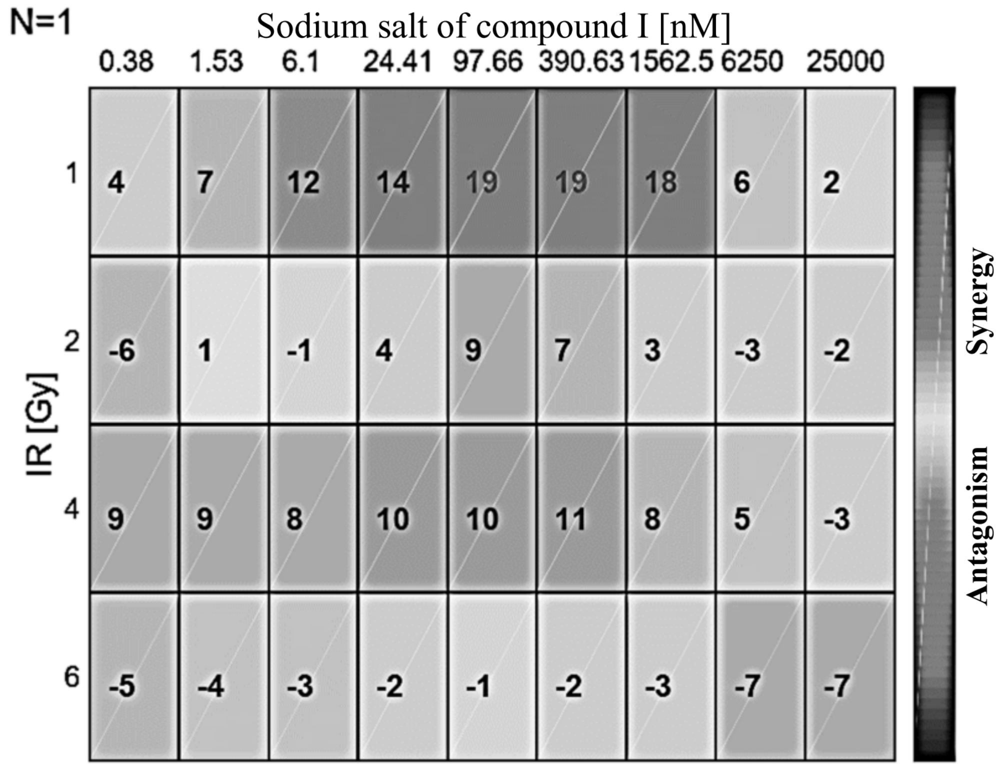


Bliss synergy and antagonism
LNCAP

**FIG. 2 (Continued)**

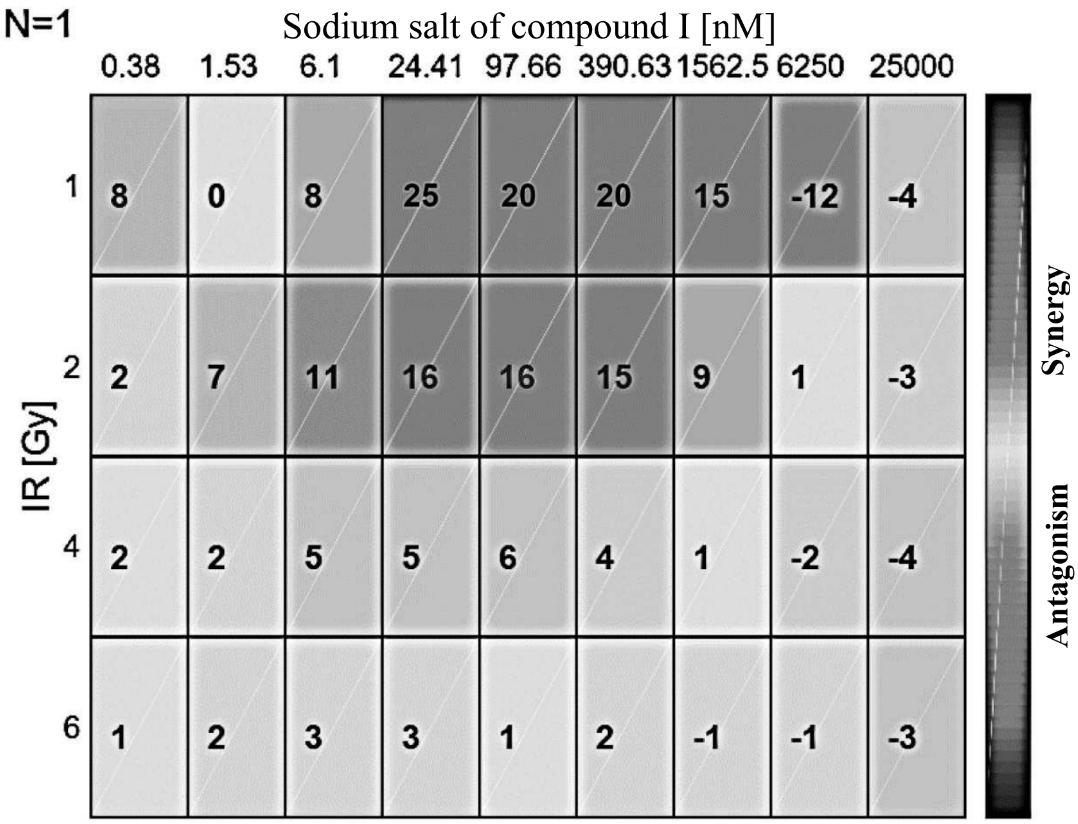


Bliss synergy and antagonism
SHP77

**FIG. 2 (Continued)**

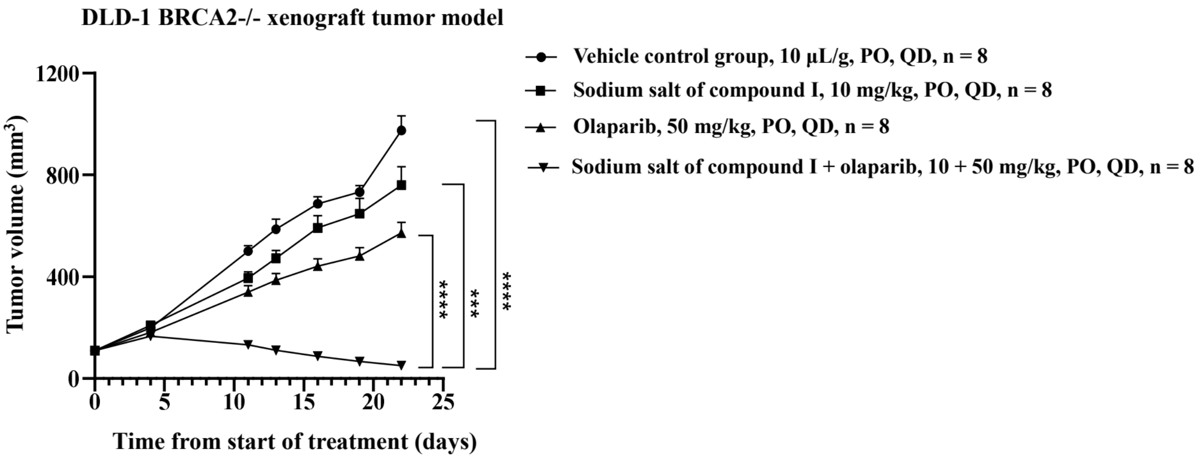
DLD-1 BRCA2-/- xenograft tumor model
Vehicle control group, 10 μL/g, PO, QD, n = 8
Sodium salt of compound I, 10 mg/kg, PO, QD, n = 8
Olaparib, 50 mg/kg, PO, QD, n = 8
Sodium salt of compound I + olaparib, 10 + 50 mg/kg, PO, QD, n = 8
Tumor volume (mm3)
1200
800
400
0
0
5
10
15
20
25
Time from start of treatment (days)
****
***
****

(A)

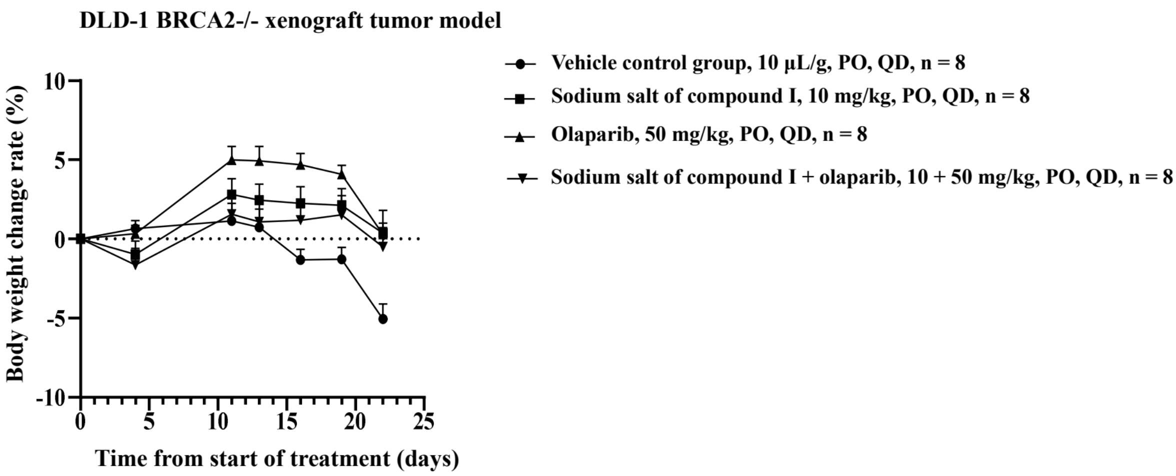
DLD-1 BRCA2-/- xenograft tumor model
Vehicle control group, 10 μL/g, PO, QD, n = 8
Sodium salt of compound I, 10 mg/kg, PO, QD, n = 8
Olaparib, 50 mg/kg, PO, QD, n = 8
Sodium salt of compound I + olaparib, 10 + 50 mg/kg, PO, QD, n = 8
Body weight change rate (%)
10
5
0
-5
-10
0
5
10
15
20
25
Time from start of treatment (days)

(B)

FIG. 3

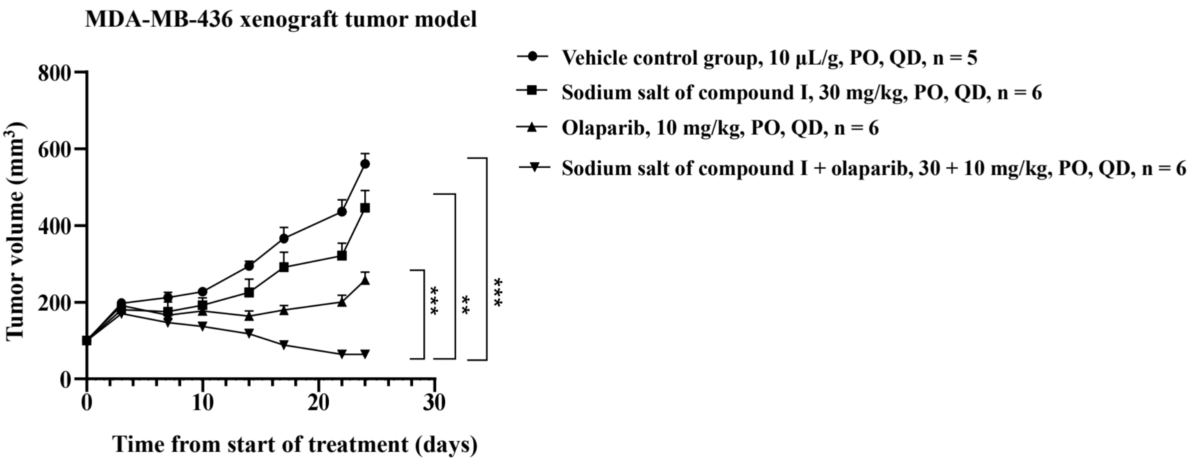
MDA-MB-436 xenograft tumor model
Vehicle control group, 10 μL/g, PO, QD, n = 5
Sodium salt of compound I, 30 mg/kg, PO, QD, n = 6
Olaparib, 10 mg/kg, PO, QD, n = 6
Sodium salt of compound I + olaparib, 30 + 10 mg/kg, PO, QD, n = 6
Tumor volume (mm³)
800
600
400
200
0
0
10
20
30
Time from start of treatment (days)
***
**
***

(A)

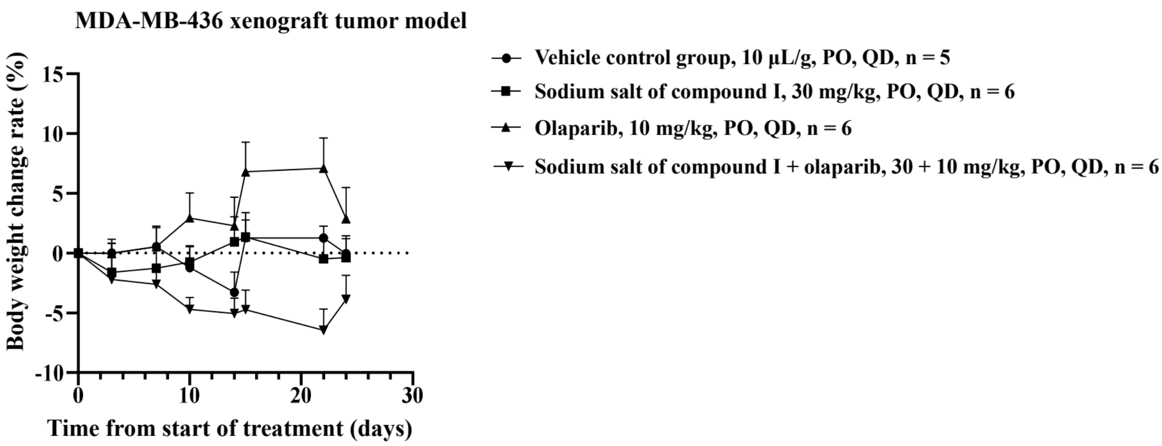
MDA-MB-436 xenograft tumor model
Vehicle control group, 10 μL/g, PO, QD, n = 5
Sodium salt of compound I, 30 mg/kg, PO, QD, n = 6
Olaparib, 10 mg/kg, PO, QD, n = 6
Sodium salt of compound I + olaparib, 30 + 10 mg/kg, PO, QD, n = 6
Body weight change rate (%)
15
10
5
0
-5
-10
0
10
20
30
Time from start of treatment (days)

(B)

**FIG. 4**

# INTERNATIONAL SEARCH REPORT

International application No.
**PCT/CN2024/105005**

**A. CLASSIFICATION OF SUBJECT MATTER**

A61K 31/519(2006.01)i; A61K31/428(2006.01)i; A61K31/429(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:A61K,A61P

Documentation searched other than minimum documentation to the extent that such documents are included in the fields searched

Electronic data base consulted during the international search (name of data base and, where practicable, search terms used)

CNABS, CNTXT, DWPI, VEN, ENTXTC, ENTXT, CNKI, PUBMED, ISI_Web of Science, Science Direct, STNext: 南京再明医药有限公司, 周峰, 蒋蕾, 刘璐, 钱烁, 薛黎婷, 李正涛, 李桢, 唐任宏, 噻唑, 癌症, 肿瘤, HR缺陷, 抑制剂, POLQ, POL0, DNA, thiazole, HR deficiency, inhibitor, cancer, tumor, anticancer, antitumor, 2954112-05-9, 2954112-03-7, 2954112-04-8

**C. DOCUMENTS CONSIDERED TO BE RELEVANT**

| Category* | Citation of document, with indication, where appropriate, of the relevant passages | Relevant to claim No. |
|---|---|---|
| PX | WO 2023134739 A1 (NANJING ZAIMING PHARMACEUTICAL CO., LTD.) 20 July 2023 (2023-07-20)<br>claims 1-22, and description, embodiments 1-4 | 1-20 |
| A | WO 2020160213 A1 (IDEAYA BIOSCIENCES, INC.) 06 August 2020 (2020-08-06)<br>abstract, claim 1, and description, paragraphs [0010] and [0080] | 1-20 |
| A | CN 108047154 A (EAST CHINA UNIVERSITY OF SCIENCE AND TECHNOLOGY) 18 May 2018 (2018-05-18)<br>claims 1-10, and description, embodiments 1-22 | 1-20 |
| A | WO 2020243459 A1 (IDEAYA BIOSCIENCES, INC.) 03 December 2020 (2020-12-03)<br>claims 1-72 | 1-20 |
| A | WO 2021028643 A1 (ARTIOS PHARMA LIMITED) 18 February 2021 (2021-02-18)<br>claims 1-33 | 1-20 |
| A | WO 2022078403 A1 (JIANGSU SIMCERE PHARMACEUTICAL CO., LTD.) 21 April 2022 (2022-04-21)<br>claims 1-14, and description, test examples 1-6 | 1-20 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 October 2024** | **21 October 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/105005**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **16, 18, 19-20 (in part)**
because they relate to subject matter not required to be searched by this Authority, namely:

Claims 16, 18 and 19-20 (in part) relate to the use of a combiniation of the compound of formula (A) of any one of claims 1-15 or a pharmaceutically acceptable salt thereof or a pharmaceutical composition thereof and at least one anti-cancer therapeutic agent or radiation therapy in the prevention or treatment of cancer, or a method for preventing or treating cancer in a mammal. The subject matter of claims 16, 18 and 19-20 (in part) belongs to a method for treatment of diseases as defined in PCT Rule 39.1(iv). However, a search is still performed on the basis of the preparation use thereof, that is, the use of a combiniation of the compound of formula (A) of any one of claims 1-15 or a pharmaceutically acceptable salt thereof or a pharmaceutical composition thereof and at least one anti-cancer therapeutic agent or radiation therapy in the preparation of a drug for preventing or treating cancer.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/105005**

| Patent document cited in search report | Publication date (day/month/year) | Patent family member(s) | Publication date (day/month/year) |
|---|---|---|---|
| WO 2023134739 A1 | 20 July 2023 | None | |
| WO 2020160213 A1 | 06 August 2020 | EP 3917919 A1 | 08 December 2021 |
| | | AU 2020215042 A1 | 19 August 2021 |
| | | BR 112021015007 A2 | 05 October 2021 |
| | | CA 3127642 A1 | 06 August 2020 |
| | | US 2021387968 A1 | 16 December 2021 |
| | | JP 2022519535 A | 24 March 2022 |
| CN 108047154 A | 18 May 2018 | None | |
| WO 2020243459 A1 | 03 December 2020 | TW 202110834 A | 16 March 2021 |
| | | CA 3141134 A1 | 03 December 2020 |
| | | US 2023078112 A1 | 16 March 2023 |
| | | JP 2022535227 A | 05 August 2022 |
| | | AU 2020282768 A1 | 23 December 2021 |
| | | AU 2020282768 B2 | 14 September 2023 |
| | | ES 2968796 T3 | 14 May 2024 |
| | | EP 3976608 A1 | 06 April 2022 |
| | | EP 3976608 C0 | 08 November 2023 |
| | | BR 112021024101 A2 | 08 February 2022 |
| WO 2021028643 A1 | 18 February 2021 | None | |
| WO 2022078403 A1 | 21 April 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310854573X


### Non-patent literature cited in the description


- **KENT et al.** *Nature Structural & Molecular Biology*, 2015, vol. 22 (3), 230-237
- **MATEOS-GOMEZ et al.** *Nature*, 2015, vol. 518 (7538), 254-257
- **WOOD ; DOUBLIE**. *DNA Repair*, 2016, vol. 44, 22-32
- **CECCALDI et al.** *Nature*, 2015, vol. 518 (7538), 258-262
- **HIGGINS et al.** *Oncotarget*, 2010, vol. 1, 175-184
- **LEMEE et al.** *PNAS*, 2010, vol. 107 (30), 13390-13395
- **KAWAMURA et al.** *International Journal of Cancer*, 2004, vol. 109 (1), 9-16
- **BLISS, C.I.** *Bacteriol. Rev.*, 1956, vol. 20, 243-258